# EUROPEAN PATENT SPECIFICATION

(11) **EP 4 422 479 B1**
(45) Date of publication and mention of the grant of the patent: **16.09.2026**
(21) Application number: 22937673.6
(22) Date of filing: 27.10.2022
(51) Int. Cl.: A61B 5/00, A61B 5/145, A61B 5/16

(54) **CONTINUOUS CORTISOL MONITORING SYSTEM WITH MICRONEEDLE ARRAY**
KONTINUIERLICHES CORTISOLÜBERWACHUNGSSYSTEM MIT MIKRONADELANORDNUNG
SYSTÈME DE SURVEILLANCE DU CORTISOL EN CONTINU DOTÉ D'UN RÉSEAU DE MICRO-AIGUILLES

(30) Priority: 27.10.2021 US 202163272640 P
(43) Date of publication of application: 04.09.2024
(73) Proprietor: Biolinq, Inc., San Diego, CA 92121 (US)
(72) Inventor: MALLIRES, Kyle, San Diego, California 92109 (US); KAVNER, Jonathan Everett, San Diego, California 92129 (US); WINDMILLER, Joshua Ray, San Diego, California 92130 (US); ARROYO, Netzahualcoyotl, Baltimore, Maryland 21218 (US)
(74) Representative: Cooley (UK) LLP
(86) International application number: PCT/US2022/078819
(87) International publication number: WO 2023/229662

(56) References cited:
- EP-A1- 3 364 183
- WO-A1-2021/062475
- WO-A1-2021/118431
- US-A1- 2019 201 675
- US-A1- 2020 000 387
- US-A1- 2020 390 395
- US-A1- 2021 236 057

## Description

### REFERENCE TO AN ELECTRONIC SEQUENCE LISTING

The contents of the electronic sequence listing submitted electronically herewith (filename: BLNQ_002_01WO_SeqList_ST26.xml; Size: 1,957 bytes; and Date of Creation: October 24, 2022).

### TECHNICAL FIELD

This invention relates generally to the field of cortisol monitoring.

### BACKGROUND

Chronic stress is recognized as a pre-morbidity associated with many risk factors of various chronic diseases. While acute stressors may induce an individual's adaptive response to environmental demands, they can reduce one's ability to complete cognitively demanding tasks when exposure is intransient. Excessive stress, whether chronic or intransient, causes cumulative negative impacts on short-term performance and long-term health outcomes through a concept known as the "allostatic load".

The ability to assess stress, in real time, for those engaged in high-performance work functions is paramount to ensuring the safety of all stakeholders and faithful execution of the task at hand. To fulfill this objective, active measures such as lines of questioning and the completion of simple tasks are typically employed to assess the effects of stress. However, these approaches are highly disruptive in most relevant operating environments. A known device to measure the stress levels is disclosed by WO 2021/062475 A1.

The need for real-time assessment of stress levels has driven a recent push towards the identification of circulating biochemical markers of cognitive function as true molecular surrogates for evaluating the systemic physiological impact of acute and chronic stressors. The glucocorticoid hormone cortisol has widespread acceptance in the scientific community as a leading biomarker of physiological stress. Sourced from the zona fasciculata of the adrenal cortex in the adrenal gland, cortisol release elicits an acute catabolic response, which is believed to enhance gluconeogenesis, suppress the immune response, and contributes to the allostatic load due to chronic elevations. Cortisol also exhibits systemic distribution and rapid uptake dynamics due to its ability to scavenge glucose in anticipation of the 'fight-or-flight' response.

### SUMMARY

Aspects of the current subject matter are directed to a microneedle array configured to sense cortisol in dermal interstitial fluid.

In some variations, a microneedle array for use in sensing cortisol in dermal interstitial fluid may include a plurality of solid microneedles, and at least one microneedle of the plurality of solid microneedles may include a tapered distal portion having an insulated distal apex. In some variations, the microneedle array further may comprise a semiconductor substrate, wherein the plurality of solid microneedles extend from the semiconductor substrate. In some variations, the at least one microneedle may comprise a columnar body portion. In some variations, a working electrode may be located on a surface of the tapered distal portion that is proximal to the insulated distal apex. In some variations, the working electrode may be an annular electrode. Optionally, a distal edge of the annular working electrode may be proximate a proximal edge of the insulated distal apex. Optionally, the annular electrode may be on only the segment of the surface of the tapered distal portion of the microneedle. In some variations, the working electrode may be configured to generate a sensor signal that is indicative of a concentration of cortisol in the dermal interstitial fluid when contacting the dermal interstitial fluid.

In some variations, the working electrode may include an electrode material and a biorecognition layer arranged at least partially over the electrode material, wherein the biorecognition layer includes an aptamer that selectively and reversibly binds cortisol.

In some variations, the biorecognition layer may include a conductive polymer layer and the aptamer, and the electrode material may include platinum. In some variations, the aptamer may be tethered to the conductive polymer layer via an amide linker. The amide linker may be formed through a reduction of a carboxyl group in the conductive polymer layer and an amine group covalently bound to a 3' end or a 5' end of the aptamer, or conversely of an amine group in the conductive polymer layer and a carboxyl group covalently bound to a 3' end or a 5' end of the aptamer.

In some variations, the electrode material may include gold, and the aptamer may be tethered to the electrode material via a thiol link between the gold and a thiol group covalently bound to a 3' end or a 5' end of the aptamer. In some variations, the biorecognition layer may further include 6-mercapto-1-hexanol tethered to the gold via a thiol link.

In some variations, the aptamer may be covalently bound to a redox-active molecule at the 3' end or the 5' end of the aptamer, such that selective binding of the cortisol to the aptamer and a resulting conformational change of the aptamer brings the redox-active molecule closer to or farther from a surface of the electrode material to facilitate or attenuate electron transfer between the redox-active molecule and the electrode material, thereby generating the sensor signal. In some variations, the redox-active molecule may be methylene blue or an anthraquinone.

In some variations, the working electrode may further include a biocompatible layer arranged at least partially over the electrode material and the biorecognition layer. In some variations, the biocompatible layer may include or be a hydrophilic polymer, which may be poly(urethane), poly(ethylene glycol), poly(vinyl alcohol), poly(lactic acid), collagen, alginate, chitosan, Nafion, or cellulose acetate.

In some variations, a cortisol monitoring device may include an above-noted embodiment of a microneedle array. In some variations, the cortisol monitoring device may be a wearable device including a wearable housing, and the microneedle array may extend outwardly from the wearable housing and is configured so that the working electrode reaches a dermal interstitial fluid of a user when the device is worn by the user. In some variations, the wearable housing may include a communication module operatively connected to the microneedle array and configured to transmit a wireless signal responsive to the sensor signal generated by the at least one microneedle.

In some variations, a method for monitoring cortisol in a user may include providing a cortisol monitoring device having a plurality of solid microneedles, wherein at least one microneedle of the plurality of solid microneedles includes a tapered distal portion having an insulated distal apex, accessing the dermal interstitial fluid of the user with the at least one microneedle; and generating with the at least one solid microneedle the sensor signal responsive to the working electrode contacting the dermal interstitial fluid. In some variations, the microneedle array further may comprise a semiconductor substrate, wherein the plurality of solid microneedles extend from the semiconductor substrate. In some variations, the at least one microneedle may comprise a columnar body portion. In some variations, a working electrode may be located on a surface of the tapered distal portion proximal to the insulated distal apex. In some variations, the working electrode may be an annular electrode. Optionally, a distal edge of the annular working electrode may be proximate a proximal edge of the insulated distal apex. Optionally, the annular electrode may be on only the segment of the surface of the tapered distal portion of the microneedle.

In some variations, the working electrode may be configured to generate a sensor signal that is indicative of a concentration of the cortisol in a dermal interstitial fluid when the working electrode is contacting the dermal interstitial fluid. In some variations, the working electrode may include an electrode material and a biorecognition layer arranged at least partially over the electrode material, wherein the biorecognition layer may include an aptamer that selectively and reversibly binds the cortisol. In some variations, the aptamer may be covalently bound to a redox-active molecule at the 3' end or the 5' end of the aptamer such that selective binding of the cortisol to the aptamer and a resulting conformational change of the aptamer brings the redox-active molecule closer to a surface of the electrode material to facilitate electron transfer between the redox-active molecule and the electrode material, thereby generating the sensor signal. In some variations, the redox-active molecule may be methylene blue or an anthraquinone. In some variations, the working electrode may further include a biocompatible layer arranged at least partially over the electrode material and the biorecognition layer.

In some variations, the method may further include transmitting a wireless signal responsive to the sensor signal generated by the at least one microneedle.

In some variations, a cortisol monitoring device may include a wearable housing having a user interface, at least one microneedle extending outwardly from the wearable housing and configured to reach a dermal interstitial fluid of a user when the device is worn by the user, and a working electrode located on a surface of the at least one microneedle and configured to generate a sensor signal that is indicative of a concentration of cortisol in the dermal interstitial fluid. In some variations, the user interface may include one or more indicator lights, each of the one or more indicator lights configured to be selectively illuminated responsive to the sensor signal.

In some variations, the working electrode may include an electrode material and a biorecognition layer arranged at least partially over the electrode material, the biorecognition layer may include an aptamer that selectively and reversibly binds to the cortisol, and the working electrode may be configured to generate the sensor signal responsive to the cortisol binding the aptamer.

In some variations, the cortisol monitoring device may include one or more processors and at least one memory storing instructions which, when executed by the one or more processor, result in operations including determining a user status based on the sensor signal and controlling illumination of the one or more indicator lights based on the user status. In some variations, the controlling of the illumination may include selectively illuminating the one or more indicator lights in a spatial pattern and/or a temporal pattern based on the user status.

In some variations, the user status may be a current cortisol level of the user. The current cortisol level may be a current cortisol concentration in the dermal interstitial fluid or may be a current cortisol concentration in the bloodstream of the user. In some variations, the user status may be a psychological state of the user, such as a degree of stress of the user.

In some variations, the one or more indicator lights include a plurality of indicator lights; and controlling of the illumination including illuminating the plurality of indicator lights in a progressive sequence responsive to an upward trend or a downward trend of the sensor signal. In some variations, the controlling of the illumination may include illuminating the plurality of indicator lights in a first direction to communicate the upward trend and illuminating the plurality of indicator lights in a second direction to communicate the downward trend.

In some variations, the cortisol monitoring device may be a skin-adhered patch.

In some variations, the cortisol monitoring device may include a plurality of solid microneedles, the plurality of solid microneedles including the at least one microneedle configured to sense cortisol. In some variations, the plurality of solid microneedles extend outwardly from the wearable housing in a direction opposite the user interface.

In some variations, the wearable housing may include a communication module operatively connected to the at least one microneedle and configured to transmit a wireless signal responsive to the sensor signal generated by the working electrode.

In some variations, a method for monitoring cortisol in a user may include accessing dermal interstitial fluid of the user with at least one solid microneedle, generating a sensor signal indicative of a concentration of the cortisol in the dermal interstitial fluid contacting the at least one microneedle, determining a user status based on the sensor signal, and selectively illuminating one or more indicator lights based on the user status. In some variations, the at least one solid microneedle may include a working electrode having a biorecognition layer arranged at least partially over an electrode material. In some variations, the biorecognition layer includes an aptamer that selectively binds to the cortisol, and the working electrode is configured to generate the sensor signal responsive to the cortisol binding the aptamer.

In some variations, at least one solid microneedle is at least one of a plurality of solid microneedles that extends outwardly from a wearable housing.

In some variations, one or more indicator lights illuminate in a spatial pattern and/or a temporal pattern based on the user status.

In some variations, the user status is a current cortisol level of the user. In some variations, the current cortisol level may be a current cortisol concentration in the dermal interstitial fluid or the bloodstream of the user. In some variations, the user status is a psychological state of the user, such as a degree of stress of the user.

In some variations, one or more indicator lights include a plurality of indicator lights, and the method may further include illuminating the one or more indicator lights in a progressive sequence responsive to an upward trend or a downward trend of the user status.

In some variations, the method further includes illuminating the plurality of indicator lights in a first direction to communicate the upward trend and illuminating the plurality of indicator lights in a second direction to communicate the downward trend.

In some variations, the method further includes transmitting a wireless signal based on the sensor signal or the user status.

Both the foregoing general description and the following detailed description are exemplary and explanatory only and are not restrictive. Further features and/or variations may be provided in addition to those set forth herein. For example, the implementations described herein may be directed to various combinations and sub-combinations of the disclosed features.

### BRIEF DESCRIPTION OF THE DRAWINGS

The accompanying drawings, which are incorporated in and constitute a part of this specification, show certain aspects of the subject matter disclosed herein and, together with the description, help explain some of the principles associated with the disclosed implementations. In the drawings,
FIG. 1 depicts an illustrative schematic of a cortisol monitoring system with a microneedle array.
FIG. 2A depicts an illustrative schematic of a cortisol monitoring device.
FIG. 2B depicts an illustrative schematic of microneedle insertion depth in a cortisol monitoring device.
FIGS. 3A-3C depict an upper perspective view, a side view, and a lower perspective view, respectively, of a cortisol monitoring device. FIG. 3D depicts a partially exploded view of the cortisol monitoring device shown in FIG. 3A including an adhesive layer. FIG. 3E depicts an exploded view of the cortisol monitoring device shown in FIG. 3A.
FIGS. 3F-3I depict an upper perspective view, a lower perspective view, a side view, and an exploded view, respectively, of a sensor assembly in a cortisol monitoring device.
FIG. 3J depicts a transparent side view of a sensor assembly in a cortisol monitoring device.
FIGS. 4A-4E depict a perspective view, a side view, a bottom view, a side cross-sectional view, and an upper perspective transparent view, respectively, of a cortisol monitoring device.
FIG. 5A depicts an illustrative schematic of a microneedle array. FIG. 5B depicts an illustrative schematic of a microneedle in the microneedle array depicted in FIG. 5A.
FIG. 6 depicts an illustrative schematic of a microneedle array used for sensing multiple analytes, of which at least one of the multiple analytes is cortisol.
FIG. 7A depicts a cross-sectional side view of a columnar microneedle having a tapered distal end. FIGS. 7B and 7C are images depicting perspective and detailed views, respectively, of an embodiment of the microneedle shown in FIG. 7A.
FIG. 8 depicts an illustrative schematic of a columnar microneedle having a tapered distal end.
FIG. 9 depicts a cross-sectional side view of a columnar microneedle having a tapered distal end.
FIG. 10 depicts an illustrative schematic of a columnar microneedle having a tapered distal end.
FIG. 11A depicts a cross-sectional side view of a pyramidal microneedle having a tapered distal end. FIG. 11B is an image depicting a perspective view of an embodiment of the microneedle shown in FIG. 11A. FIG. 11C is an image depicting an illustrative variation of a microneedle array including microneedles similar to that shown in FIG. 11B.
FIG. 12 depicts an illustrative schematic of a pyramidal microneedle having a tapered distal end.
FIG. 13A depicts an illustrative schematic of a pyramidal microneedle having a tapered distal end and asymmetric cut surface. FIG. 13B is an image depicting an illustrative variation of the microneedle shown in FIG. 13A.
FIGS. 13C-13E illustrate a process for forming the pyramidal microneedle shown in FIG. 13A.
FIG. 14A depicts an illustrative schematic of a columnar-pyramidal microneedle having a tapered distal end. FIG. 14B depicts a detailed view of the distal portion of the microneedle depicted in FIG. 14A.
FIGS. 15A-15D depict illustrative schematics of formation of conductive pathways within a microneedle array.
FIGS. 16A-16C depict illustrative schematics of layered structures of a working electrode, a counter electrode, and a reference electrode, respectively.
FIGS. 16D-16F depict illustrative schematics of layered structures of a working electrode, a counter electrode, and a reference electrode, respectively.
FIGS. 16G-16I depict illustrative schematics of layered structures of a working electrode, a counter electrode, and a reference electrode, respectively.
FIGS. 16J-16P depict illustrative schematics of layered structures of a working electrode in which the biorecognition element is a cortisol-binding aptamer.
FIG. 17 depicts an illustrative schematic of a microneedle array configuration.
FIGS. 18A and 18B depict perspective and orthogonal views, respectively, of an illustrative variation of a die including a microneedle array.
FIGS. 19A-19J depict illustrative schematics of different variations of microneedle array configurations.
FIG. 20 is an illustrative schematic of pairing between a cortisol monitoring device and a mobile computing device executing a mobile application.
FIGS. 21A and 21B depict illustrative schematics of a microneedle array and a microneedle, respectively. FIGS. 21C-21F depict detailed partial views of an illustrative variation of a microneedle.
FIGS. 22A and 22B depict an illustrative variation of a microneedle.
FIGS. 23A and 23B depict illustrative schematics of a microneedle array configuration.
FIGS. 24A and 24B depict illustrative schematics of a microneedle array configuration.
FIGS. 25A and 25B depict illustrative schematics of a housing of a cortisol monitoring device including a user interface with indicator light elements.
FIGS. 26A-26C depict illustrative schematics of illumination modes in a cortisol monitoring device for indicating cortisol measurement data.
FIGS. 27A-27D depict illustrative schematics of illumination modes in a cortisol monitoring device for indicating cortisol measurement data.
FIGS. 28A-28C depict illustrative schematics of illumination modes in a cortisol monitoring device for indicating cortisol measurement data.
FIGS. 29A and 29B depict illustrative schematics of illumination modes in a cortisol monitoring device for indicating device information (e.g., operational status, and/or fault modes).

### DETAILED DESCRIPTION

Non-limiting examples of various aspects and variations of the invention are described herein and illustrated in the accompanying drawings.

Although single-shot immunoassays are widely used in clinical practice for cortisol determinations in multiple physiological fluids, including those with minimal sample preparation (e.g., saliva), the development of wearable devices that can passively quantify cortisol in a continuous fashion remains an unmet need in multiple fields. To this end, contemporary research activity has explored excreted sweat cortisol as an intermediary for circulating cortisol. However, the viability of this compartment for real-time quantitative cortisol determination has been brought into question owing to a number of limitations implicated with analyte measurements in secreted, non-circulating fluids. These limitations include poor correlation with the clinical correlate in the circulatory system, hysteresis, large level of latency / lag time, and complexity associated with sample replenishment.

Variations of the current subject matter address the limitations of cortisol determination by providing an aptamer-based approach for measuring and monitoring cortisol in dermal interstitial fluid using a microneedle array. Before providing additional details regarding aspects of the aptamer-based approach for measuring and monitoring cortisol, the following provides a description of some examples of a cortisol monitoring device, including examples of a microneedle arrays, in which aspects of the current subject matter may be implemented. The following descriptions are meant to be exemplary, and aspects related to the aptamer-based approach for measuring and monitoring cortisol consistent with the current subject matter are not limited to the example cortisol monitoring device and the example microneedle arrays described herein.

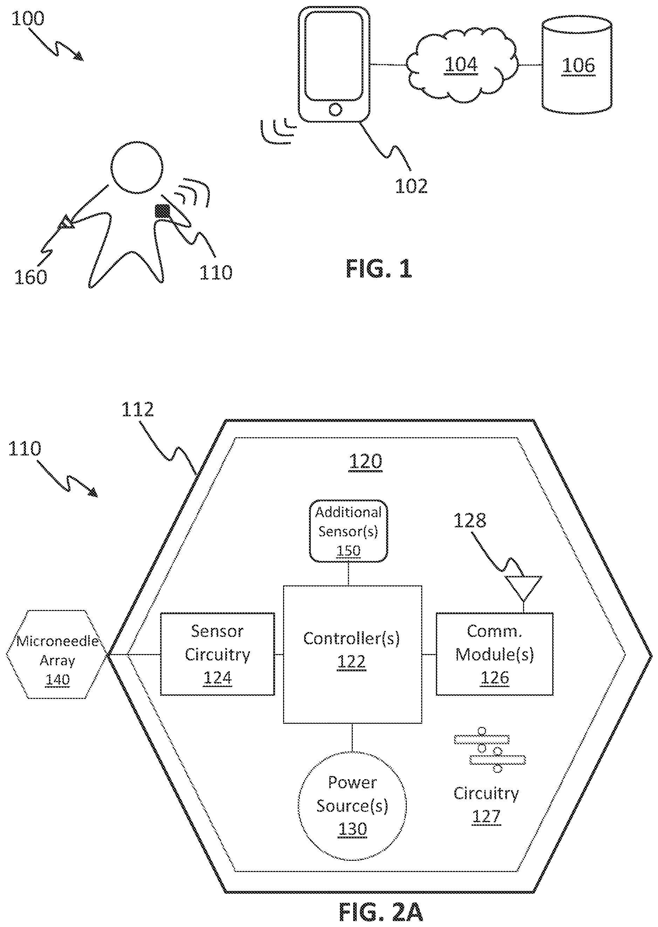

As generally described herein, a cortisol monitoring system may include a cortisol monitoring device that is worn by a user and includes one or more sensors for monitoring cortisol in a user. The sensors may, for example, include one or more electrodes configured to perform electrochemical detection of cortisol. The cortisol monitoring device may communicate sensor data to an external computing device for storage, display, and/or analysis of sensor data. For example, as shown in FIG. 1, a cortisol monitoring system 100 may include a cortisol monitoring device 110 that is worn by a user, and the cortisol monitoring device 110 may be a continuous cortisol monitoring device. The cortisol monitoring device 110 may include, for example, a microneedle array comprising at least one electrochemical sensor for detecting and/or measuring cortisol in body fluid of a user. In some variations, the cortisol monitoring device may be applied to the user using suitable applicator 160 or may be applied manually. The cortisol monitoring device 110 may include one or more processors for performing analysis on sensor data, and/or a communication module (e.g., wireless communication module) configured to communicate sensor data to a mobile computing device 102 (e.g., smartphone) or other suitable computing device. In some variations, the mobile computing device 102 may include one or more processors executing a mobile application to handle sensor data (e.g., displaying data, analyzing data for trends, etc.) and/or provide suitable alerts or other notifications related to the sensor data and/or analysis thereof. It should be understood that while in some variations the mobile computing device 102 may perform sensor data analysis locally, other computing device(s) may alternatively or additionally remotely analyze sensor data and/or communicate information related to such analysis with the mobile computing device 102 (or other suitable user interface) for display to the user. Furthermore, in some variations the mobile computing device 102 may be configured to communicate sensor data and/or analysis of the sensor data over a network 104 to one or more storage devices 106 (e.g., server) for archiving data and/or other suitable information related to the user of the cortisol monitoring device.

The cortisol monitoring devices described herein have characteristics that improve a number of properties that are advantageous for a continuous cortisol monitoring device. For example, the cortisol monitoring device described herein have improved sensitivity (amount of sensor signal produced per given concentration of cortisol), improved selectivity (rejection of endogenous and exogenous circulating compounds that can interfere with the detection of the cortisol), and improved stability to help minimize change in sensor response over time through storage and operation of the cortisol monitoring device. The cortisol monitoring devices described herein have a relatively brief warm-up time that enables the sensor(s) to quickly provide a stable sensor signal following implantation, as well as a rapid response time that enables the sensors(s) to quickly provide a stable sensor signal following a change in cortisol concentration in the user. Furthermore, as described in further detail below, the cortisol monitoring devices described herein may be applied to and function in a variety of wear sites and provides for pain-free sensor insertion for the user. Other properties such as biocompatibility and mechanical integrity are also optimized in the cortisol monitoring devices described herein.

Various aspects of example variations of the cortisol monitoring systems, and methods of use thereof, are described in further detail below.

### Cortisol monitoring device

As shown in FIG. 2A, in some variations, a cortisol monitoring device 110 may generally include a housing 112 and a microneedle array 140 extending outwardly from the housing. The housing 112, may, for example, be a wearable housing configured to be worn on the skin of a user such that the microneedle array 140 extends at least partially into the skin of the user. For example, the housing 112 may include an adhesive such that the cortisol monitoring device 110 is a skin-adhered patch that is simple and straightforward for application to a user. The microneedle array 140 may be configured to puncture the skin of the user and include one or more electrochemical sensors (e.g., electrodes) configured for measuring cortisol that is accessible after the microneedle array 140 punctures the skin of the user. In some variations, the cortisol monitoring device 110 may be integrated or self-contained as a single unit, and the unit may be disposable (e.g., used for a period of time and replaced with another instance of the cortisol monitoring device 110).

An electronics system 120 may be at least partially arranged in the housing 112 and include various electronic components, such as sensor circuitry 124 configured to perform signal processing (e.g., biasing and readout of electrochemical sensors, converting the analog signals from the electrochemical sensors to digital signals, etc.). The electronics system 120 may also include at least one microcontroller 122 for controlling the cortisol monitoring device 110, at least one communication module 126, at least one power source 130, and/or other various suitable passive circuitry 127. The microcontroller 122 may, for example, be configured to interpret digital signals output from the sensor circuitry 124 (e.g., by executing a programmed routine in firmware), perform various suitable algorithms or mathematical transformations (e.g., calibration, etc.), and/or route processed data to and/or from the communication module 126. In some variations, the communication module 126 may include a suitable wireless transceiver (e.g., Bluetooth transceiver or the like) for communicating data with an external computing device 102 via one or more antennas 128. For example, the communication module 126 may be configured to provide uni-directional and/or bi-directional communication of data with an external computing device 102 that is paired with the cortisol monitoring device 110. The power source 130 may provide power for the cortisol monitoring device 110, such as for the electronics system. The power source 130 may include battery or other suitable source, and may, in some variations, be rechargeable and/or replaceable. Passive circuitry 127 may include various non-powered electrical circuitry (e.g., resistors, capacitors, inductors, etc.) providing interconnections between other electronic components, etc. The passive circuitry 127 may be configured to perform noise reduction, biasing and/or other purposes, for example. In some variations, the electronic components in the electronics system 120 may be arranged on one or more printed circuit boards (PCB), which may be rigid, semi-rigid, or flexible, for example. Additional details of the electronics system 120 are described further below.

In some variations, the cortisol monitoring device 110 may further include one or more additional sensors 150 to provide additional information that may be relevant for user monitoring. For example, the cortisol monitoring device 110 may further include at least one temperature sensor (e.g., thermistor) configured to measure skin temperature, thereby enabling temperature compensation for the sensor measurements obtained by the microneedle array electrochemical sensors.

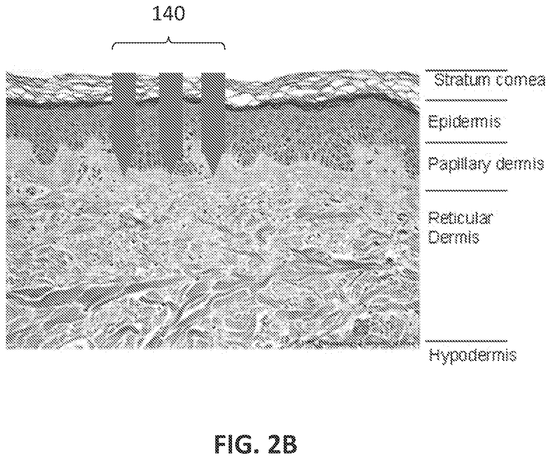

In some variations, the microneedle array 140 in the cortisol monitoring device 110 may be configured to puncture skin of a user. As shown in FIG. 2B, when the device 110 is worn by the user, the microneedle array 140 may extend into the skin of the user such that electrodes on distal regions of the microneedles rest in the dermis. Specifically, in some variations, the microneedles may be designed to penetrate the skin and access the upper dermal region (e.g., papillary dermis and upper reticular dermis layers) of the skin, in order to enable the electrodes to access interstitial fluid that surrounds the cells in these layers. For example, in some variations, the microneedles may have a height generally ranging between at least 350 µm and about 515 µm. In some variations, one or more microneedles may extend from the housing such that a distal end of the electrode on the microneedle is located less than about 5 mm from a skin-interfacing surface of the housing, less than about 4 mm from the housing, less than about 3 mm from the housing, less than about 2 mm from the housing, or less than about 1 mm from the housing.

In contrast to traditional continuous monitoring devices, which include sensors typically implanted between about 8 mm and about 10 mm beneath the skin surface in the subcutis or adipose layer of the skin, the cortisol monitoring device 110 has a shallower microneedle insertion depth of about 0.25 mm (such that electrodes are implanted in the upper dermal region of the skin) that provides numerous benefits. These benefits include access to dermal interstitial fluid including cortisol for detection, which is advantageous at least because cortisol measurements of dermal interstitial fluid have been found to closely correlate to those of blood.

Furthermore, when the microneedle array rests in the upper dermal region, the lower dermis beneath the microneedle array includes very high levels of vascularization and perfusion to support the dermal metabolism, which enables thermoregulation (via vasoconstriction and/or vasodilation) and provides a barrier function to help stabilize the sensing environment around the microneedles. Yet another advantage of the shallower insertion depth is that the upper dermal layers lack pain receptors, thus resulting in a reduced pain sensation when the microneedle array punctures the skin of the user, and providing for a more comfortable, minimally-invasive user experience.

Thus, the cortisol monitoring devices and methods described herein enable improved continuous monitoring of cortisol of a user. For example, as described above, the cortisol monitoring device may be simple and straightforward to apply, which improves ease-of-use and user compliance. Additionally, cortisol measurements of dermal interstitial fluid may provide for highly accurate cortisol detection. Furthermore, compared to traditional continuous cortisol monitoring devices, insertion of the microneedle array and its sensors may be less invasive and involve less pain for the user. Additional advantages of other aspects of the cortisol monitoring devices and methods are further described below.

### Housing

As described above, a cortisol monitoring device may include a housing. The housing may at least partially surround or enclose other components of the cortisol monitoring device (e.g., electronic components), such as for protection of such components. For example, the housing may be configured to help prevent dust and moisture from entering the cortisol monitoring device. In some variations, an adhesive layer may attach the housing to a surface (e.g., skin) of a user, while permitting a microneedle array to extend outwardly from the housing and into the skin of the user. Furthermore, in some variations the housing may generally include rounded edges or corners and/or be low-profile so as to be atraumatic and reduce interference with clothing, etc. worn by the user.

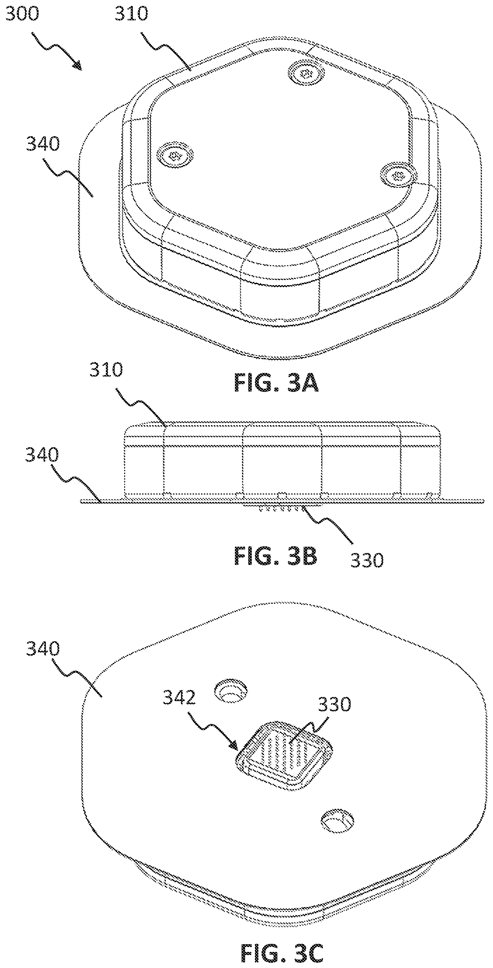

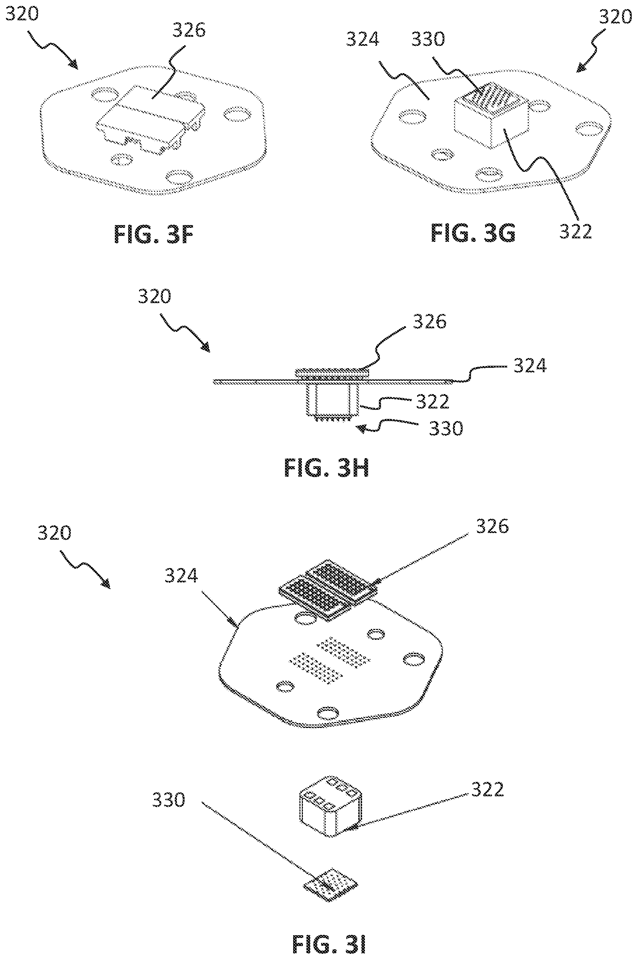

For example, as shown in FIGS. 3A-3E, an example variation of a cortisol monitoring device 300 may include a housing 310 configured to at least partially surround other various internal components of the device 300, and a microneedle array 330 that extends outwardly from a skin-facing surface (e.g., underside) of the housing 310.

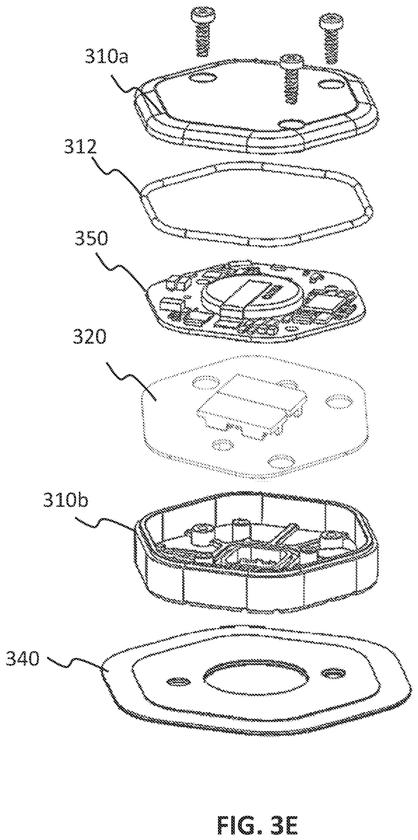

The housing 310 may, for example, include one or more rigid or semi-rigid protective shell components that may couple together via suitable fasteners (e.g., mechanical fasteners), mechanically interlocking or mating features, and/or an engineering fit. For example, as shown in FIG. 3E, the housing may include a housing cover 310a and a housing base 310b, where the cover 310a and the base 310b may be secured together with one or more threaded fasteners (e.g., fasteners that engage threaded holes in the upper and/or lower housing portions). The cover 310a and the base 310b may include radiused edges and corners, and/or other atraumatic features. When coupled together, the cover 310a and the base 310b may form an internal volume that houses other internal components such as a device printed circuit board 350 (PCB), a sensor assembly 320, and/or other components such as a gasket 312. For example, the internal components arranged in the internal volume may be arranged in a compact, low profile stack-up as shown in FIG. 3E. While FIG. 3E illustrates a housing 310 include multiple housing components, in some variations the housing 310 may include a single component defining the internal volume for housing internal device components. In some embodiments, the housing 310 may be filled with a suitable potting compound (e.g., epoxy) to reduce deleterious environmental effects such as temperature, humidity, pressure, and light.

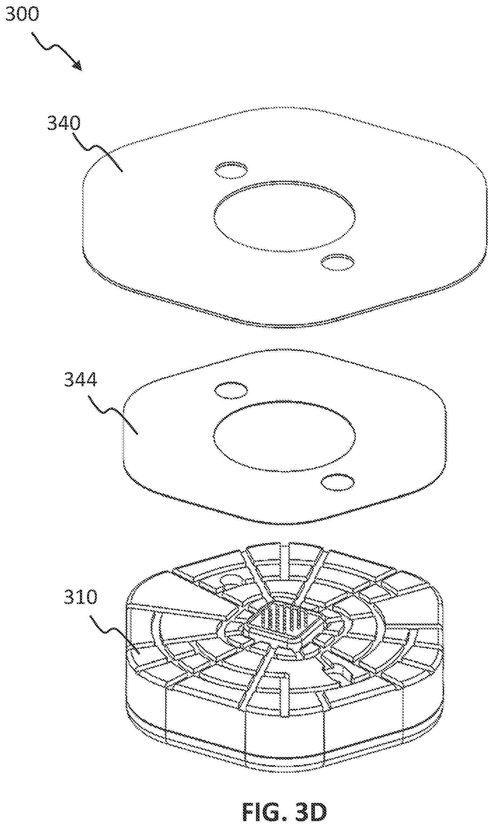

Furthermore, the cortisol monitoring device 300 may include an adhesive layer 340 configured to attach the housing 310 to a surface (e.g., skin) of a user. The adhesive layer 340 may, for example, be attached to a skin-facing side of the housing 310 via a double-sided adhesive liner 344 as shown in in the variation depicted in FIG. 3D. Alternatively, the adhesive layer 340 may be coupled directly to the skin-facing side of the housing 310 with one or more suitable fasteners (e.g., adhesive, mechanical fasteners, etc.). The adhesive layer 340 may be protected by a release liner that the user removes prior to skin application, in order to expose the adhesive. In some variations, the cortisol monitoring device may include 3M^{®} 1504XL^{™} double-sided adhesive and 3M^{®} 4076^{™} skin-facing adhesive, available from 3M^{®}. These materials are selected for their: breathability, wearability, mean water vapor transmission rate (MWVTR), biocompatibility, compatibility with sensor sterilization method / strategy, appearance, durability, tackiness, and ability to retain said tackiness for the duration of sensor wear.

The adhesive layer 340 may, in some variations, have a perimeter that extends farther than the perimeter or periphery of the housing 310 (e.g., which may increase surface area for attachment and increase stability of retention, or the attachment to the skin of a user). Furthermore, in some variations, the adhesive layer 340 may include an opening 342 that permits passage of the outwardly extending microneedle array 330. The opening 342 may closely circumscribe the shape of the microneedle array 330 as shown in FIG. 3C (e.g., square opening closely corresponding in size and shape to a square microneedle array), or have another suitable size and shape that is larger than the footprint area of the microneedle array (e.g., circular opening larger than a square microneedle array).

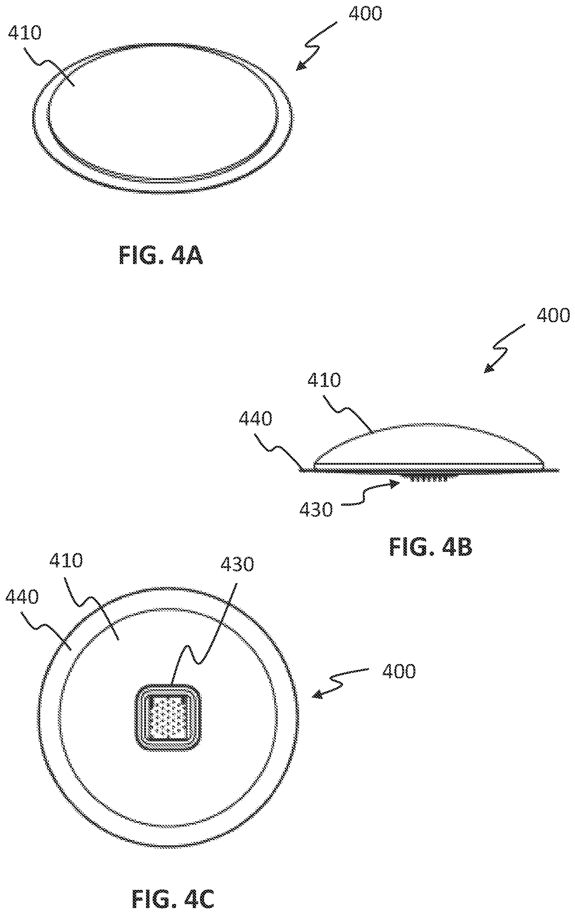

Although the housing 310 depicted in FIGS. 3A-3E is hexagonal shaped and generally prismatic, it should be understood that in other variations, the housing 310 may have any suitable shape. For example, in other variations the housing may be generally prismatic and have a base that has an elliptical (e.g., circular), triangular, rectangular, pentagonal, or other suitable shape. As another example, FIGS. 4A-4C illustrate an example variation of a cortisol monitoring device 400 including a dome-shaped housing 410. While the dome-shaped housing 410 depicted in FIGS. 4A-4C is generally circular, in other variations the dome-shaped housing may have a base that has another suitable elliptical shape or polygonal shape.

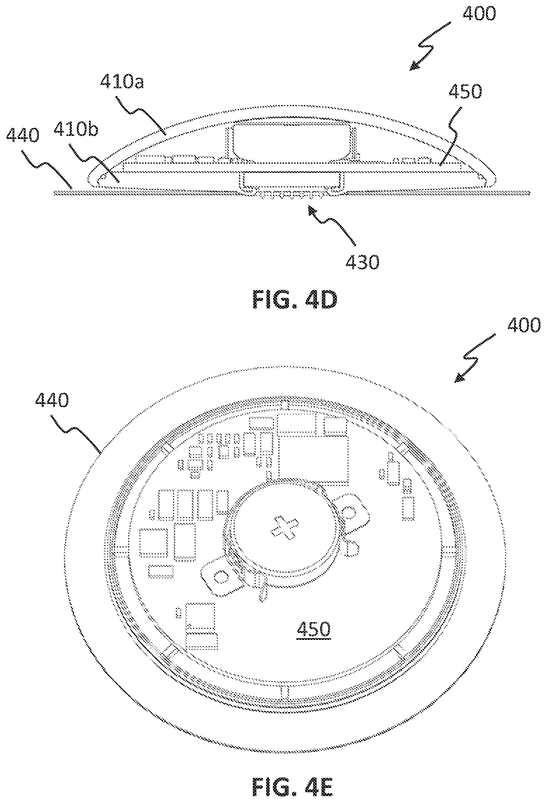

Similar to the housing 310, the housing 410 may include an internal volume configured to at least partially surround other components of the cortisol monitoring device 400. For example, as shown in the cross-sectional view of FIG. 4D, the housing 410 may include a domed cover 410a coupled to a base 410b, so as to form an internal volume within which a device PCB 450 and a sensor assembly with a microneedle array 430 may be arranged. Additionally, the housing 410 may be configured to couple to a surface via an adhesive layer 440, and the microneedle array 430 may extend outwardly from the housing and beyond the adhesive layer 440. Furthermore, as shown in FIGS. 4D and 4E, the adhesive layer 440 may extend beyond the perimeter of the housing 410.

### User interface

In some variations, a cortisol monitoring system may provide user status, cortisol monitoring device status, and/or other suitable information directly via a user interface (e.g., display, indicator lights, etc. as described below) on the cortisol monitoring device. Thus, in contrast to cortisol monitoring systems that may solely communicate information to a separate peripheral device (e.g., mobile phone, etc.) that in turn communicates the information to a user, in some variations such information may be directly provided by the cortisol monitoring device. Advantageously, in some variations, such a user interface on the cortisol monitoring device may reduce the need for a user to constantly maintain a separate peripheral device in order to monitor user status and/or cortisol monitoring device status (which may be impractical due to cost, inconvenience, etc.). Additionally, the user interface on the cortisol monitoring device may reduce risks associated with loss of communication between the cortisol monitoring device and a separate peripheral device, such as a user having an inaccurate understanding of their current cortisol levels (e.g., leading the user to assume their cortisol levels are high when they are actually low, which could, for example, result in the user self-administering an inaccurate dose of drug or withholding a therapeutic intervention when it is medically necessary).

Additionally, the ability to communicate information to a user via the cortisol monitoring device itself, independently of a separate peripheral device, may reduce or eliminate the need to maintain compatibility between the cortisol monitoring device and separate peripheral devices as such peripheral devices are upgraded (e.g., replaced with new device models or other hardware, run new versions of operating systems or other software, etc.).

Accordingly, in some variations, the housing may include a user interface, such as an interface to provide information in a visual, audible, and/or tactile manner to provide information regarding user status based on cortisol measurements and/or status of the cortisol monitoring device, and/or other suitable information.

Examples of user status based on cortisol measurements that may be communicated via the user interface include information representative of cortisol measurement in the user, such as: cortisol concentration in a bodily fluid such as dermal interstitial fluid or bloodstream; the cortisol measurements being below a predetermined cortisol measurement threshold or range, within a predetermined cortisol measurement range, or above a predetermined cortisol measurement threshold or range; increase or decrease of cortisol measurement over time; rate of change of cortisol measurement; cortisol variability indicating a standard deviation of cortisol measurements during a time period; information relating to trends of cortisol measurements; and/or other suitable alerts associated with cortisol measurement.

Examples of cortisol monitoring device status that may be communicated via the user interface include device operation mode (e.g., associated with device warm-up state, cortisol monitoring state, battery power status such as low battery, etc.), a device error state (e.g., operational error, pressure-induced sensing attenuation, fault, failure mode, etc.), device power status, device life status (e.g., anticipated sensor end-of-life), status of connectivity between device and a mobile computing device, and/or the like.

In some variations, the user interface may by default be in an enabled or "on" state to communicate such information at least whenever the cortisol monitoring device is performing cortisol measurements) or whenever the cortisol monitoring device is powered on, thereby helping to ensure that information is continuously available to the user. For example, user interface elements may communicate through a display or indicator light(s) (e.g., as described below) not only alerts to flag user attention or recommend remedial action, but also when user status and/or device status are normal. Accordingly, in some variations, a user is not required to perform an action to initiate a scan to learn their current cortisol measurement level(s), and such information may always readily be available to the user. In some variations, however, a user may perform an action to disable the user interface temporarily (e.g., similar to a "snooze" button) such as for a predetermined amount of time (e.g., 30 minutes, 1 hour, 2 hours, etc.) after which the user interface is automatically reenabled, or until a second action is performed to reenable the user interface.

In some variations, the user interface of the housing may include a display configured to visually communicate information. The display may, for example, include a display screen (e.g., LCD screen, OLED display, electrophoretic display, electrochromic display, etc.) configured to display alphanumeric text (e.g., numbers, letters, etc.), symbols, and/or suitable graphics to communicate information to the user. For example, the display screen may include a numerical information, textual information, and/or a graphics (e.g., sloped line, arrows, etc.) of information such as user status and/or status of the cortisol monitoring device. For example, the display screen may include text or graphical representations of cortisol measurement levels, trends, and/or recommendations. For example, the display screen may include text and/or graphical representations related to recommendations for physical activity, meditation, rest, food, dietary supplements, and/or medical consultation.

As another example, the display on the housing may include one or more indicator lights (e.g., including LEDs, OLEDs, lasers, electroluminescent material, or other suitable light source, waveguides, etc.) that may be controlled in one or more predetermined illumination modes to communicate different statuses and/or other suitable information. An indicator light may be controlled to illuminate with multiple colors (e.g., red, orange, yellow, green, blue, and/or purple, etc.) or in only one color. For example, an indicator light may include a multicolored LED. As another example, an indicator light may include a transparent or semitransparent material (e.g., acrylic) positioned over one or more different-colored light sources (e.g., LED) such that different-colored light sources may be selectively activated to illuminate the indicator light in a selected color. The activation of light sources can either occur simultaneously or in sequence. An indicator light may have any suitable form (e.g., raised, flush, recessed, etc. from housing body) and/or shape (e.g., circle or other polygon, ring, elongated strip, etc.). In some variations, an indicator light may have a pinhole size and/or shape to present the same intensity of the light as a larger light source, but with significantly less power requirements, which may help conserve onboard power in the cortisol monitoring device.

Indicator light(s) on the display may be illuminated in one or more various manners to communicate different kinds of information. For example, an indicator light may be selectively illuminated on or off to communicate information (e.g., illumination "on" indicates one status, while illumination "off" indicates another status). Additionally or alternatively, an indicator light may be illuminated in a selected color or intensity to communicate information (e.g., illumination in a first color or intensity indicates a first status, while illumination in a second color or intensity indicates a second status). Additionally or alternatively, an indicator light may be illuminated in a selected temporal pattern to communicate information (e.g., illumination in a first temporal pattern indicates a first status, while illumination in a second temporal pattern indicates a second status). For example, an indicator light may be selectively illuminated in one of a plurality of predetermined temporal patterns that differ in illumination frequency (e.g., repeated illumination at a rapid or slow frequency), regularity (e.g., periodic repeated illumination vs. intermittent illumination), duration of illumination "on" time, duration of illumination "off" time, rate of change in illumination intensity, duty cycle (e.g., ratio of illumination "on" time to illumination "off" time), and/or the like, where each predetermined temporal pattern may indicate a respective status.

Additionally or alternatively, in some variations, a display may include multiple indicator lights that may be collectively illuminated in one or more predetermined illumination modes or sequences in accordance with one or more predetermined spatial and/or temporal patterns. For example, in some variations, some or all of the indicator lights arranged on a display may be illuminated in synchrony or in sequence to indicate a particular status. Accordingly, the selected subset of indicator lights (e.g., the spatial arrangement of the indicator lights that are illuminated) and/or the manner in which they are illuminated (e.g., illumination order, illumination rate, etc.) may indicate a particular status. Additionally or alternatively, a plurality of indicator lights may illuminate simultaneously or in sequence to increase the diversity of the color palette. For example, in some variations, red, green, and blue LEDs may be illuminated in rapid succession to create the impression of white light to a user.

It should furthermore be understood that one or more of the above-described illumination modes may be combined in any suitable manner (e.g., combination of varying color, intensity, brightness, luminosity, contrast, timing, location, etc.) to communicate information. Additionally or alternatively, an ambient light sensor may be incorporated into the device body to enable dynamic adjustment light levels in the indicator light(s) to compensate for environmental light conditions to help conserve power. The ambient light sensor may, in some variations, be used in conjunction with a kinetic sensor (e.g., as described in further detail below) to further determine appropriate periods for the cortisol monitoring device to enter into a power saving mode or reduced power state. For example, detection of darkness and no motion of the cortisol monitoring device may indicate that the wearer of the cortisol monitoring device is asleep, which may trigger the cortisol monitoring device to enter into a power saving mode or reduced power state.

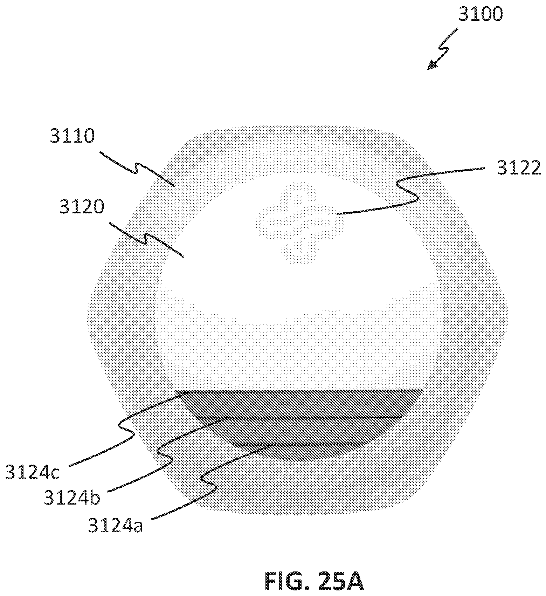

FIG. 25A illustrates an example variation of a cortisol monitoring device 3100 including a user interface 3120 with multiple indicator lights (3122, 3124a-3124c). Indicator light 3122 may, for example, be selectively illuminated to indicate a device state (e.g., operation mode, error state, power status, life status, etc.). Although indicator light 3122 is in the shape of a symbol (e.g., logo), it should be understood that in other variations, the indicator light 3122 may have any suitable shape (e.g., text, other geometric shape, etc.). Indicator lights 3124a, 3124b, 3124c may be selectively illuminated to indicate a user status (e.g., information representative of cortisol measurement). Although indicator lights 3124a, 3124b, 3124c are linear elements extending across the user interface (e.g., chords across a circular display), it should be understood that in other variations, the indicator lights 3124a, 3124b, 3124c have other suitable shapes (e.g., wavy lines, circular, etc.). In some variations, a 1-dimensional array of indicator lights of any suitable shape may be arranged on the housing (e.g., arranged in a row, a column, an arc, etc.). Alternatively, the housing may include a multi-dimensional array of indicator lights of any suitable shape.

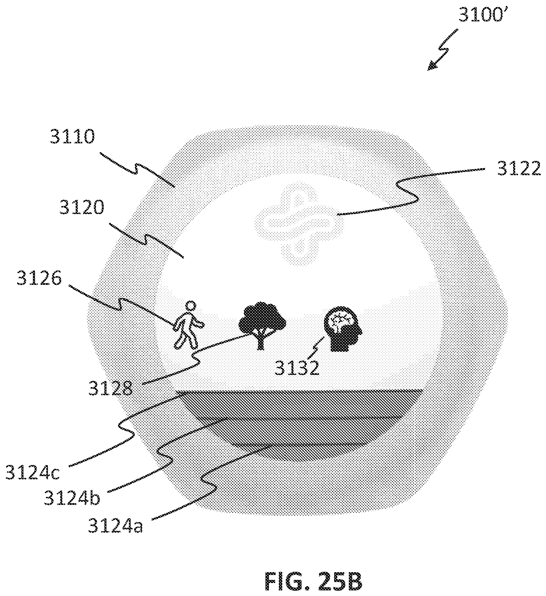

Furthermore, in some variations, an indicator light may include an icon (e.g., symbol) that may be indicative of cortisol information (e.g., up arrow to indicate rising cortisol measurement level trend, down arrow to indicate falling cortisol measurement level trend), cortisol monitoring device status (e.g., exclamation point to indicate a device error state), and/or other suitable information. Additionally or alternatively, iconography in the indicator light(s) may be used to communicate recommendations for the user such as behavioral recommendations. Iconography may, for example, have the advantage of communicating recommendations to a user in a more universal or language-agnostic manner (e.g., without the need for language translations to tailor the device to different geographical regions or user preferences, etc.). In an example, rising cortisol levels may be correlated to an increase in user stress. For example, as shown in FIG. 25B, in some variations, a user interface for a cortisol monitoring device 3100' may include a running person icon 3126 to indicate a recommendation that the user engage in physical activity, a tree icon 3218 to indicate a recommendation that the user step outside, and/or a thinking head icon 3132 to indicate a recommendation that the user meditate.

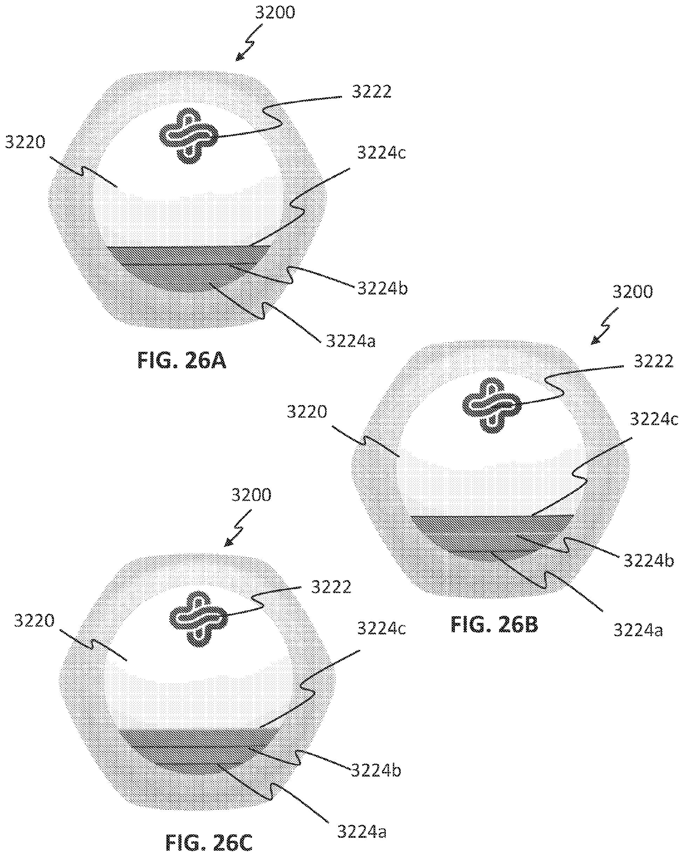

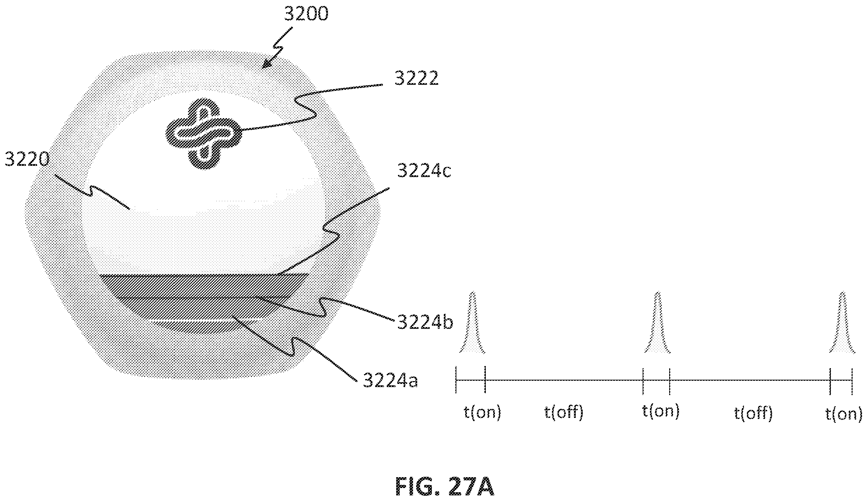

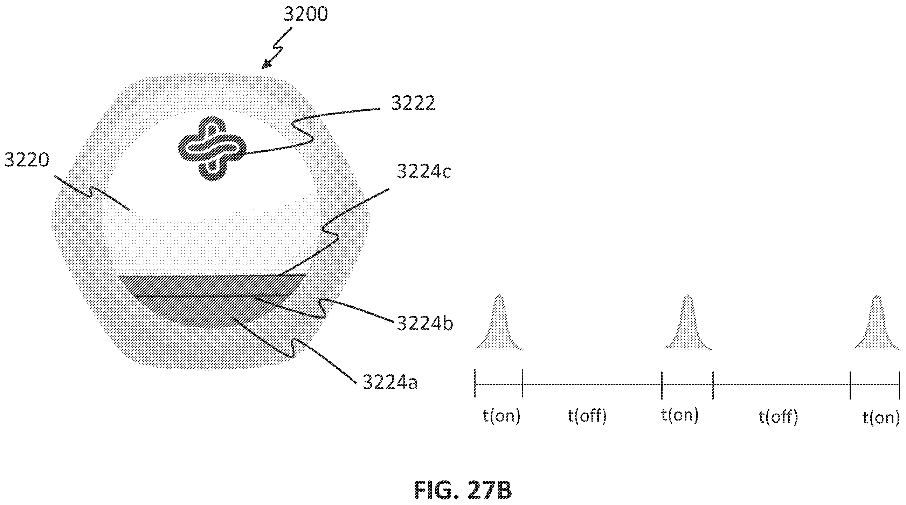

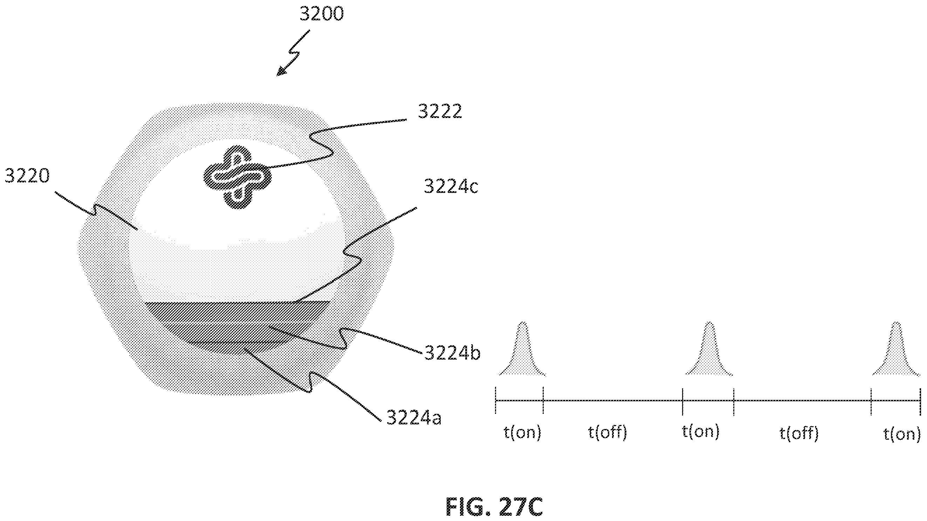

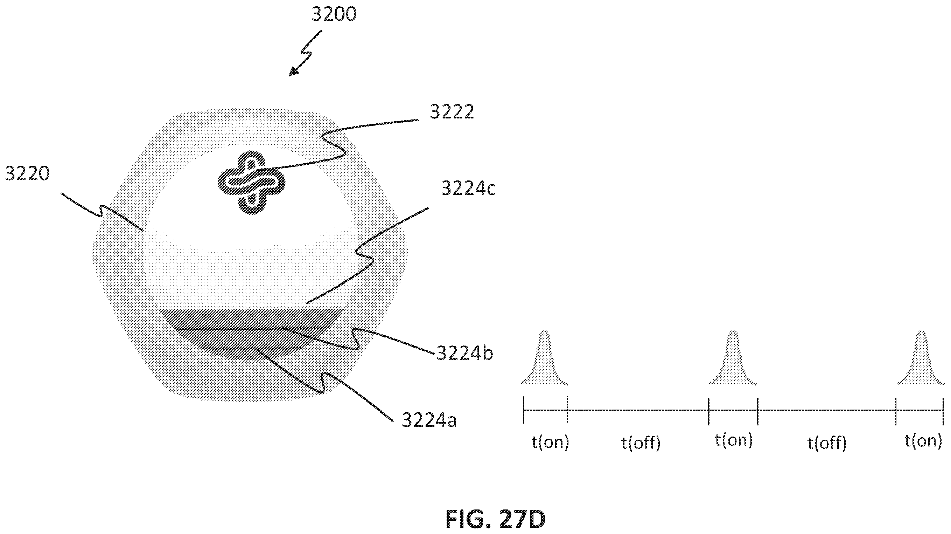

In the variations shown in FIGS. 25A and 25B, each of the indicator lights 3124a, 3124b, 3124c may be exclusively illuminated to indicate a different cortisol measurement (e.g., in target range, below target range, significantly below target range, above target range, significantly above target range, etc.). Furthermore, the indicator lights 3124a, 3124b, 3124c may be arranged adjacent to each other, such that they may be selectively illuminated in a progressive sequence to communicate trend information of cortisol measurements (e.g., progressive sequence of illumination in a first direction that corresponds to an increase in measured quantity of cortisol, progressive sequence of illumination in a second direction that corresponds to a decrease in measured quantity of cortisol, pace of illumination progression in the first direction or the second direction that corresponds to a rate of increase or decrease in measured quantity of cortisol, etc.). An example of such progressive sequence of illumination is further described below with reference to FIGS. 27A-27D. While one device status indicator light 3122 and three user status indicator lights 3124a, 3124b, 3124c are shown in FIGS. 25A and 25B, it should be understood that in other variations, a cortisol monitoring device may include any suitable number of indicator lights, such as one, two, three, four, five or more device status indicator lights, and one, two, three, four, five or more user status indicator lights. Further details regarding an example operation of the user interface 3120 to communicate device status and/or user status are described below (e.g., with reference to FIGS. 26A-26C, 27A-27D, 28A-28C, and 29A-29B).

### Microneedle array

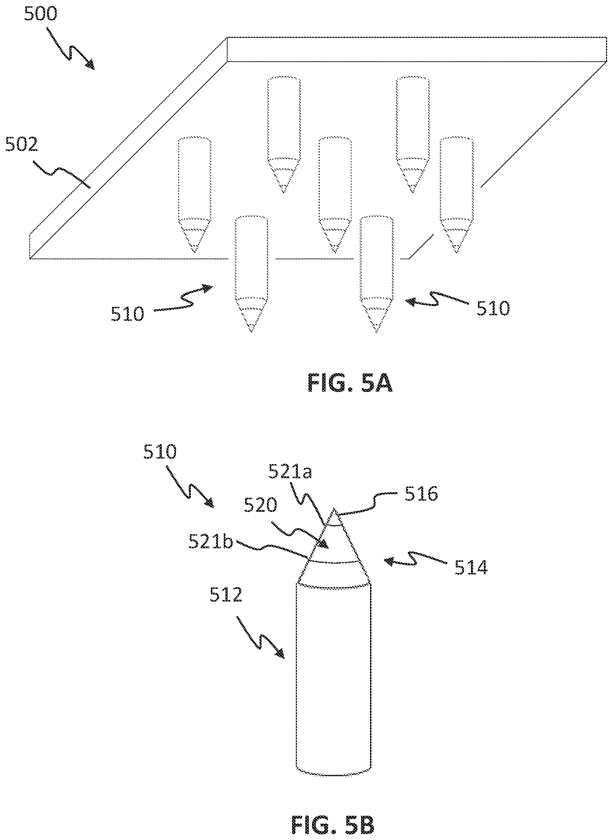

As shown in the schematic of FIG. 5A, in some variations, a microneedle array 510 for use in sensing cortisol may include one or more microneedles 510 projecting from a substrate surface 502. The substrate surface 502 may, for example, be a generally planar semiconductor (e.g. Silicon) substrate and one or more microneedles 510 may project orthogonally from the planar surface. Generally, as shown in FIG. 5B, a microneedle 510 may include a body portion 512 (e.g., shaft) and a tapered distal portion 514 configured to puncture skin of a user. In some variations, the tapered distal portion 514 may terminate in an insulated distal apex 516. The microneedle 510 may further include an electrode 520 on a surface of the tapered distal portion. In some variations, electrode-based measurements may be performed at the interface of the electrode and interstitial fluid located within the body (e.g., on an outer surface of the overall microneedle). In some variations, the microneedle 510 may have a solid core (e.g., solid body portion), though in some variations the microneedle 510 may include one or more lumens, which may be used for drug delivery or sampling of the dermal interstitial fluid, for example. Other microneedle variations, such as those described below, may similarly either include a solid core or one or more lumens.

The microneedle array 500 may be at least partially formed from a semiconductor (e.g., silicon) substrate and include various material layers applied and shaped using various suitable microelectromechanical systems (MEMS) manufacturing techniques (e.g., deposition and etching techniques), as further described below. The microneedle array may be reflow-soldered to a circuit board, similar to a typical integrated circuit. Furthermore, in some variations the microneedle array 500 may include a three electrode setup including a working (sensing) electrode having an electrochemical sensing coating (including a biorecognition element such as an enzyme) that enables detection of cortisol, a reference electrode, and a counter electrode. In other words, the microneedle array 500 may include at least one microneedle 510 that includes a working electrode, at least one microneedle 510 including a reference electrode, and at least one microneedle 510 including a counter electrode. Additional details of these types of electrodes are described in further detail below.

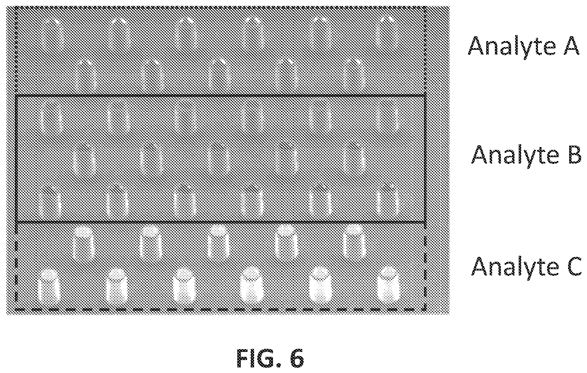

In some variations, the microneedle array 500 may include a plurality of microneedles that are insulated such that the electrode on each microneedle in the plurality of microneedles is individually addressable and electrically isolated from every other electrode on the microneedle array. The resulting individual addressability of the microneedle array 500 may enable greater control over each electrode's function, since each electrode may be separately probed. For example, the microneedle array 500 may be used to provide multiple independent measurements of cortisol, which improves the device's sensing reliability and accuracy. Furthermore, in some variations the electrodes of multiple microneedles may be electrically connected to produce augmented signal levels. As another example, the same microneedle array 500 may additionally or alternatively be interrogated to simultaneously measure multiple analytes to provide a more comprehensive assessment of physiological status. For example, as shown in the schematic of FIG. 6, a microneedle array may include a portion of microneedles to detect cortisol, a second portion of microneedles to detect a second Analyte B, and a third portion of microneedles to detect a third Analyte C. It should be understood that the microneedle array may be configured to detect any suitable number of analytes (e.g., 1, 2, 3, 4, 5 or more, etc.), provided that at least one of the analytes is cortisol.

In some variations of microneedles (e.g., microneedles with a working electrode), the electrode 520 may be located proximal to the insulated distal apex 516 of the microneedle. In other words, in some variations the electrode 520 does not cover the apex of the microneedle. Rather, the electrode 520 may be offset from the apex or tip of the microneedle. The electrode 520 being proximal to or offset from the insulated distal apex 516 of the microneedle advantageously provides more accurate sensor measurements. For example, this arrangement prevents concentration of the electric field at the microneedle apex 516 during manufacturing, thereby avoiding non-uniform electro-deposition of sensing chemistry on the electrode surface 520 that would result in faulty sensing. The electrode 520 may be configured to have an annular shape and may comprise a distal edge 521a and a proximal edge 521b.

As another example, placing the electrode 520 offset from the microneedle apex further improves sensing accuracy by reducing undesirable signal artefacts and/or erroneous sensor readings caused by stress upon microneedle insertion. The distal apex of the microneedle is the first region to penetrate into the skin, and thus experiences the most stress caused by the mechanical shear phenomena accompanying the tearing or cutting of the skin. If the electrode 520 were placed on the apex or tip of the microneedle, this mechanical stress may delaminate the electrochemical sensing coating on the electrode surface when the microneedle is inserted, and/or cause a small yet interfering amount of tissue to be transported onto the active sensing portion of the electrode. Thus, placing the electrode 520 sufficiently offset from the microneedle apex may improve sensing accuracy. For example, in some variations, a distal edge 521a of the electrode 520 may be located at least about 10 µm (e.g., between about 20 µm and about 30 µm) from the distal apex or tip of the microneedle, as measured along a longitudinal axis of the microneedle.

The body portion 512 of the microneedle 510 may further include an electrically conductive pathway extending between the electrode 520 and a backside electrode or other electrical contact (e.g., arranged on a backside of the substrate of the microneedle array). The backside electrode may be soldered to a circuit board, enabling electrical communication with the electrode 520 via the conductive pathway. For example, during use, the in-vivo sensing current (inside the dermis) measured at a working electrode is interrogated by the backside electrical contact, and the electrical connection between the backside electrical contact and the working electrode is facilitated by the conductive pathway. In some variations, this conductive pathway may be facilitated by a metal via running through the interior of the microneedle body portion (e.g., shaft) between the microneedle's proximal and distal ends. Alternatively, in some variations the conductive pathway may be provided by the entire body portion being formed of a conductive material (e.g., doped silicon). In some of these variations, the complete substrate on which the microneedle array 500 is built upon may be electrically conductive, and each microneedle 510 in the microneedle array 500 may be electrically isolated from adjacent microneedles 510 as described below. For example, in some variations, each microneedle 510 in the microneedle array 500 may be electrically isolated from adjacent microneedles 510 with an insulative barrier including electrically insulative material (e.g., dielectric material such as silicon dioxide) that surrounds the conductive pathway extending between the electrode 520 and backside electrical contact. For example, body portion 512 may include an insulative material that forms a sheath around the conductive pathway, thereby preventing electrical communication between the conductive pathway and the substrate. Other example variations of structures enabling electrical isolation among microneedles are described in further detail below.

Such electrical isolation among microneedles in the microneedle array permits the sensors to be individually addressable. This individually addressability advantageously enables independent and parallelized measurement among the sensors, as well as dynamic reconfiguration of sensor assignment (e.g., to different analytes). In some variations, the electrodes in the microneedle array can be configured to provide redundant cortisol measurements, which is an advantage over conventional cortisol monitoring devices. For example, redundancy can improve performance by improving accuracy (e.g., averaging multiple cortisol measurement values from different microneedles which reduces the effect of extreme high or low sensor signals on the determination of cortisol levels) and/or improving reliability of the device by reducing the likelihood of total failure.

In some variations, as described in further detail below with respective different variations of the microneedle, the microneedle array may be formed at least in part with suitable semiconductor and/or MEMS fabrication techniques and/or mechanical cutting or dicing. Such processes may, for example, be advantageous for enabling large-scale, cost-efficient manufacturing of microneedle arrays.

### Microneedle structures

Described herein are multiple example variations of microneedle structures incorporating one or more of the above-described microneedle features for a microneedle array in a cortisol monitoring device.

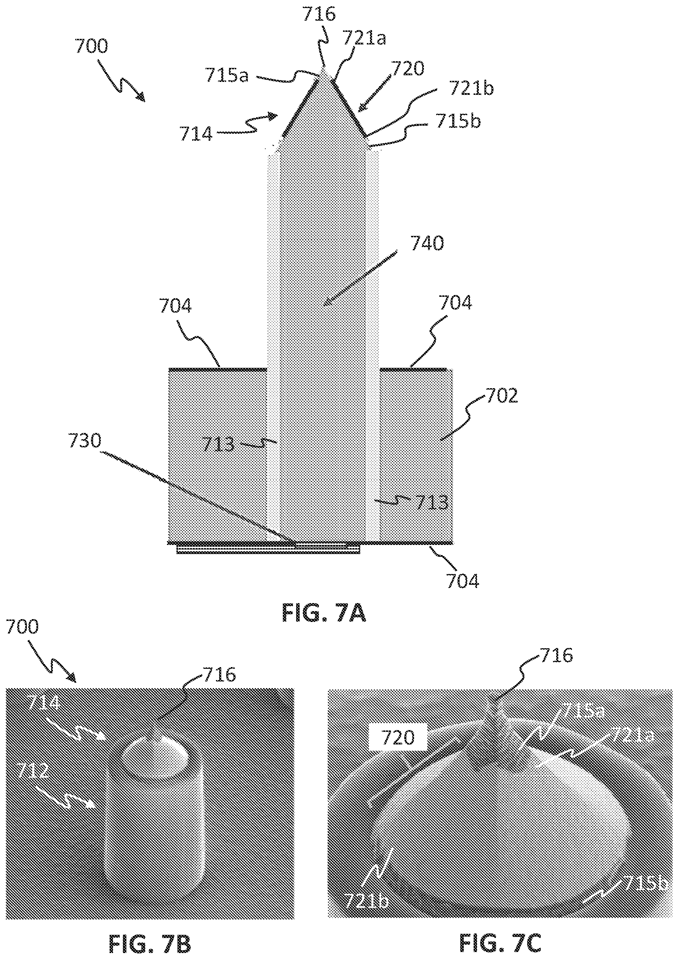

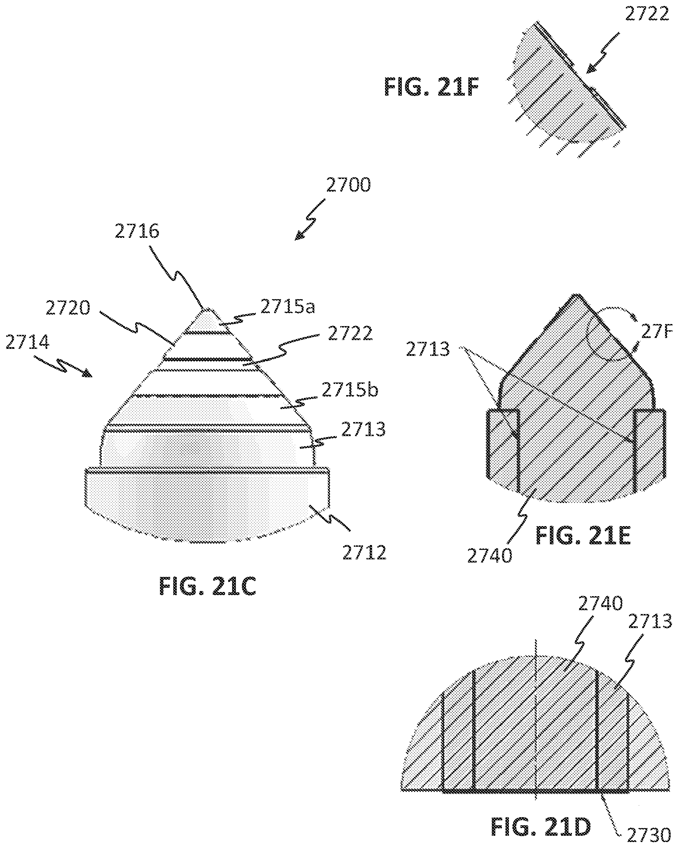

In some variations, a microneedle may have a generally columnar body portion and a tapered distal portion with an electrode. For example, FIGS. 7A-7C illustrate an example variation of a microneedle 700 extending from a substrate 702. FIG. 7A is a side cross-sectional view of a schematic of microneedle 700, while FIG. 7B is a perspective view of the microneedle 700 and FIG. 7C is a detailed perspective view of a distal portion of the microneedle 700. As shown in FIGS. 7B and 7C, the microneedle 700 may include a columnar body portion 712, a tapered distal portion 714 terminating in an insulated distal apex 716, and an annular electrode 720. The annular electrode 720 includes a conductive material (e.g., Pt, Ir, Au, Ti, Cr, Ni, combinations thereof, etc.) arranged on the tapered distal portion 714, such as, for example, on a segment thereof, and comprises a distal edge 721a and a proximal edge 721b. As shown in FIG. 7A, the annular electrode 720 may be proximal to (offset or spaced apart from) the distal apex 716. The annular electrode 720 may be electrically isolated from the distal apex 716 by a distal insulating surface 715a including an insulating material (e.g., SiO₂). For example, the distal edge 721a of the annular electrode 720 may be proximate to a proximal edge of the distal insulating surface 715a of the insulated distal apex 716. In some variations, the distal edge 721a of the annular electrode 720 may be proximal to (e.g., just proximal to, adjacent, abutting) a proximal edge of the distal apex 716 (a proximal edge of the distal insulating surface 715a), while in other variations, the distal edge 721a of the annular electrode 720 may be distal to (e.g., just distal to, adjacent) the proximal edge of the insulated distal apex 716 (proximal edge of the distal insulating surface 715a), but may remain proximal to the apex itself. Accordingly, in some variations, the annular electrode 720 may overlie a portion of the distal insulating surface 715a, but may remain proximal to (and offset from) the insulated distal apex itself (*see, e.g.,* Fig. 21C).

Also as shown in FIG. 7A, the proximal edge 721b of the annular electrode 720 may be distal to, and in some variations, offset or spaced apart from, the columnar body portion 712. In some variations, the proximal edge 721b of the annular electrode 720 may also be electrically isolated from the columnar body portion 712 by a second distal insulating surface 715b comprising an insulating material (e.g., SiO₂) at a proximal end or region of the tapered distal portion 714. For example, the proximal edge 721b of the annular electrode 720 may be proximate to a distal edge of the second distal insulating surface 715b. In some variations, the proximal edge 721b of the annular electrode 720 may be proximal to (e.g., just proximal to, adjacent, abutting) a distal edge the second distal insulating surface 715b, while in other variations, the proximal edge 721b of the annular electrode 720 may be distal to (e.g., just distal to, adjacent) the distal edge of the second distal insulating surface 715b, but may remain proximal to the columnar body portion 712. Accordingly, in some variations, the annular electrode 720 may overlie a portion of the second distal insulating surface 715b but may remain proximal to (and offset from) the columnar body portion 712 (*see, e.g.,* Fig. 21C). As shown in FIG. 7A and in some other variations, the annular electrode 720 may be on only a segment of the surface of the tapered distal portion 714, and may or may not extend to the columnar boy portion 712.

The electrode 720 may be in electrical communication with a conductive core 740 (e.g., conductive pathway) passing along the body portion 712 to a backside electrical contact 730 (e.g., made of Ni/Au alloy) or other electrical pad in or on the substrate 702. For example, the body portion 712 may include a conductive core material (e.g., highly doped silicon). As shown in FIG. 7A, in some variations, an insulating moat 713 including an insulating material (e.g., SiO₂) may be arranged around (e.g., around the perimeter) of the body portion 712 and extend at least partially through the substrate 702. Accordingly, the insulating moat 713 may, for example, help prevent electrical contact between the conductive core 740 and the surrounding substrate 702. The insulating moat 713 may further extend over the surface of the body portion 712. Upper and/or lower surfaces of the substrate 702 may also include a layer of substrate insulation 704 (e.g., SiO₂). Accordingly, the insulation provided by the insulating moat 713 and/or substrate insulation 704 may contribute at least in part to the electrical isolation of the microneedle 700 that enables individual addressability of the microneedle 700 within a microneedle array. Furthermore, in some variations the insulating moat 713 extending over the surface of the body portion 712 may function to increase the mechanical strength of the microneedle 700 structure.

The microneedle 700 may be formed at least in part by suitable MEMS fabrication techniques such as plasma etching, also called dry etching. For example, in some variations, the insulating moat 713 around the body portion 712 of the microneedle may be made by first forming a trench in a silicon substrate by deep reactive ion etching (DRIE) from the backside of the substrate, then filling that trench with a sandwich structure of SiO₂ / polycrystalline silicon (poly-Si) / SiO₂ by low pressure chemical vapor deposition (LPCVD) or other suitable process. In other words, the insulating moat 713 may passivate the surface of the body portion 712 of the microneedle, and continue as a buried feature in the substrate 702 near the proximal portion of the microneedle. By including largely compounds of silicon, the insulating moat 713 may provide good fill and adhesion to the adjoining silicon walls (e.g., of the conductive core 740, substrate 702, etc.). The sandwich structure of the insulating moat 713 may further help provide excellent matching of coefficient of thermal expansion (CTE) with the adjacent silicon, thereby advantageously reducing faults, cracks, and/or other thermally-induced weaknesses in the insulating structure 713.

The tapered distal portion may be fashioned out by an isotropic dry etch from the frontside of the substrate, and the body portion 712 of the microneedle 700 may be formed from DRIE. The frontside metal electrode 720 may be deposited and patterned on the distal portion by specialized lithography (e.g., electron-beam evaporation) that permits metal deposition in the desired annular region for the electrode 720 without coating the distal apex 716. Furthermore, the backside electrical contact 730 of Ni/Au may be deposited by suitable MEMS manufacturing techniques (e.g., sputtering).

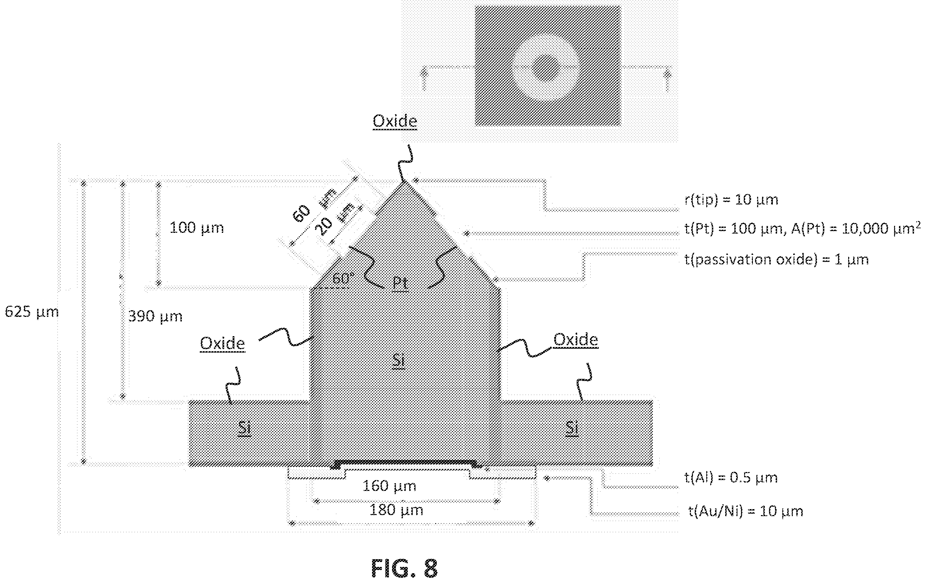

The microneedle 700 may have any suitable dimensions. By way of illustration, the microneedle 700 may, in some variations, have a height of between about 300 µm and about 500 µm. In some variations, the tapered distal portion 714 may have a tip angle between about 60 degrees and about 80 degrees, and an apex diameter of between about 1 µm and about 15 µm. In some variations, the surface area of the annular electrode 720 may include between about 9,000 µm² and about 11,000 µm², or about 10,000 µm². FIG. 8 illustrates various dimensions of an example variation of a columnar microneedle with a tapered distal portion and annular electrode, similar to microneedle 700 described above. As with the microneedle 700 described above, the columnar microneedle of FIG. 8 comprises a columnar body portion, a tapered distal portion terminating in an insulated distal apex, a contact trench formed within the tapered distal portion, and an annular electrode (denoted by "Pt" in FIG. 8) that is arranged on the tapered distal portion and overlays the contact trench. The annular electrode may comprise a conductive material (e.g., Pt, Ir, Au, Ti, Cr, Ni, combinations thereof, etc.). In some variations, the contact trench may have a width of about 1 µm, about 2 µm, about 3 µm, about 4 µm, about 5 µm, about 10 µm, about 15 µm, about 20 µm, about 25 µm, about 30 µm, about 35 µm, about 40 µm, about 45 µm, about 50 µm, or, as shown in FIG. 8, about 20 µm. The annular electrode may comprise a distal edge and a proximal edge, and in some variations, a distance between the distal edge and the proximal edge of the annular electrode may be about 20 µm, about 30 µm, about 40 µm about 50 µm, about 60 µm, about 70 µm, about 80 µm, about 90 µm, about 100 µm, or, as shown in FIG. 8, about 60 µm. In some variations, and as shown in FIG. 8 by the dimensional callouts 60 µm and 20 µm, the annular electrode may overlie the contact trench and, in some instances, a portion of the insulating surfaces (denoted by "Oxide" in FIG. 8) of the tapered distal portion.

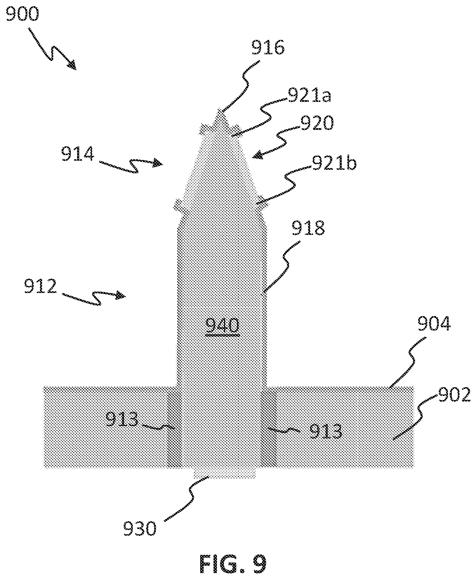

FIG. 9 illustrates another example variation of a microneedle 900 having a generally columnar body portion. The microneedle 900 may be similar to microneedle 700 as described above, except as described below. For example, like the microneedle 700, the microneedle 900 may include a columnar body portion 912, and a tapered distal portion 914 terminating in an insulated distal apex 916. The microneedle 900 may further include an annular electrode 920 that includes a conductive material and is arranged on the tapered distal portion 914 at a location proximal to (or offset from or spaced apart from) the distal apex 916. Other elements of microneedle 900 have numbering similar to corresponding elements of microneedle 700.

However, compared to the microneedle 700, the microneedle 900 may have a sharper tip at the distal apex 916 and a modified insulating moat 913. For example, the distal apex 916 may have a sharper tip angle, such as between about 25 degrees and about 45 degrees, and an apex radius of less than about 100 nm, which provides a sharper microneedle profile that may penetrate skin with greater ease, lower velocity, less energy, and/or less trauma. Furthermore, in contrast to the insulating moat 713 (which extends through the substrate 702 and along the height of the microneedle body portion 712 as shown in FIG. 7A), the modified insulating moat 913 may extend only through the substrate 902 such that the sandwich structure filling the trench (e.g., created by DRIE as described above) forms only the buried feature in the substrate. Although the sidewall of the microneedle 900 is shown in FIG. 9 as extending generally orthogonal to the substrate surface, it should be understood that because the modified insulating moat 913 need not extend the entire height of the microneedle body portion 712, in some variations the sidewall of the microneedle 900 may be angled at non-orthogonal angles relative to the substrate (e.g., the sidewall may have a slight positive taper of between about 1 degree to about 10 degrees, or between about 5 degrees and about 10 degrees).

In some variations, the rest of the microneedle surface 900 (aside from the annular electrode 920) may include an insulating material extending from substrate insulation 904. For example, a layer of an insulating material (e.g., SiO₂) may extend from a frontside surface of the substrate 902 to provide a body portion insulation 918, and may further extend up over a proximal edge 921b of the electrode 920 as shown in FIG. 9. Another region of insulating material may similarly cover a distal edge 921a of the electrode 920 and insulate the distal apex 916. Such region of insulating material and/or modified insulating moat 913 may help prevent electrical contact between the conductive core 940 and the surrounding substrate 902. Accordingly, like the microneedle 700, the microneedle 900 may maintain electrical isolation for individual addressability within a microneedle array. In some variations, the process to form microneedle 900 may result in higher yield and/or provide lower production cost compared to the process to form microneedle 700.

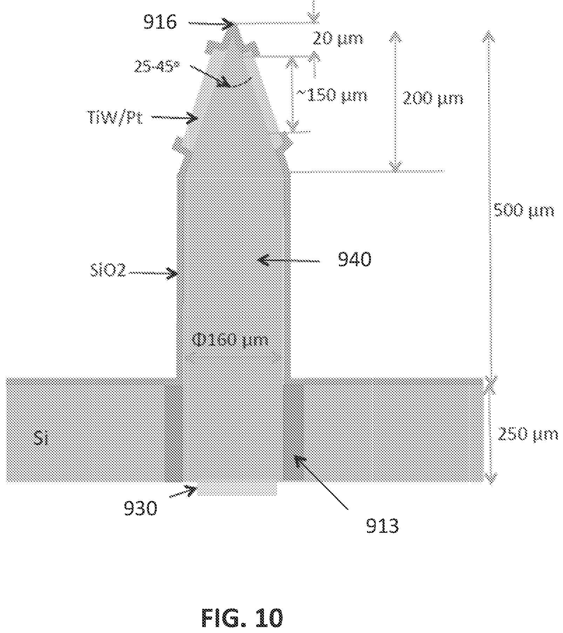

The microneedle 900 may have any suitable dimensions. By way of illustration, the microneedle 900 may, in some variations, include a height of between about 400 µm and about 600 µm, or about 500 µm. In some variations, the tapered distal portion 914 may have a tip angle of between about 25 degrees and about 45 degrees, with a tip radius of less than about 100 nm. Furthermore, the microneedle may have a shaft diameter of between about 160 µm and about 200 µm. FIG. 10 illustrates additional various dimensions of an example variation of a columnar microneedle with a tapered distal portion and annular electrode, similar to microneedle 900 described above.

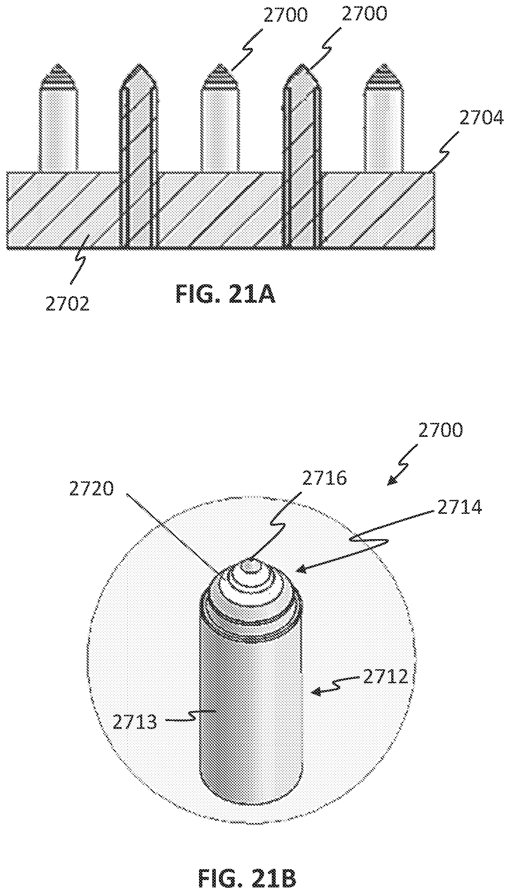

FIGS. 21A-21F illustrate another example variation of a microneedle 2700 having a generally columnar body portion. The microneedle 2700 may be similar to microneedle 700 as described above. For example, as shown in FIG. 21B, like the microneedle 700, the microneedle 2700 may include a columnar body portion 2712, and a tapered distal portion 2714 arranged on a cylinder 2713 and terminating in an insulated distal apex 2716. The cylinder 2613 may be insulated and have a smaller diameter than the columnar body portion 2712. The microneedle 2700 may further include an annular electrode 2720 that includes a conductive material and is arranged on the tapered distal portion at a location proximal to (or offset or spaced apart from) the distal apex 2916. The electrode 2720 may be electrically isolated from the distal apex 2716 by a distal insulating surface 2715a including an insulating material (e.g., SiO₂). In some variations, the proximal edge of the electrode 2720 may be electrically isolated from the columnar body portion 2712 by a second distal insulating surface 2715b comprising an insulating material (e.g., SiO₂). Other elements of microneedle 2700 as shown in FIGS. 21A-21F have numbering similar to corresponding elements of microneedle 700.

As can most easily be seen in FIGS. 21B, 21C and 21F, the tapered distal portion 2714, and more specifically, the electrode 2720 on the tapered distal portion 2714 of the microneedle 2700 may include a tip contact trench 2722. This contact trench may be configured to establish ohmic contact between the electrode 2720 and the underlying conductive core 2740 of the microneedle. In some variations, the shape of the tip contact trench 2722 may include an annular recess formed in the surface of the tapered distal portion 2714. In some variations, the shape of the tip contact trench 2722 may include an annular recess formed in the surface of the conductive core 2740 (e.g., into the body portion of the microneedle, or otherwise in contact with a conductive pathway in the body portion). In some variations, the tip contact trench 2722 may be formed in the insulating material on the tapered distal portion 2714, and may have a depth about equal to the thickness of the insulating material (e.g., the distal insulating surface 2715a and/or the second distal insulating surface 2715b). In some instances, the depth of the contact trench may be greater than the thickness of the insulating material such that the contact trench extends beyond a surface of the conductive core 2740 (e.g., into the conductive core 2740). The electrode 2720 may overlie the tip contact trench 2722 such that ohmic contact is established between the electrode 2720 and the conductive core 2740. In some variations, the electrode 2720 may extend beyond the tip contact trench 2722 such that when the electrode 2720 material is deposited onto the conductive core 2740, the electrode 2720 with the tip contact trench 2722 may have a stepped profile when viewed from the side. The tip contact trench 2722 may thus advantageously help ensure contact between the electrode 2720 and the underlying conductive core 2740. Any of the other microneedle variations described herein may also have a similar tip contact trench to help ensure contact between the electrode (which may be, for example, a working electrode, reference electrode, counter electrode, etc.) with a conductive pathway within the microneedle.

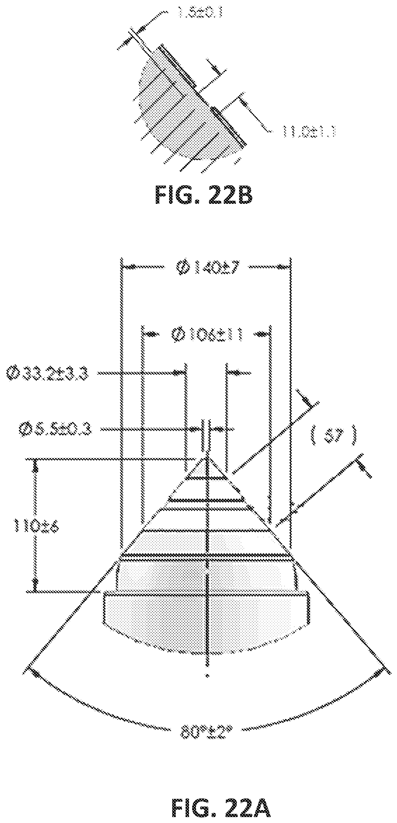

FIGS. 22A and 22B illustrate additional various dimensions of an example variation of a columnar microneedle with a tapered distal portion and annular electrode, similar to microneedle 2700 described above. For example, the variation of the microneedle shown in FIGS. 22A and 22B may have a tapered distal portion generally having a taper angle of about 80 degrees (or between about 78 degrees and about 82 degrees, or between about 75 degrees and about 85 degrees), and a cone diameter of about 140 µm (or between about 133 µm and about 147 µm, or between about 130 µm and about 150 µm). The cone of the tapered distal portion may be arranged on a cylinder such that the overall combined height of the cone and cylinder is about 110 µm (or between about 99 µm and about 116 µm, or between about 95 µm and about 120 µm). The annular electrode on the tapered distal portion may have an outer or base diameter of about 106 µm (or between about 95 µm and about 117 µm, or between about 90 µm and about 120 µm), and an inner diameter of about 33.2 µm (or between about 30 µm and about 36 µm, or between about 25 µm and about 40 µm). The length of the annular electrode, as measured along the slope of the tapered distal portion, may be about 57 µm (or between about 55 µm and about 65 µm), and the overall surface area of the electrode may be about 12,700 µm² (or between about 12,500 µm² and about 12,900 µm², or between about 12,000 µm² and about 13,000 µm²). As shown in FIG. 22B, the electrode may furthermore have a tip contact trench extending around a central region of the cone of the tapered distal portion, where the contact may have a width of about 11 µm (or between about 5 µm and about 50 µm , between about 10 µm and about 12 µm, or between about 8 µm and about 14 µm) as measured along the slope of the tapered distal portion, and a trench depth of about 1.5 µm (or between about 0.1 µm and about 5 µm , or between about 0.5 µm and about 1.5 µm , or between about 1.4 µm and about 1.6 µm, or between about 1 µm and about 2 µm). The microneedle has an insulated distal apex having a diameter of about 5.5 µm (or between about 5.3 µm and about 5.8 µm, or between about 5 µm and about 6 µm).

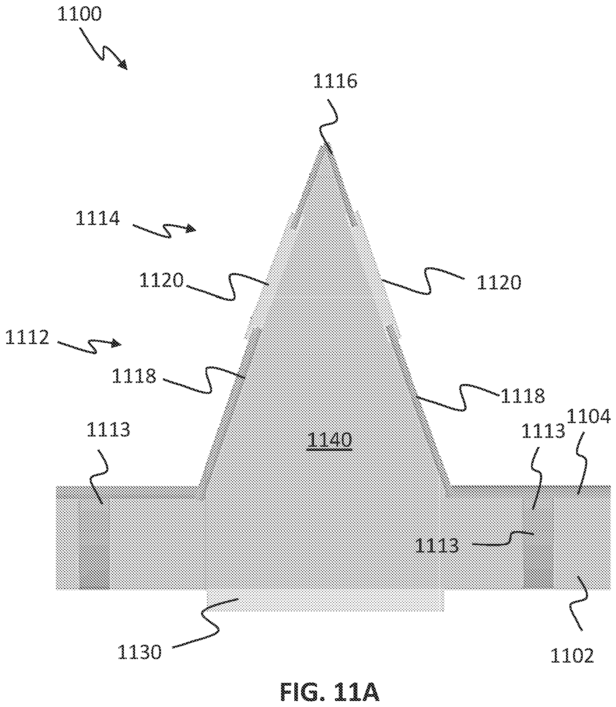

In some variations, a microneedle may have a generally pyramidal body portion and a tapered distal portion with an electrode. For example, FIG. 11A illustrates an example variation of a microneedle 1100 having a generally pyramidal body portion 1112 and a tapered distal portion 1114 extending from the body portion 1112. The microneedle 1100 may also include an annular electrode 1120 arranged on the tapered distal portion 1114 and proximal to an insulated distal apex 1116. The electrode 1120 may be conductively coupled via a conductive pathway through the conductive core 1140 of the microneedle to a backside electrical contact 1130. Like the microneedle 900 described above with respect to FIG. 9, the microneedle 1100 may include an insulating moat 1113 that is arranged around the base of the body portion 1112 and extends through the substrate 1102 to provide electrical insulation around the microneedle 1100 (e.g., for individual addressability) and help prevent electrical contact between the conductive core 1140 and the surrounding substrate 1102. However, in contrast to the insulating moat 913 shown in FIG. 9, the insulating moat 1113 may be offset from the base of the microneedle 1100. The moat may, for example, be offset between about 10 µm and about 400 µm, between about 10 µm and about 300 µm, between about 10 µm and about 200 µm, or between about 10 µm and about 100 µm from where the base of the microneedle 1100 meets the substrate 1102 to which it is attached. In some variations, the insulating moat may include a filler material including parylene, Si₃N₄, and SiO₂, which may provide for low thermal stress and an insulating material that is chemical- and water-resistant. Additional body portion insulation 1118 may extend from a frontside surface of the substrate 1102 up to the proximal edge of the electrode 1120. Another region of insulating material may extend from the distal edge of the electrode 1120 and insulate the distal apex 1116.

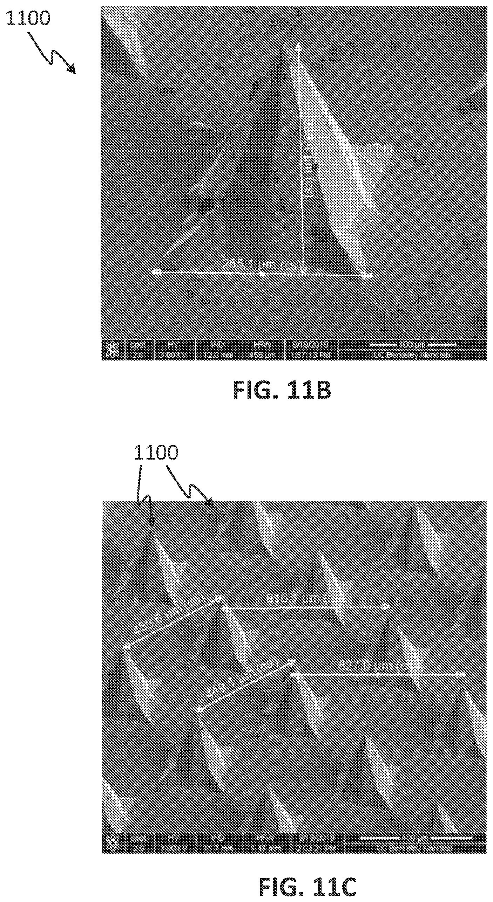

As shown in FIG. 11B, in some variations a microneedle 1100 having a pyramidal body portion 1112 may include a polygonal base, though the base may have any suitable shape (e.g., circular). The pyramidal body portion 1112 may include a plurality of planar facets each extending from a respective of the polygonal base of the microneedle. In some variations, the planar facets may include anisotropically etched <311> planar facets for increased mechanical strength (e.g., compressive strength and shear strength) of the microneedle 1110 and/or increased electrode surface area relative to a circular cone with a non-planar faceted surface. For example, the microneedle 1110 may have an octagonal base with anisotropically etched <311> planar facets that increase the mechanical strength and increase the metallization surface of the microneedle 110 for the electrode surface.

The microneedle 1100 may be formed at least in part by suitable MEMS fabrication techniques. For example, the microneedle pyramidal structure may be formed by a timed anisotropic wet etch of a silicon wafer substrate. To form the annular electrode surface, metal deposition on the tapered distal portion of the microneedle may be performed, such as using specialized lithographic techniques as described above with respect to electrode 720, without coating the distal apex 1116. However, compared to the process described above to form microneedle 700, much of the process to form microneedle 1100 does not involve expensive RIE techniques, which may thereby substantially reduce manufacturing costs. Furthermore, in some variations, instead of utilizing dry etch processes as described above with respect to microneedle 700, a process of forming the microneedle 1100 may include mechanical dicing, bulk micromachining, or other cutting techniques to shape the microneedle 1100 into having a pyramidal body. Furthermore, such techniques may be performed at a large scale, so as to form, for example, multiple microneedles 1110 arranged in an array as shown in FIG. 11C.

The microneedle 1100 may have any suitable dimensions. By way of illustration, the microneedle 1100 may, in some variations, have a height of between about 400 µm and about 600 µm, or about 500 µm. In some variations, the tapered distal portion 714 may have a tip angle between about 30 degrees and about 50 degrees, or about 40 degrees, which may provide a good balance between sharpness for skin penetration and lithography processability on the sloped surface on which the electrode 1120 is to be disposed.

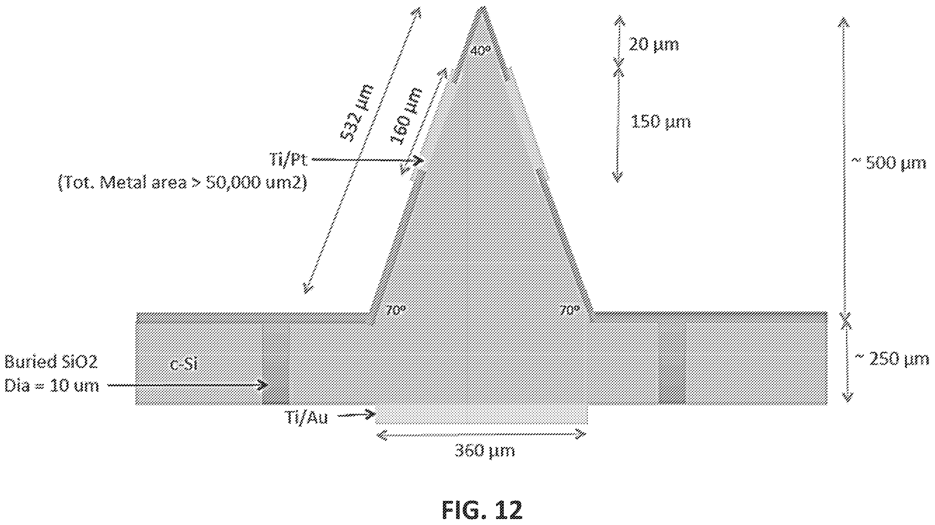

FIG. 12 illustrates various dimensions of an example variation of a pyramidal microneedle with a tapered distal portion having planar facets and an electrode arranged on at least a portion of the planar facets. While in some variations the electrode may be annular or annular-like in that all of the planar facets on the pyramidal microneedle may include a metallization surface for the electrode, it should be understood that alternatively, in some variations only a portion of the planar facets on the pyramidal microneedle may include a metallization surface (e.g., one, two, three, four, five, six, or seven planar facets of a pyramidal microneedle having an octagonal base and eight planar facets extending distally from the octagonal base).

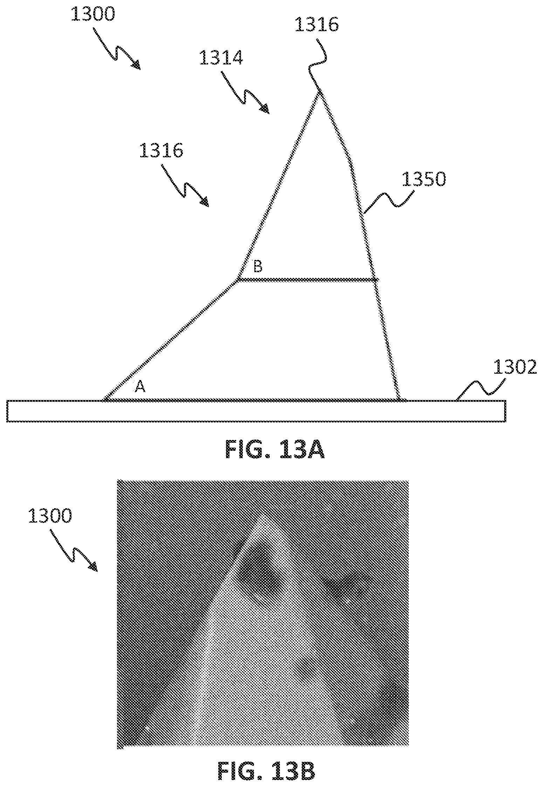

In some variations, a pyramidal microneedle may be similar to that described above with respect to FIG. 11A, except that the microneedle may have an asymmetrical shape as shown in FIGS. 13A and 13B. For example, in some variations as shown in FIG. 13A, a microneedle 1300 may have a non-circular or polygonal (e.g., square, octagonal) base, but may taper in a radially asymmetric manner. For example, the microneedle 1300 may include at least one cut surface 1350 (e.g., planar surface) that is offset from the distal apex 1316 of the microneedle (that is, not extending through a central z-axis defined as passing from the base of the microneedle 1300 to the distal apex 1316. The insulated distal apex 1316 may be kept intact so as to not compromise surface area for metallization for the electrode. In some variations, the cut surface 1350 may be angled at a non-orthogonal angle relative to the base of the microneedle (and/or surface of the substrate 1302), as shown in FIG. 13A. For example, in some variations the cut surface may configured to produce a sharpened asymmetrical distal tip at distal apex 1316 that is less than about 50 degrees, less than 40 degrees, less than about 30 degrees, or less than about 20 degrees. Alternatively, in some variations the cut surface 1350 may be angled normal to or orthogonal to the base of the microneedle (and/or surface of the substrate 1302).

Additionally or alternatively, as shown in FIG. 13A, an example variation of an asymmetric microneedle 1300 may have a polygonal (e.g., octagonal) base, but include various sloped surfaces that taper at different angles. As shown in FIG. 13A, a body portion 1316 of the microneedle 1300 may have a first taper angle (A) and a second taper angle (B) measured relative to a base of the body portion (and/or surface of the substrate 1302). The second taper angle (B) may be greater than the first taper angle (A) such that the microneedle has a sharper penetrating tip extending from a stable, mechanically strong base. For example, in some variations, the first taper angle (A) may be between about 10 degrees and about 30 degrees, between about 15 degrees and about 25 degrees, or about 20 degrees. Additionally, in some variations, the second taper angle (B) may be between about 60 degrees and about 80 degrees, between about 65 degrees and about 75 degrees, or about 70 degrees.

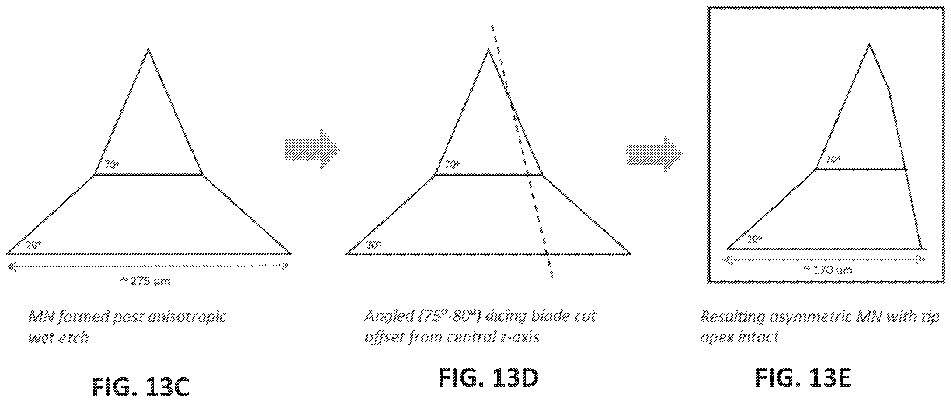

FIGS. 13C-13E depict a series of steps in an example variation of forming a pyramidal microneedle with an asymmetric cut surface. As shown in FIG. 13C, a symmetric pyramidal microneedle with two taper angles may be formed through an anisotropic wet etch process. The two taper angles of the microneedle may include, for example, a first taper angle of about 20 degrees located near the base of the microneedle, and a second taper angle of about 70 degrees located distal to the first tape angle, thereby forming progressively sloping surfaces (e.g., along planar facets of the pyramidal microneedle). As shown in FIG. 13D, a dicing blade may be applied at an angle offset from the distal apex of the microneedle, so as to form a cut surface similar to cut surface 1350 described above. The cut surface may leave a reduced microneedle base diameter (e.g., between about 150 µm and about 190 µm, or about 170 µm) so as to result in low tissue trauma. As shown in FIG. 13E, the resulting microneedle (with its offset cut surface) is asymmetric but has an intact, sharp distal apex.

Like the pyramidal microneedle 1100 described above with respect to FIG. 11A, the microneedle 1300 may derive its mechanical strength at least in part from anisotropically etched <311> planes and the pyramidal shape. However, an asymmetric pyramidal microneedle with a asymmetric cut surface may be advantageous in that it may reduce the longitudinal shear forces compared to a symmetric microneedle having similar dimensions but lacking the asymmetric cut. Furthermore, a sharper (e.g., more acute angle) distal microneedle tip may be achieved with such an asymmetric cut surface. Although the cut surface 1350 is shown in FIG. 13A as positioned at a non-orthogonal angle relative to the base of the microneedle, alternatively as described above, in some variations the cut surface 1350 may be generally orthogonal or normal to the base of the microneedle (and/or surface of the substrate 1302), which may further reduce the longitudinal shear forces in the microneedle.

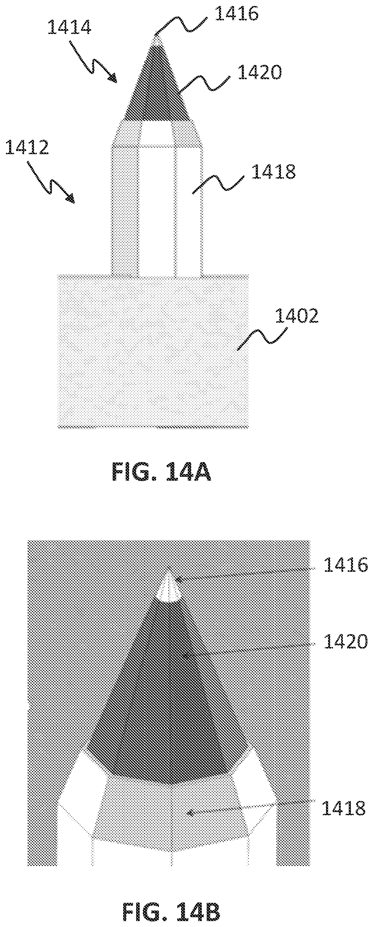

In some variations, a microneedle may be similar to those described above, except that the microneedle may include a columnar body portion and a pyramidal distal portion. For example, as shown in FIGS. 14A-14B, a columnar-pyramidal microneedle 1400 may include a columnar body portion 1412 that may extend from a polygonal (e.g., octagonal) base out of a non-electrically conductive substrate 1402 such as intrinsic (undoped) silicon. Additionally, the columnar-pyramidal microneedle 1400 may include a tapered distal portion 1414 having a pyramidal shape with a plurality of planar facets. For example, the columnar-pyramidal microneedle 1400 may include a tapered distal portion 1414 having a pyramidal shape with eight facets extending from the octagonal columnar body portion 1412. However, the pyramidal shape may have any suitable number of planar facets (e.g., one, two, three, four, five, six, seven, nine, or more). An annular electrode 1420 may be formed on all the planar facets of the pyramidal distal portion 1414, or on only a portion of the planar facets (e.g., on one, two, three, four, five, six, seven facets) may include a metallization surface for the electrode. Similar to that described above, the columnar body portion 1412 may include a conductive core including an electrically conductive material functioning as a conductive pathway for signals to and from the electrode 142. The columnar body portion 1412 may further include an insulation material 1418 may extend along the body portion 1412 and up to (or slightly overlapping) a proximal edge of the electrode 1420. The distal apex 1416 may or may not be covered by similar insulation material.

In some variations, the tapered distal portion 1414 may be similar to that described above with respect to FIGS. 11A-11C, 12, and/or 13A-13E. For example, the tapered distal portion 1414 may be formed using anisotropic wet etching techniques. The electrode 1420 may be formed on the tapered distal portion 1414 by lithography, electrodeposition or other suitable technique. The tapered distal portion 1414 may then be protected by an etch resistant material while the body portion 1412 is formed out of the substrate by dry etching (e.g., DRIE) or other suitable process(es).

The combination of columnar and pyramidal aspects of the microneedle 1400 has a number of advantages. Similar to that described above, the tapered distal portion 1414 and apex 1416 have high mechanical strength due to the <311> wet etched planes and the pyramidal shape. Additionally, because the substrate is formed from a non-conductive material, an insulation "moat" as described above may not be required to electrically isolate the microneedle, thereby simplifying and reducing cost of fabrication. The absence of the insulation moat also permits material continuity in the substrate, which may lead to better mechanical integrity of the overall microneedle array structure.

Although the columnar-pyramidal microneedle 1400 is described above as including a non-conductive substrate, it should be understood alternatively, in some variations a columnar-pyramidal microneedle may include a conductive core extending from a conductive substrate (e.g., doped silicon). For example, in some variations the columnar body portion 1412 may be similar to that described above with respect to FIGS. 7A-7C, and 8-10 (e.g., may include an insulation moat to electrically isolate the microneedle, etc.).

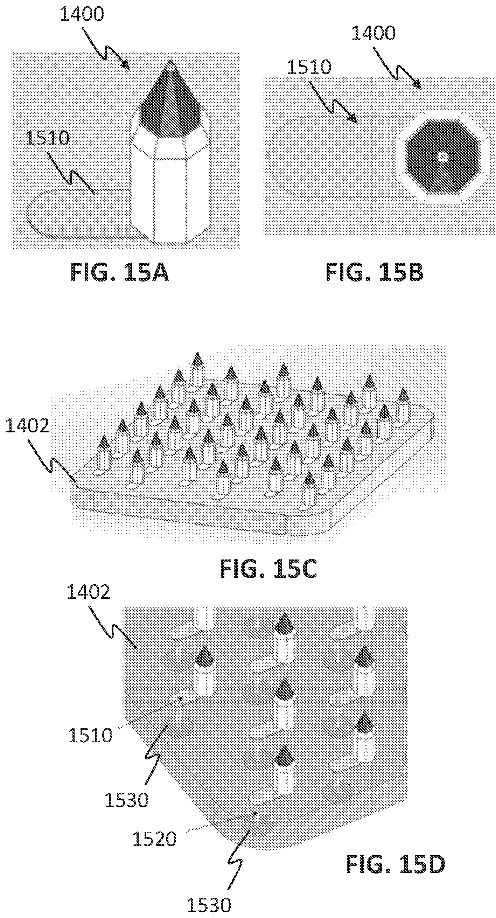

In some variations of microneedle arrays including one or more microneedles 1400, conductive pathways may be formed in the non-conductive substrate to facilitate communication with the electrode(s) 1420. For example, as described above, the body portion 1412 of each microneedle may include a conductive core including a conductive material. Such conductive material may extend between the electrode 1420 to the substrate 1402. As shown in FIG. 15D, the microneedle array may include one or more connectors 1510 made of a conductive material (e.g., gold, aluminum) that is each in turn coupled to a backside electrical contact 1530 for further sensor communication. In some variations, as shown FIGS. 15A-15D, the one or more connectors 1510 may extend laterally along the surface of the substrate and then connect to the backside electrical contact 1530 with a conductive via 1520 within the substrate.

Additional details of example variations of microneedle array configurations are described in further detail below.

### Electrodes

As described above, each microneedle in the microneedle array may include an electrode. In some variations, multiple distinct types of electrodes may be included among the microneedles in the microneedle array. For example, in some variations the microneedle array may function as an electrochemical cell operable in an electrolytic manner with three types of electrodes. In other words, the microneedle array may include at least one working electrode, at least one counter electrode, and at least one reference electrode. Thus, the microneedle array may include three distinct electrode types, though one or more of each electrode type may form a complete system (e.g., the system might include multiple distinct working electrodes). Furthermore, multiple distinct microneedles may be electrically joined to form an effective electrode type (e.g., a single working electrode may be formed from two or more connected microneedles with working electrode sites). Each of these electrode types may include a metallization layer and may include one or more coatings or layers over the metallization layer that help facilitate the function of that electrode.

Generally, the working electrode is the electrode at which oxidation and/or reduction reaction of interest occurs for detection of an analyte of interest. The counter electrode functions to source (provide) or sink (accumulate) the electrons, via an electrical current, that are required to sustain the electrochemical reaction at the working electrode. The reference electrode functions to provide a reference potential for the system; that is, the electrical potential at which the working electrode is biased is referenced to the reference electrode. A fixed, time-varying, or at least controlled potential relationship is established between the working and reference electrodes, and within practical limits no current is sourced from or sinked to the reference electrode. Additionally, to implement such a three-electrode system, the cortisol monitoring device may include a suitable potentiostat or electrochemical analog front end to maintain a fixed potential relationship between the working electrode and reference electrode contingents within the electrochemical system (via an electronic feedback mechanism), while permitting the counter electrode to dynamically swing to potentials required to sustain the redox reaction of interest.

Turning to the aptamer-based approach for measuring and monitoring cortisol in dermal interstitial fluid, consistent with implementations of the current subject matter, aspects of a working electrode, a counter electrode, and a reference electrode are provided.

### Working electrode

As described above, the working electrode is the electrode at which the detection of an analyte such as cortisol occurs. In some variations, sensing may be performed at the interface of the working electrode and interstitial fluid located within the body (e.g., on an outer surface of the overall microneedle). In some variations, a working electrode may include an electrode material and a biorecognition layer in which a biorecognition element (e.g., an aptamer) is immobilized on the working electrode to facilitate selective analyte quantification. In some variations, the biorecognition layer may also function as an interference-blocking layer and may help prevent endogenous and/or exogenous species from directly oxidizing (or reducing) at the electrode.

In some variations, a redox current detected at the working electrode may be correlated to a concentration of an analyte of interest. This is because assuming a steady-state, diffusion-limited system, the redox current detected at the working electrode follows the Cottrell relation in equation (1) below: $i \left(t\right) = \frac{nFA \sqrt{DC}}{\sqrt{πt}}$ where n is the stoichiometric number of electrons mitigating a redox reaction, F is Faraday's constant, A is electrode surface area, D is the diffusion coefficient of the analyte of interest, C is the concentration of the analyte of interest, and t is the duration of time that the system is biased with an electrical potential. Thus, the detected current at the working electrode scales linearly with the analyte concentration. This relationship is applicable when the system is biased at a constant potential.

In some variations, such as when the system is biased at a time-varying potential, a redox current detected at the working electrode may be correlated to a concentration of an analyte of interest based upon the relationship in equation (2): $Δ {i}_{p} = \frac{nFA \sqrt{DC}}{\sqrt{πt}} Δ {ψ}_{p}$ where Δψₚ is the dimensionless peak current and gauges the peak current height based on the system's diffusion-limited response. This relationship assumes a steady-state, diffusion-limited system.

In some variations, the surface of the electrode is functionalized with a redox-active molecule via immobilization through an aptamer, and cortisol binding to these surface sites follows the Michaelis-Menten model. Upon binding cortisol, a cortisol-binding aptamer experiences a conformational change that moves the redox-active molecule closer, or further, from the electrode. The redox-active molecule is held in its oxidized state, and a sweep to more negative potential reduces the redox-active molecule within range of electron transfer. The final relationship is non-linear and is quasi-linear over a limited range when comparing the signal gain with the logarithm of cortisol concentration. The relationship for redox current detected at the working electrode is represented by equation (3) below: $i = - nFA ⋅ dΓO / dt$ where dΓO/dt is the change in surface concentration of the oxidized form of the redox-active molecule with time.

Moreover, because the detected current is a direct function of electrode surface area A, the surface area of the electrode may be increased to enhance the sensitivity (e.g., amperes per molar of analyte) of the sensor. For example, multiple singular working electrodes may be grouped into arrays of two or more constituents to increase total effective sensing surface area. Additionally or alternatively, to obtain redundancy, multiple working electrodes may be operated as parallelized sensors to obtain a plurality of independent measures of the concentration of an analyte of interest. The working electrode can either be operated as the anode (such that an analyte is oxidized at its surface) or as the cathode (such that an analyte is reduced at its surface).

In some variations, the biorecognition element in the biorecognition layer may be an aptamer that selectively and reversibly binds to the glucocorticoid hormone cortisol (a "cortisol-binding aptamer"). An aptamer is a single-stranded oligonucleotide that folds into a defined structure that selectively binds to a specific target, which may be, by way of example, a protein, a peptide, a hormone, or a small molecule. Recognition and binding of an aptamer to its target involve three-dimensional, shape-dependent interactions as well as hydrophobic interactions, base-stacking, and intercalation, and are typically reversible through dissociation. Aptamers with affinity for a desired target, such as cortisol, are conventionally selected from a large oligonucleotide library through a process called SELEX (Sequential Evolution of Ligands by Exponential Enrichment). Through an iterative process, non-binding aptamers are discarded and aptamers binding to the proposed target are amplified by polymerase chain reaction (PCR). Initial positive selection rounds may be followed by negative selection, which improves the selectivity of the resulting aptamer candidates. Multiple rounds of SELEX may be performed with increasing stringency to enhance enrichment of the oligonucleotide pool, until one or more oligonucleotides having a desired degree of affinity and selectivity for the desired target are selected for use.

In some variations, the oligonucleotides comprised in an aptamer may be DNA or RNA. The oligonucleotide may be functionalized at the 3' end or the 5' end. One end may provide a chemical moiety ("immobilization moiety") for surface immobilization, such as an amine, aldehyde, carboxylic acid, thiol, disulfide, azide, n-hydroxysuccinimide (NHS), maleimide, vinyl, silane, chlorosilane, methoxysilane, ethoxysilane, or acetylene group. The immobilization moiety may be separated from the oligonucleotide sequence by a linker selected for its ability to create distance between the oligonucleotide sequence and the surface to which it is immobilized. The linker may also be chosen for its compatibility with other chemical layers on the electrode surface, for example, a hydrocarbon linker with equal or similar length to the hydrocarbon chain used in a self-assembled monolayer that is coating the remainder of the electrode surface. The opposite end of the oligonucleotide may be functionalized with one or more redox active molecules, by way of example methylene blue, ferrocene, pentamethyl ferrocene, C5-ferrocene, Nile blue, thionine, anthraquinone, C5-anthraquinone, gallocyanine, indophenol, neutral red, dabcyl, or carboxy-X-rhodamine, that serve as a probe. These redox-active molecules may also be attached to the oligonucleotide through a custom linker. The backbone of the oligonucleotide may be modified to increase stability in physiological conditions. For example, an RNA sequence incorporating L-ribose or a DNA sequence incorporating L-deoxyribose, as opposed to their natural respective dextrorotary sugars, may be used to protect the oligonucleotide from degradation by enzymes in the body. In some variations, a backbone modification may include replacing ribose in RNA or deoxyribose in DNA with 2'-O-methyl ribose, also with the effect of protection from enzyme cleavage in physiological conditions. In some variations, the cortisol-binding aptamer is defined by the following DNA sequence, 5'-GGACGACGCCAGAAGTTTACGAGGATATGGTAACATAGTCGT-3', where G, A, C, and T represent the typical DNA nucleotides containing guanine, adenine, cytosine, and thymine, respectively.

In some variations, the cortisol-binding aptamer may be selected not for maximal affinity for cortisol, but for an intermediate degree of affinity such that the portion of a population of the selected aptamer having a cortisol molecule bound to it is sensitive to a physiological concentration range of cortisol within dermal interstitial fluid, which may be between about 0.001 µmol/L and about 1 µmol/L. In some variations, selection criteria of the cortisol-binding aptamer may include the cortisol-binding aptamer having between about 10% and about 75% "on" gain from minimum to maximum cortisol concentrations and/or having between about 10% to about 40% "off" gain from minimum to maximum cortisol concentrations. A signal "on gain" may refer to a set of square wave voltammetry parameters (frequency, peak value, step height) selected towards maximizing a current signal obtained in the presence of a target analyte. A signal "off gain" may refer to a set of square wave voltammetry parameters selected towards minimizing the current signal obtained in the presence of a target analyte. Sensitivity of the aptamer to cortisol in dermal interstitial fluid advantageously allows for avoiding interference or signal degradation over time from biofouling or irreversible changes to the aptamer structure due to folding or damage.

The cortisol-sensing aptamer may be functionalized at the 3' end or the 5' end by a redox-active molecule, by way of example methylene blue, ferrocene, pentamethyl ferrocene, C5-ferrocene, Nile blue, thionine, anthraquinone, C5-anthraquinone, gallocyanine, indophenol, neutral red, dabcyl, or carboxy-X-rhodamine, such that specific and reversible binding of cortisol to the cortisol-binding aptamer and the resultant conformational change of the cortisol-binding aptamer leads to a change in the proximity, and thus electron transfer characteristics, between the redox-active molecule and the working electrode that is corresponding to the cortisol concentration. This change in the electron transfer characteristics of the electrode caused by cortisol binding may be interrogated by various electrochemical techniques such as voltammetry, potentiometry, chronoamperometry, and/or electrochemical impedance spectroscopy. Voltammetry techniques vary the potential as a function of time and the resulting current is plotted as a function of potential. For example, cyclic voltammetry (CV) sweeps the potential of the cell linearly across a voltage range, while a fast scan CV (FSCV) technique does this at a faster rate. Square wave voltammetry (SWV) uses a square wave superimposed over a staircase function to provide a sweeping measurement that provides two sampling instances per potential. As a result of this sampling technique, the contribution to the total current that results from non-faradic currents is minimized in SWV. In potentiometry, an open circuit potential is measured between a reference electrode and a working electrode. In chronoamperometry, the potential is stepped at the beginning of a measurement and then remains constant throughout the duration of the measurement, and the current that results from this stimulus may be plotted as a function of time. In electrochemical impedance spectroscopy, the complex impedance of the electrode is determined at one or more frequencies. Contributions to impedance (or admittance) from resistive and reactive circuit elements may be dependent on the position of redox probes tethered to surface-bound aptamers and correlate with cortisol concentration.

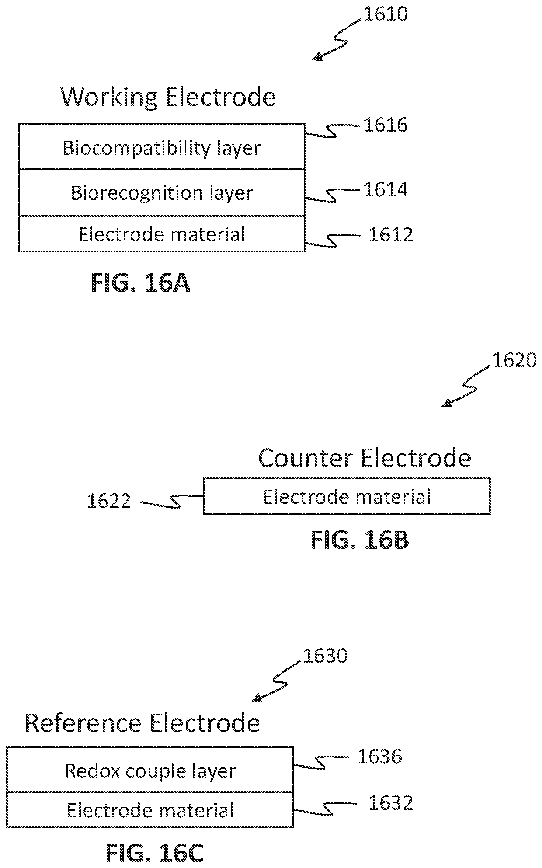

FIG. 16A depicts a schematic of an exemplary set of layers for a cortisol-sensing working electrode 1610. For example, as described above, in some variations the working electrode 1610 may include an electrode material 1612 and a biorecognition layer 1614 comprising a cortisol-binding aptamer functionalized by a redox-active molecule. The electrode material 1612 is used to electrically detect the change in electron transfer properties between the redox-active molecule attached to the cortisol-binding aptamer comprised in biorecognition layer 1614 and the electrode material 1612 caused by cortisol binding to the cortisol-binding aptamer. The electrode material 1612 also provides ohmic contact and routes an electrical signal from the electrocatalytic reaction to processing circuitry. In some variations, the electrode material 1612 may be platinum, as shown in FIG. 16A, or may be gold. In other variations, electrode material 1612 may include, for example, palladium, iridium, rhodium, ruthenium, titanium, nickel, alloys of the aforementioned metals, cobalt chrome alloy, cobalt chrome molybdenum alloy, stainless steel 316L, iridium oxide, titanium nitride, carbon, doped diamond, silicon, doped silicon, or other suitable material. The carbon may be pyrolytic carbon, pyrolytic graphite, or glassy carbon.

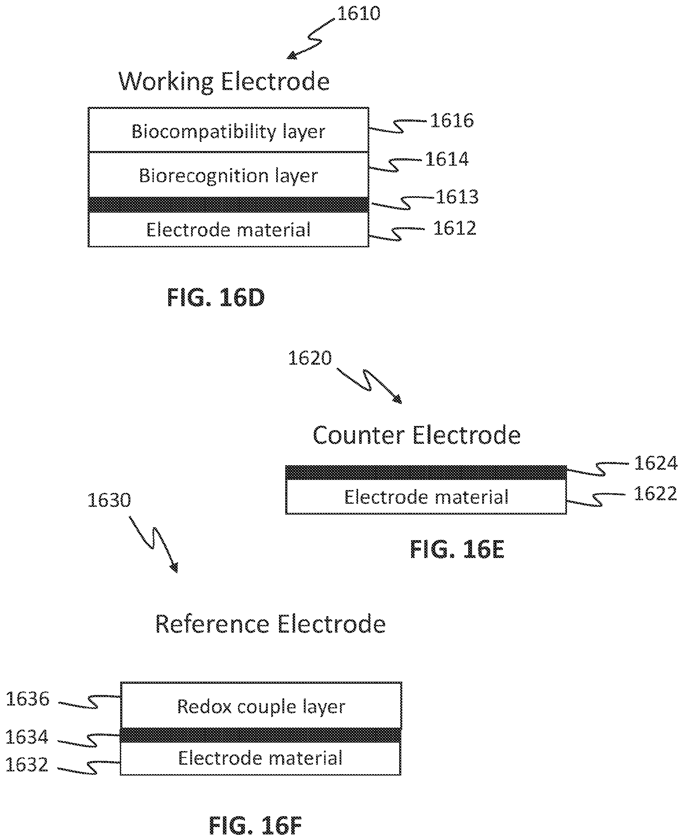

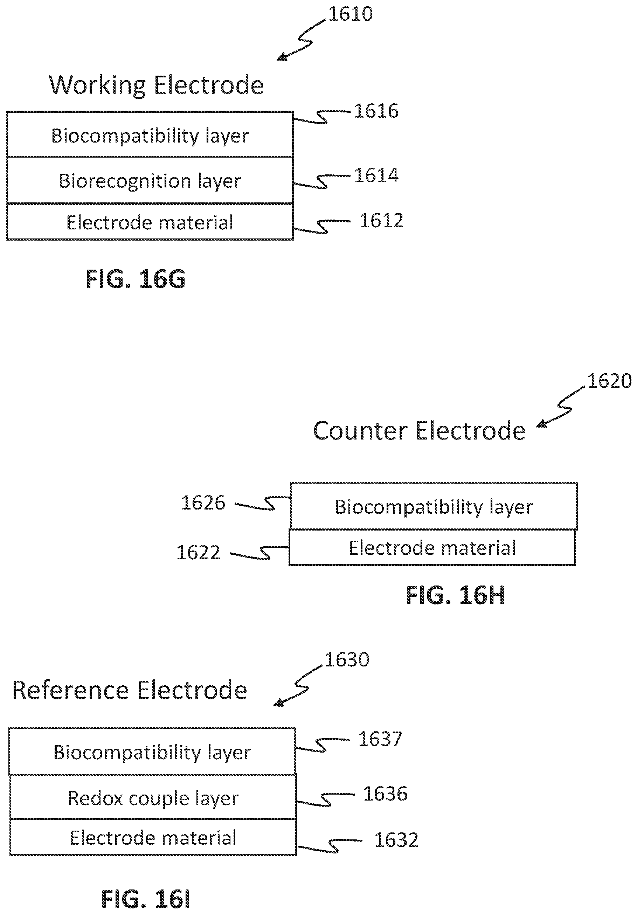

In some variations, the electrode material 1612 may be coated with a highly porous electrocatalytic layer, such as a platinum black layer 1613, which may augment the electrode surface area for enhanced sensitivity (as shown in FIG. 16D, for example). In some variations the platinum black layer 1613 may be omitted (as shown in FIGS. 16A and 16G, for example).

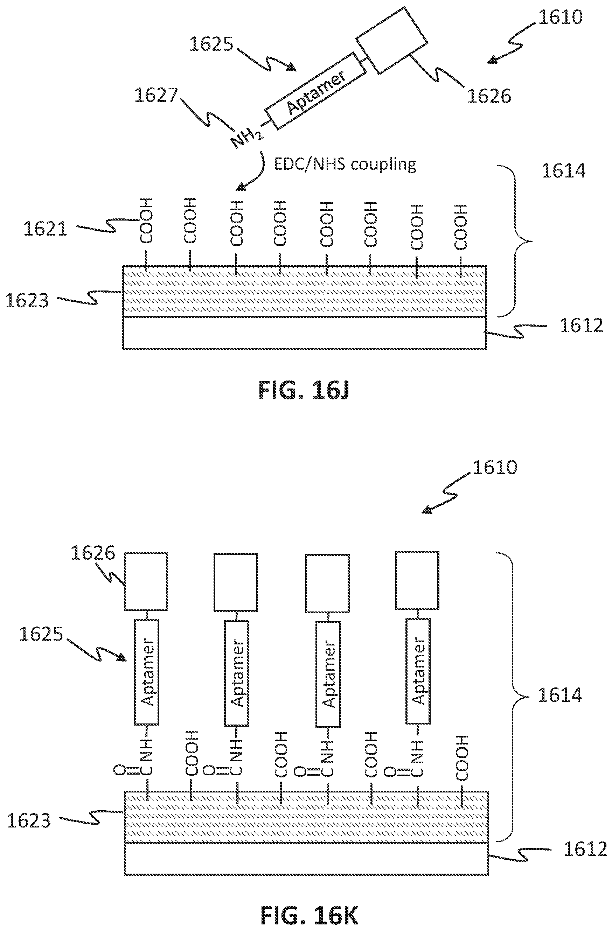

In some variations, the biorecognition layer 1614 may comprise a conducting polymer. The conducting polymer may be permselective to contribute to the biorecognition layer's robustness against circulating endogenous electroactive species (e.g., ascorbic acid, vitamin C, etc.), fluctuations of which may adversely affect the sensitivity of the sensor. Such a permselective conducting polymer in the biorecognition layer may further be more robust against pharmacological interferences (e.g., acetaminophen) in the interstitial fluid that may affect sensor accuracy. Conducting polymers may be made permselective by, for example, removing excess charge carriers by an oxidative electropolymerization process, or by intentionally overoxidizing the conductive polymer at an elevated potential after its polymerization, disrupting its conjugated backbone and rendering it non-conductive. These oxidatively-polymerized conducting polymers exhibit permselectivity and are hence able to reject ions of similar charge polarity to the dopant ion (net positive or negative) or by size exclusion due to the dense and compact form of the conducting polymers. In some variations, the conductive polymer may include one or more of aniline, pyrrole, pyrrole-3-carboxylic acid, pyrrole-1-propionic acid, acetylene, phenylene, phenylene vinylene, phenylenediamine, thiophene, 3-methylthiophene, 3-hexylthiophene, 3-thiophene carboxylic acid, 3,4-ethylenedioxythiophene (EDOT), EDOT carboxylic acid, and aminophenylboronic acid. An example of a working electrode 1610 in which the biorecognition layer 1614 comprises a film 1623 of conductive polymer is shown in FIGS. 16J-16K.

In some variations, the conducting polymer may exhibit self-sealing and/or self-healing properties. For example, the conducting polymer may undergo oxidative electropolymerization, during which the conducting polymer may lose its conductivity as the thickness of the deposited conducting polymer on the electrode increases, until the lack of sufficient conductivity causes the deposition of additional conducting polymer to diminish. In the event that the conducting polymer has succumbed to minor physical damage (e.g., during use), the polymeric backbone may re-assemble to neutralize free charge and thereby lower overall surface energy of the molecular structure, which may manifest as self-sealing and/or self-healing properties.

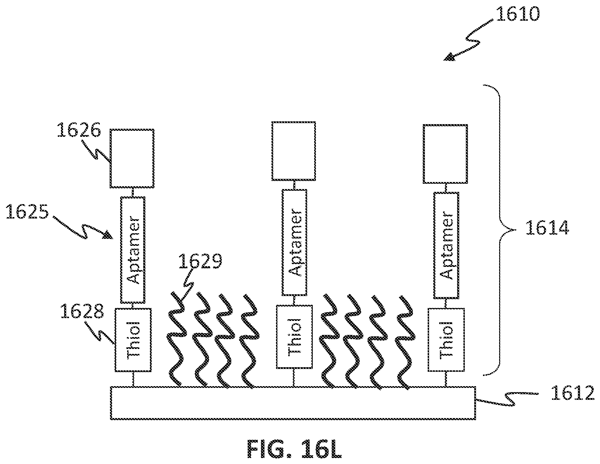

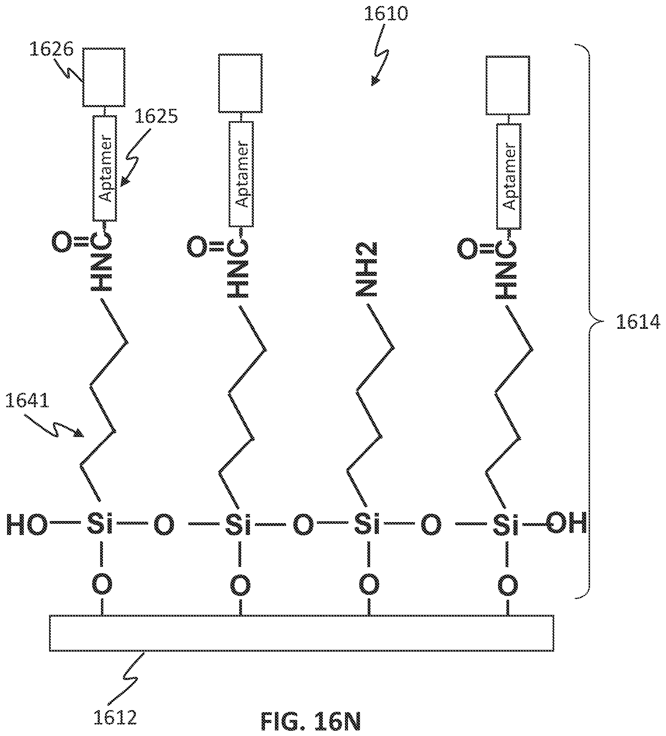

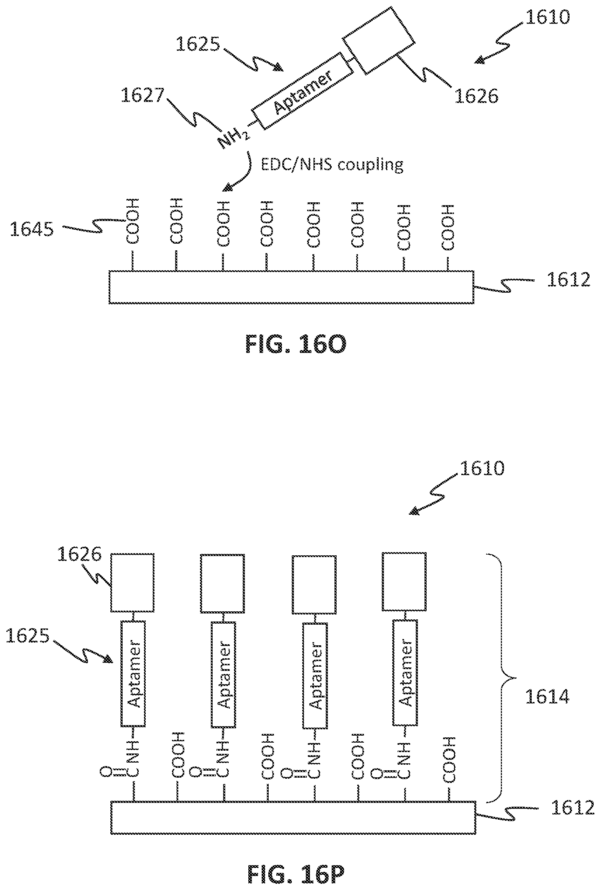

Examples of a working electrode 1610 comprising electrode material 1612 and an aptamer-based cortisol-sensing biorecognition layer 1614 are shown in FIGS. 16K, 16L, 16N, and 16P. In a first example variation shown in FIG. 16K, the electrode material 1612 may be platinum, gold, palladium, iridium, rhodium, ruthenium, titanium, nickel, alloys of the aforementioned metals, cobalt chrome alloy, cobalt chrome molybdenum alloy, stainless steel 316L, iridium oxide, titanium nitride, carbon, doped diamond, silicon, doped silicon, or other suitable material. The carbon may be pyrolytic carbon, pyrolytic graphite, or glassy carbon.

The biorecognition layer 1614 may comprise a conducting polymer layer 1623 with cortisol-binding aptamers 1625 tethered to the conducting polymer layer 1623, optionally via a linker. The linker may be an amide linker formed from a carboxyl group and a primary amine group. In some variations, the carboxyl group may be replaced with NHS-ester, isocyanate, isothiocyanate, or benzoyl fluoride. The present disclosure also provides for aptamers tethered via other linkers, such as a triazole linker, or a thioether linker. The thioether linker may be based on a combination of a maleimide moiety on the conductive polymer and a thiol moiety on an end of the aptamer, a combination of a vinyl surface attached to the conductive polymer layer and a thiol moiety on an end of the aptamer, or a combination of epoxide moiety on the conductive polymer and a thiol moiety on an end of the aptamer.

The cortisol-binding aptamers 1625 may be functionalized with a redox-active molecule 1626, by way of example methylene blue (MB), ferrocene, pentamethyl ferrocene, C5-ferrocene, Nile blue, thionine, anthraquinone, C5-anthraquinone, gallocyanine, indophenol, neutral red, dabcyl, or carboxy-X-rhodamine.

The conducting polymer layer 1623 may be between about 10 nm and about 100 nm in thickness and comprises a conducting polymer and optionally a counter ion(s). Examples of the conducting polymer include one or more of aniline, pyrrole, pyrrole-3-carboxylic acid, pyrrole-1-propionic acid, acetylene, phenylene, phenylene vinylene, phenylenediamine, thiophene, 3-methylthiophene, 3-hexylthiophene, 3-thiophene carboxylic acid, EDOT, EDOT carboxylic acid, and aminophenylboronic acid. The conducting polymer may also be a copolymer of two or more of the monomers listed. Examples of the counter-ion(s) include sulfate, bisulfate, nitrate, bromide, perchlorate, hexafluorophosphate, tetrafluoroborate, para-toluenesulfonate, benzenesulfonate, camphor-10-sulfonate, trifluoromethanesulfonate, bis(trifluoromethylsulfonyl)imide, dodecylbenzenesulfonate, poly(styrene sulfonate), poly(styrene sulfonate-co-acrylic acid), poly(acrylic acid), poly(methacrylic acid), poly(acrylic acid-co-acrylamide), sulfonated branched polytetrafluoroethylene (ie. Nafion), poly(maleic acid), poly(maleic acid-co-acrylic acid), poly(maleic acid-co-acrylamide), poly(2-acrylamido-2-methylpropane sulfonic acid), poly(styrene sulfonate-block-butylene-ran-ethylene-block-styrene sulfonate), alginate, glycosaminoglycans, hyaluronic acid, collagen, and any combination of the aforementioned polymer as copolymers or block copolymers not explicitly mentioned.

In a second example variation shown in FIG. 16L, the electrode material 1612 may be gold, and the biorecognition layer 1614 may comprise cortisol-sensing aptamers 1625 functionalized with a redox reporter molecule 1626, by way of example methylene blue (MB), ferrocene, pentamethyl ferrocene, C5-ferrocene, Nile blue, thionine, anthraquinone, C5-anthraquinone, gallocyanine, indophenol, neutral red, dabcyl, or carboxy-X-rhodamine. The cortisol-sensing aptamers may be tethered to the electrode material 1612 via a thiol linker 1628. Optionally, biorecognition layer 1614 further comprises other thiol-group-comprising small molecules 1629 (which may be referred to herein as "small-molecule thiols") tethered to the electrode material 1612. The small-molecule thiols may act as chemically neutral spacers that provide a desired surface density of cortisol-sensing aptamers 1625 on the surface of electrode material 1612. The small-molecule thiols may also act to passivate the exposed gold surface from unwanted chemical reactions, like the reduction of oxygen. In some variations, the small-molecule thiol has the following structure:

HS-Cx-Z (formula 1)

wherein X has a value of 2 to 16, which composes a linear, non-branching hydrocarbon chain, and wherein Z is a terminal functional group selected from the groups consisting of: hydrogen, hydroxyl, carboxyl, amine, trimethyl ammonium, phosphatidyl choline, sulfonate, sulfate ester, phosphonate, phosphate ester, an oligomer of polyethylene glycol (PEG) 1 to 50 units long (also known as PEG/polyethylene oxide/PEO). The small-molecule thiols may also act to provide biocompatibility and non-fouling properties, by way of example by comprising a hydrophilic moiety on the end opposite from the thiol group tethered to the electrode material 1612. The small-molecule thiol 1629 comprising a hydrophilic moiety may be, by way of example, a zwitterion such as phosphatidylcholine. In some variations, the small-molecule thiols may comprise 6-mercapto-1-hexanol, 1-hexanethiol, 6-mercaptohexanoic acid, 6-mercapto-1-hexanamine, 6-mercapto-1-phosphatidylcholine hexane, and the entire homologous series of those previously mentioned thiols with different carbon chain lengths.

In a third example variation shown in FIG. 16N, the electrode material 1612 may be a silicon that is optionally doped. Biorecognition layer 1614 may comprise a monolayer of silanes 1641 bound to the silicon surface, and cortisol-sensing aptamers 1625 functionalized with a redox reporter molecule 1626 (by way of example methylene blue, ferrocene, pentamethyl ferrocene, C5-ferrocene, Nile blue, thionine, anthraquinone, C5-anthraquinone, gallocyanine, indophenol, neutral red, dabcyl, or carboxy-X-rhodamine) that are tethered to the silicon electrode material optionally via amide linkers.

In a fourth example variation shown in FIG. 16P, the electrode material 1612 may be a carbon, optionally pyrolytic carbon, pyrolytic graphite, or glassy carbon. Biorecognition layer 1614 may comprise cortisol-sensing aptamers 1625 functionalized with a redox reporter molecule 1626 (by way of example methylene blue (MB), ferrocene, pentamethyl ferrocene, C5-ferrocene, Nile blue, thionine, anthraquinone, C5-anthraquinone, gallocyanine, indophenol, neutral red, dabcyl, or carboxy-X-rhodamine) that are tethered to the carbon electrode material optionally via amide linkers.

The working electrode 1610 may further include, as shown in FIG. 16A, in some variations, a biocompatible layer 1616 that provides for a biocompatible interface to, for example, reduce the foreign body response while allowing passage of cortisol to reach the cortisol-binding aptamers 1625. The biocompatible layer may comprise one or more hydrophilic polymers, by way of example poly(urethane), poly(N-vinylpyrrolidone), poly(acrylamide), poly(acrylic acid), poly(methacrylic acid), poly(2-hydroxyethyl methacrylate), poly(acrylic acid-co-acrylamide), poly(N-isopropyl acrylamide), poly(2-acrylamido-2-methylpropane sulfonic acid), poly(ethylene glycol), poly(vinyl alcohol), poly(lactic acid), poly(glycolic acid), poly(glycolic acid-co-lactic acid), collagen, alginate, hyaluronic acid, heparin, glycosaminoglycans, chitosan, Nafion, carboxymethylcellulose, or cellulose acetate. The biocompatible layer 1616 may be applied over the biorecognition layer 1614 and/or diffusion limiting layer 1615 (if present) by means of at least one of spray coating, dip coating, drop casting, plasma vapor deposition, and electro-deposition. In some variations, the biocompatible layer 1616 may be omitted, by way of example if the biorecognition layer 1614 comprises hydrophilic moieties that serve to reduce the foreign body response while allowing cortisol to reach the cortisol binding aptamers 1625.

### Counter electrode

As described above, the counter electrode is the electrode that is sourcing or sinking electrons (via an electrical current) required to sustain the electrochemical reaction at the working electrode. The number of counter electrode constituents can be augmented in the form of a counter electrode array to enhance surface area such that the current-carrying capacity of the counter electrode does not limit the change in electron transfer properties between the redox-active molecule and the electrode material 1612 of the working electrode. It thus may be desirable to have an excess of counter electrode area versus the working electrode area to circumvent the current-carrying capacity limitation. If the working electrode is operated as an anode, the counter electrode will serve as the cathode and vice versa. Similarly, if an oxidation reaction occurs at the working electrode, a reduction reaction occurs at the counter electrode and vice versa. Unlike the working or reference electrodes, the counter electrode is permitted to dynamically swing to electrical potentials required to sustain the change in electron transport properties of the working electrode.

As shown in FIG. 16B, a counter electrode 1620 may include an electrode material 1622, similar to electrode material 1612. For example, like the electrode material 1612, the electrode material 1622 in counter electrode 1620 may include gold, platinum, palladium, iridium, rhodium, ruthenium, titanium, nickel, alloys of the aforementioned metals, cobalt chrome alloy, cobalt chrome molybdenum alloy, stainless steel 316L, iridium oxide, titanium nitride, carbon, doped diamond, silicon, doped silicon, or other suitable material.

In some variations, counter electrode 1620 may have few or no additional layers over the electrode material 1622 (as shown in FIG. 16B). However, in some variations the counter electrode 1620 may benefit from increase surface area to increase the amount of current it can support. For example, the counter electrode material 1622 may be textured or otherwise roughened in such a way to augment the surface area of the electrode material 1622 for enhanced current sourcing or sinking ability. Additionally or alternatively, the counter electrode 1620 may include a layer of platinum black 1624 as shown in FIG. 16E, which may augment electrode surface as described above with respect to some variations of the working electrode. However, in some variations of the counter electrode, the layer of platinum black may be omitted (by way of example as shown in FIGS. 16B and 16H).

Additionally or alternatively, in some variations as shown in FIG. 16H, the counter electrode 1620 may include a biocompatible layer 1626 arranged over the electrode in order to, for example, reduce the foreign body response. The biocompatible layer 1626 may, for example, be similar in structure and composition to the biocompatible layer 1616 described above with respect to FIG. 16A.

### Reference electrode

As described above, the reference electrode functions to provide a reference potential for the system; that is, the electrical potential at which the working electrode is biased is referenced to the reference electrode. A fixed or at least controlled potential relationship may be established between the working and reference electrodes, and within practical limits no current is sourced from or sinked to the reference electrode.

As shown in FIG. 16C, a reference electrode 1630 may include an electrode material 1632, similar to electrode material 1612. In some variations, like the electrode material 1612, the electrode material 1632 in the reference electrode 1630 may include a metal salt or metal oxide, which serves as a stable redox coupled with a well-known electrode potential. For example, the metal salt may, for example, include silver-silver chloride (Ag/AgCl) and the metal oxide may include iridium oxide (IrOx / Ir₂O₃ / IrO₂). In other variations, noble and inert metal surfaces, and those coated with a conductive polymer such as poly(3,4-ethylenedioxythiophene), may function as quasi-reference electrodes and include gold, platinum, palladium, iridium, carbon, doped diamond, and/or other suitable catalytic and inert material. Furthermore, in some variations the reference electrode 1630 may be textured or otherwise roughened in such a way to enhance adhesion with any subsequent layers. Such subsequent layers on the electrode material 1632 may include a platinum black layer 1634 (as shown in FIG. 16F), and/or biocompatible layer 1637 (as shown in FIG. 16I). The biocompatible layer 1637 may, for example, be similar to the biocompatible layer 1616 described above with respect to FIG. 16A. In some variations, the platinum black layer may be omitted (e.g., as shown in FIGS. 16C and 16I).

The reference electrode 1630 may, in some variations, further include a redox-couple layer 1636, which may contain a surface-immobilized, solid-state redox couple with a stable thermodynamic potential. For example, the reference electrode may operate at a stable standard thermodynamic potential with respect to a standard hydrogen electrode (SHE). The high stability of the electrode potential may be attained by employing a redox system with constant (e.g., buffered or saturated) concentrations of each participant of the redox reaction. For example, the reference electrode may include a metal with an Ag/AgCl salt film (E = +0.197V vs. SHE) or IrOx (E = +0.177 vs. SHE, pH = 7.00) in the redox-couple layer 1636. In some variations, the reference electrode may be used as a half-cell to construct a complete electrochemical cell.

### Exemplary electrode layer formation processes

Various layers of the working electrode, counter electrode, and reference electrode may be applied to the microneedle array and/or functionalized, etc. using suitable processes such as those described below.

In a pre-processing step for the microneedle array, the microneedle array may be plasma cleaned in an inert gas (e.g., RF-generated inert gas such as argon) plasma environment to render the surface of the material, including the electrode material (e.g., electrode material 1612, 1622, and 1632 as described above), to be more hydrophilic and chemically reactive. This pre-processing functions to not only physically remove organic debris and contaminants, but also to clean and prepare the electrode surface to enhance adhesion of subsequently deposited films on its surface.

### Working electrode

*Anodization:* To configure the working electrode after the pre-processing step, the electrode material 1612 may undergo an anodization treatment using an amperometry approach in which the electrode constituent(s) assigned for the working electrode function is (are) subject to a fixed high anodic potential (e.g., between +1.0 V and +1.3 V vs. Ag/AgCl reference electrode) for a suitable amount of time (e.g., between about 30 sec and about 10 min) in a moderate-strength acid solution (e.g., between 0.1 M and 3M H₂SO₄). In this process, a thin, yet stable native oxide layer may be generated on the electrode surface. Owing to the low pH arising at the electrode surface, any trace contaminants may be removed as well.

In an alternative embodiment using a coulometry approach, anodization can proceed until a specified amount of charge has passed (measured in Coulombs). The anodic potential may be applied as described above; however, the duration of this might vary until the specified amount of charge has passed.

*Activation:* Following the anodization process, the working electrode constituents may be subjected to a cyclically-scanned potential waveform in an activation process using cyclic voltammetry. In the activation process, which may occur in a moderate-strength acid solution (e.g. 0.1 - 3M H₂SO₄), the potential applied may time-varying in a suitable function (e.g., sawtooth or triangular function). For example, the voltage may be linearly scanned between a cathodic value (e.g., between -0.3 V and -0.2 V vs. Ag/AgCl reference electrode) and an anodic value (e.g., between +1.0 V and +1.3 V vs. Ag/AgCl reference electrode) in an alternating function (e.g., between 15 and 50 linear sweep segments). The scan rate of this waveform can take on a value between 1 mV/sec and 1000 mV/sec. It should be noted that a current peak arising during the anodic sweep (sweep to positive extreme) corresponds to the oxidation of a chemical species, while the current peak arising during the ensuing cathodic sweep (sweep to negative extreme) corresponds to the reduction of said chemical species.

*Electrodeposition of Gold:* Following the activation and cleaning process, the working electrode constituents may be subjected to a cyclically-scanned potential waveform or a constant potential to electrodeposit gold metal onto the surface. In the deposition process, which may occur in dilute concentrations of gold complexes (e.g., 0.5% to 3% by weight AuCl₃ or Au(CN)₂), the potential applied may vary with time in a suitable function. For example, the voltage may be linearly scanned between an anodic value (e.g., between +0.2 V and +1.2 V vs. Ag/AgCl reference electrode) in an alternating function (e.g., 10 to 30 linear sweep segments). The scan rate of this waveform can take on a value between 100 mV/sec and 1000 mV/sec. It should be noted that a rise in current during the cathodic sweep (sweep to negative extreme) corresponds to the reduction of gold species in solution and plating to the working electrode surface. It should also be noted that a sharp increase in current during the first scan is due to the formation of nucleation sites.

*Activation of the gold surface:* Whether the gold electrode is plated onto the native electrode material applied at the foundry during microfabrication, such as platinum, or the gold has been deposited by the foundry itself, both types of gold electrodes require electrochemical activation. Activation may begin in a basic solution (e.g., 0.1 M to 3 M NaOH), and the potential applied may be time-varying in a suitable function (e.g., triangle function) such as cyclic voltammetry. For example, the voltage may be linearly scanned between a cathodic value (e.g., between -2.0 V and -1.2 V vs. Ag/AgCl reference electrode) and an anodic value (e.g., between - 1.1 V and -0.5 V vs. Ag/AgCl) in an alternating function (e.g., 50 to 1000 sweep segments). The scan rate of this waveform can take on a value between 10 mV/s and 2000 mV/s. After potential cycling in alkaline media the gold electrodes must be cycled in an acidic solution (e.g., between 0.1 M and 3 M H₂SO₄). The potential may be time-varying in a suitable function (e.g., triangle function). For example, the voltage me be linearly scanned between a cathodic value (e.g., between -0.2 V and +0.2 V vs. Ag/AgCl reference electrode) and an anodic value (e.g., between +1.2 V and +1.8 V vs. Ag/AgCl reference electrode) in an alternating function (e.g., 50 to 1000 sweep segments). The scan rate of this waveform can take on a value between 10 mV/s and 2000 mV/s. It should be noted that in both basic and acidic media, a current peak arising during the anodic sweep (sweep to positive extreme) corresponds to the oxidation of a chemical species, while the current peak arising during the ensuing cathodic sweep (sweep to negative extreme) corresponds to the reduction of said chemical species.

*Functionalization of the biorecognition layer:* Following the activation process, the working electrode constituents may be functionalized with the biorecognition layer 1614 such as that described above. Assuming that the working electrode contingent of the microneedle array has undergone the aforementioned steps such as deposition and activation, the biorecognition layer may be applied in a variety of ways. The application process for the biorecognition layer may depend on various factors, including what material is used for the working electrode material 1612. Various exemplary processes are discussed herein below.

In some variations, the electrode material 1612 is electrochemically coated with a conductive polymer film through oxidation of the reactive monomer(s) at the electrode surface, either potentiostatically (e.g., chronoamperometry), potentiodynamically (e.g., cyclic voltammetry), or galvanostatically (e.g. chronopotentiometry). The conditions necessary for polymerization of the monomer(s) is dependent chemical properties of the conductive polymer being used. By adjusting current, potential, sweep rate, and/or the duration of the electrolytic process, the final properties of the conductive polymer film may be controlled (e.g., thickness, conductivity, permselectivity). When desired, the conductive polymer film may be overoxidized with a secondary oxidation step through a potentiostatic, potentiodynamic, or galvanostatic electrochemical process. This secondary oxidation step may be used to alter electrical conductivity and barrier properties of the conductive polymer film. The conductive polymer and/or counter ion(s) incorporated within the conductive polymer may provide pendent chemical moieties for covalent attachment to aptamers functionalized with a compatible moiety. By way of example, the pendant chemical moiety in the conductive polymer film may comprise a carboxyl group and the aptamer may be functionalized on the 3' or 5' end with a primary amine. Alternatively, the pendant chemical moiety in the conductive polymer film may be a primary amine and the aptamer may be functionalized on the 3' or 5' end with a carboxyl group.

In some variations, by way of example where the electrode material 1612 is gold, the aptamer may be functionalized on one terminal end with a reactive organosulfur compound (by way of example thiol/mercaptan, disulfide), which spontaneously reacts and attaches to the surface of the gold working electrode material. The remaining gold surface of the working electrode may optionally be passivated with small molecule(s) containing a terminal thiol group to form a self-assembled monolayer.

In an example variation as shown in FIG. 16J, the working electrode 1610 may be functionalized with an aqueous solution comprising a monomeric precursor of a conducting polymer and optionally a counter ion. Functionalization may take place through a galvanostatic electrodeposition (e.g., chronopotentiometry) where the anodic current density through the working electrode may be between 0.05 mA/cm² and 5 mA/cm² and the duration may be from about 1 minute to about 100 minutes. In other variations, the functionalization may take place through a chronocoulometric deposition, where the deposit charge may be between about 1 mC/cm² and 10 C/cm² and the duration may be from about 1 minute to about 100 minutes. The conducting polymer, the counter ion, or both may comprise carboxyl group 1621. In this process, as shown in FIG. 16J, a thin film (e.g., between about 10 nm and about 100 nm) of biorecognition layer 1614 comprising a conducting polymer layer 1623 may be generated (e.g., electrodeposited or electropolymerized) on the surface of electrode material 1612, which may include platinum. After the conducting polymer layer 1623 is formed, cortisol-sensing aptamers 1625 functionalized with a primary amine group 1267 on one end and functionalized with a redox-active molecule 1626 on the other end are introduced. The aptamers 1625 are covalently linked to the carboxyl groups 1621 comprised in the conductive polymer layer 1623 using a carbodiimide cross-linking method, by way of example EDC/NHS coupling that employs 1-ethyl-3-(3-dimethylaminopropyl) carbodiimide hydrochloride (EDC) and N-Hydroxysuccinimide (NHS). An alternative method for coupling a primary amine and carboxylic acid into an amide linkage is through DMTMM cross-linking with 4-(4,6-dimethoxy-1,3,5-triazin-2-yl)-4-methyl-morpholinium chloride. The remainder of pendent activated carboxylic acid groups on the silane layer not attached to aptamer may optionally be cross-linked to another amine containing molecule (e.g., ethanolamine, glycine, lysine, PEG-amine) to alter the working electrode surface properties and biocompatibility. The resulting functionalized working electrode 1610 is shown in FIG. 16K.

In some variations, the placement of the carboxyl group and the primary amine group for the carbodiimide cross-linking may be reversed, such that conducting polymer, the counter ion, or both may comprise the primary amine group and the aptamer may be functionalized with the carboxyl group. The two moieties may then be linked by EDC/NHS coupling or DMTMM cross-linking as noted above with respect to FIG. 16J. An example of a conducting polymer comprising a primary amine group is a p-Phenylenediamine (PPD)-based polymer.

Whereas FIGS. 16J-16K show aptamers tethered via an amide linker formed between a carboxyl group and a primary amine group, the present disclosure provides for the use of other moieties. By way of example, the carboxyl group may be replaced with NHS-ester, isocyanate, isothiocyanate, or benzoyl fluoride. The present disclosure also provides for aptamers tethered via other linkers, such as a triazole linker, or a thioether linker. The thioether linker may be based on a combination of a maleimide moiety on the conductive polymer and a thiol moiety on an end of the aptamer, a combination of a vinyl surface attached to the conductive polymer layer and a thiol moiety on an end of the aptamer, or a combination of epoxide moiety on the conductive polymer and a thiol moiety on an end of the aptamer.

In an example variation shown in FIG. 16L a gold working electrode material 1612 may be functionalized with an aqueous solution containing aptamer 1625 with a terminal thiol groups 1628 on one end and a redox active molecule 1626 on the other end. Functionalization may proceed for between about 30 minutes to about 10 hours, and the concentration of aptamer may be between about 0.1 and about 2 uM. The functionalization process may include an optional secondary step ("backfilling") to functionalize the remainder of the surface of the gold working electrode with a small molecule thiol 1629 in order to passivate the surface and/or add biocompatible functionality. Backfilling the surface of the electrode material 1612 with small-molecule thiols 1629 may take place in an aqueous solution or an organic solution (e.g. methanol, ethanol, isopropanol) at a concentration of about 0.1 to about 5 mM for about 3 to 30 hours. After this process, as shown in FIG. 16L, a monolayer of the aptamer 1625 is immobilized on the surface together with the optionally backfilled small molecule thiol 1629 serving to passivate the remainder of the surface of the working electrode material 1612.

The gold-to-thiol-based functionalization as shown in FIG. 16L may be achieved through an alternative functionalization process, in which a gold working electrode 1612 is functionalized using an organic solution (e.g., methanol, ethanol, isopropanol) containing both an aptamer 1625 (functionalized on one end with a terminal thiol groups 1628 and a redox active molecule 1626 on the other end) as well as a small molecule thiol 1629 which is added to passivate the surface and/or add biocompatible functionality. Functionalization may proceed for between about 3 hours to about 30 hours. The concentration of the aptamer 1625 may be between about 0.1 uM and about 2 uM, and the concentration of the small molecule thiol 1629 may be about 0.1 mM to about 5 mM. In the resulting working electrode 1610 as shown in FIG. 16L, a monolayer of the biorecognition element is immobilized on the surface with the optional addition of small molecule thiol 1629 shown passivating the remainder of the surface.

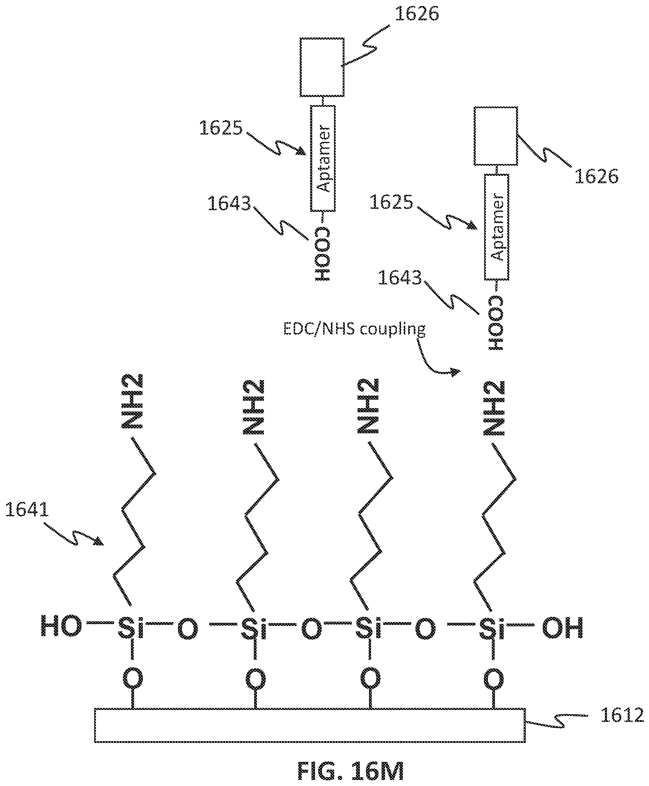

In another example variation shown in FIG. 16M, a working electrode 1610 comprising a silicon electrode material 1612 may be functionalized with an aptamer 1625 via silane chemistry. The silicon electrode material may comprise doped silicon. First, the surface of the silicon electrode material 1612 is activated by exposure to oxygen plasma at a pressure between about 200 mTorr and about 700 mTorr for about 0.5 min to about 10 min, at an operating power of about 50 W to about 500 W. The activated electrode material surface is then moved to a vacuum chamber at a pressure of about 1 Torr to about 100 Torr containing a small volume of a suitable reactive silane 1641 comprising a terminal primary amine (by way of example 4-aminobutyltriemethoxysilane as shown in FIG. 16M) for 10 to 90 min. Afterwards, the working electrodes are removed and rinsed with an organic solvent(s) (by way of example methanol, ethanol, or isopropanol) then baked at about 80°C to about 140°C for a duration of about 10 min to about 60 min to cure the adhered silane 1641. FIG. 16M schematically shows a silicon electrode material 1612 with a monolayer of adhered silanes 1641. Also as shown in FIG. 16M, the electrode material is treated with a solution containing an aptamer 1625 terminating with a carboxyl group 1643 at one end and a redox active molecule 1626 on the other end. The solution may be an aqueous solution such as a phosphate buffered solution, with the aptamers at a concentration of about 0.1 uM to 2 uM. The carboxyl group 1643 forms an amide linker with the pendent amine groups of the adhered silanes 1641, resulting in the aptamer 1625 being tethered to the electrode material 1612. The resulting biorecognition layer 1614 comprising the tethered aptamers 1625 and silanes 1641 that is formed on the silicon electrode material 1612 is shown in FIG. 16N. The amide linker may be formed via a EDC/NHS cross-linking chemistry, or a DMTMM cross-linking chemistry. The remainder of pendent amine groups (not shown) on the silane layer not attached to an aptamer may optionally be cross-linked to another carboxyl group-containing molecule (eg. glycolic acid, oxalic acid, glycine, PEG-carboxylic acid) to alter the working electrode surface properties and biocompatibility.

In an example variation shown in FIG. 16O, a working electrode 1610 comprising a glassy carbon electrode material 1612 may be functionalized with an aptamer via activation of the carbon surface and formation of an amide bond that tethers the aptamer to the carbon surface. In a first step, the surface of the glassy carbon electrode material 1612 is activated electrochemically by a potentiostatic hold at 1.2 V to 2.0 V for 1 to 60 min in an acidic solution (by way of example a 0.1 M to 3 M H₂SO₄ solution). The activation step forms terminal carboxyl groups 1645 on the surface of the carbon electrode material 1612. The activated surface of the electrode material 1612 is then functionalized with an aptamer 1625 having a primary amine group 1627 at its 3' or 5' end and a redox active molecule 1626 on the other end. The carboxyl groups 1645 form an amide linker with the amine groups 1627, resulting in the aptamers 1625 being tethered to the electrode material 1612. The resulting biorecognition layer 1614 comprising the tethered aptamers 1625 that is formed on the carbon electrode material 1612 is shown in FIG. 16P. The amide linker may be formed via a EDC/NHS cross-linking chemistry, or a DMTMM cross-linking chemistry.

Whereas FIGS. 16M-16P show aptamers tethered via an amide linker formed between a carboxyl group and a primary amine group, the present disclosure provides for the use of other moieties. By way of example, the carboxyl group may be replaced with NHS-ester, isocyanate, isothiocyanate, or benzoyl fluoride. The present disclosure also provides for aptamers tethered via other linkers, such as a triazole linker, or a thioether linker. The thioether linker may be based on a combination of a maleimide moiety on the conductive polymer and a thiol moiety on an end of the aptamer, a combination of a vinyl surface attached to the conductive polymer layer and a thiol moiety on an end of the aptamer, or a combination of epoxide moiety on the conductive polymer and a thiol moiety on an end of the aptamer.

In some variations, the working electrode surface may be electrochemically roughened in order to enhance adhesion of the biorecognition layer to the electrode material 1612 surface (and/or Pt black layer). The roughening process may involve a cathodization treatment (e.g., cathodic deposition, a subset of amperometry) wherein the electrode is subject to a fixed cathodic potential (e.g., between -0.4 V and +0.2 V vs. Ag/AgCl reference electrode) for a certain amount of time (e.g., 5 sec to 10 min) in an acid solution containing the desired metal cation dissolved therein (e.g., 0.01 mM to 100 mM H₂PtCl₆). Alternatively, the electrode is subject to a fixed cathodic potential (e.g., between about -0.4 V to about +0.2 V vs. Ag/AgCl reference electrode) until a certain amount of charge has passed (e.g., 0.1 mC - 100 mC) in an acid solution containing the desired metal cation dissolved therein (e.g., 0.01 mM to 100 mM H₂PtCl₆). In this process, a thin, yet highly porous layer of the metal may be generated on the electrode surface, thereby augmenting the electrode surface area dramatically. Additionally or alternatively, in some variations as described above, elemental platinum metal may deposited on the electrode to form or deposit a platinum black layer 1613.

### Counter electrode

*Anodization:* In some variations, the counter electrode material may undergo an anodization treatment using an amperometry approach in which the electrode constituent(s) assigned for the counter electrode function is subject to a fixed high anodic potential or a suitable amount of time in a moderate-strength acid solution. Exemplary parameters and other specifics of the anodization process for the counter electrode may be similar to that described above for the working electrode. Similarly, anodization for the counter electrode may alternatively use a coulometry approach as described above.

*Activation:* In some variations, following the anodization process, the counter electrode constituents may be subjected to a cyclically-scanned potential waveform in an activation process using cyclic voltammetry. In some variations, the activation process may be similar to that described above for the working electrode.

*Roughening:* Furthermore, in some variations, the counter electrode surface may be electrochemically roughened in order to enhance the current-sinking or current-sourcing capacity of this electrode contingent. The electrochemical roughening process may be similar to that described above for the working electrode. Additionally or alternatively, in some variations as described above, elemental platinum metal may deposited on the electrode to form or deposit a platinum black layer 1624.

### Reference electrode

*Anodization:* Like the working and counter electrodes as described above, the reference electrode may undergo an anodization treatment using an amperometry approach in which the electrode constituent(s) assigned for the counter electrode function is subject to a fixed high anodic potential or a suitable amount of time in a moderate-strength acid solution. Exemplary parameters and other specifics of the anodization process for the counter electrode may be similar to that described above for the working electrode. Similarly, anodization for the counter electrode may

*Activation:* Following the anodization process, the reference electrode constituents may be subjected to a cyclically-scanned potential waveform in an activation process using cyclic voltammetry. In some variations, the activation process may be similar to that described above for the working electrode.

*Functionalization:* Following the activation process, the reference electrode constituents may be functionalized. Assuming that the reference electrode contingent of the microneedle array has undergone the aforementioned steps, a fixed anodic potential (e.g., between +0.4 - +1.0 V vs. Ag/AgCl reference electrode) may be applied for a certain suitable duration (e.g., between about 10 sec and about 10 min) in an aqueous solution. Alternatively, the reference electrode is subject to a fixed anodic potential (e.g., between about +0.4 V to about +1.0 V vs. Ag/AgCl reference electrode) until a certain amount of charge has passed (e.g., 0.01 mC - 10 mC) in an aqueous solution. In some variations, the aqueous solution may include a monomeric precursor to a conducting polymer and a charged dopant counter ion or material (e.g., poly(styrene sulfonate)) carrying an opposing charge. In this process, a thin film (e.g., between about 10 nm and about 10,000 nm) of a conducting polymer with a dispersed counter ion or material may be generated on the reference electrode surface. This creates a surface-immobilized, solid-state redox coupled with a stable thermodynamic potential. In some variations, the conducting polymer may include one or more of aniline, pyrrole, acetylene, phenylene, phenylene vinylene, phenylene diamine, thiophene, 3,4-ethylenedioxythiophene, and aminophenylboronic acid.

In some alternative embodiments, a native iridium oxide film (e.g., IrO₂ or Ir₂O₃ or IrO₄) may be electrochemically grown on an iridium electrode surface in an oxidative process. This also creates a stable redox couple, as discussed above.

Furthermore, in some variations the reference electrode surface may be electrochemically roughened in order to enhance adhesion of the surface-immobilized redox couple. The electrochemical roughening process may be similar to that described above for the working electrode. Additionally or alternatively, in some variations as described above, elemental platinum metal may deposited on the electrode to form or deposit a platinum black layer 1633.

### Microneedle array configurations

Multiple microneedles (e.g., any of the microneedle variations described herein, each of which may have a working electrode, counter electrode, or reference electrode as described above) may be arranged in a microneedle array. Considerations of how to configure the microneedles include factors such as desired insertion force for penetrating skin with the microneedle array, optimization of electrode signal levels and other performance aspects, manufacturing costs and complexity, etc.

For example, the microneedle array may include multiple microneedles that are spaced apart at a predefined pitch (distance between the center of one microneedle to the center of its nearest neighboring microneedle). In some variations, the microneedles may be spaced apart with a sufficient pitch so as to distribute force (e.g., avoid a "bed of nails" effect) that is applied to the skin of the user to cause the microneedle array to penetrate the skin. As pitch increases, force required to insert the microneedle array tends to decrease and depth of penetration tends to increase. However, it has been found that pitch only begins to affect insertion force at low values (e.g., less than about 150 µm). Accordingly, in some variations the microneedles in a microneedle array may have a pitch of at least 200 µm, at least 300 µm, at least 400 µm, at least 500 µm, at least 600 µm, at least 700 µm, or at least 750 µm. For example, the pitch may be between about 200 µm and about 800 µm, between about 300 µm and about 700 µm, or between about 400 µm and about 600 µm. In some variations, the microneedles may be arranged in a periodic grid, and the pitch may be uniform in all directions and across all regions of the microneedle array. Alternatively, the pitch may be different as measured along different axes (e.g., X, Y directions) and/or some regions of the microneedle array may include a smaller pitch while other may include a larger pitch.

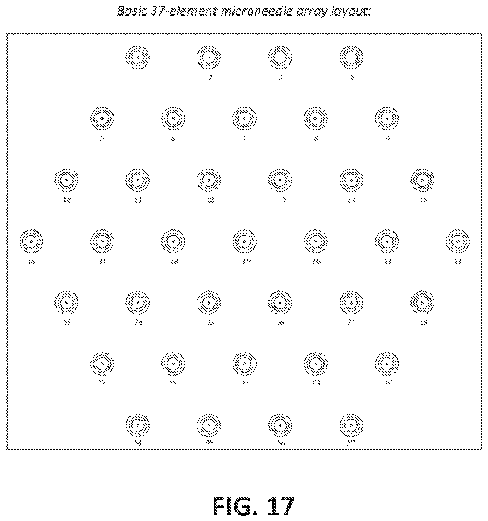

Furthermore, for more consistent penetration, microneedles may be spaced equidistant from one another (e.g., same pitch in all directions). To that end, in some variations, the microneedles in a microneedle array may be arranged in a hexagonal configuration as shown in FIG. 17. Alternatively, the microneedles in a microneedle array may arranged in a rectangular array (e.g., square array), or in another suitable symmetrical manner.

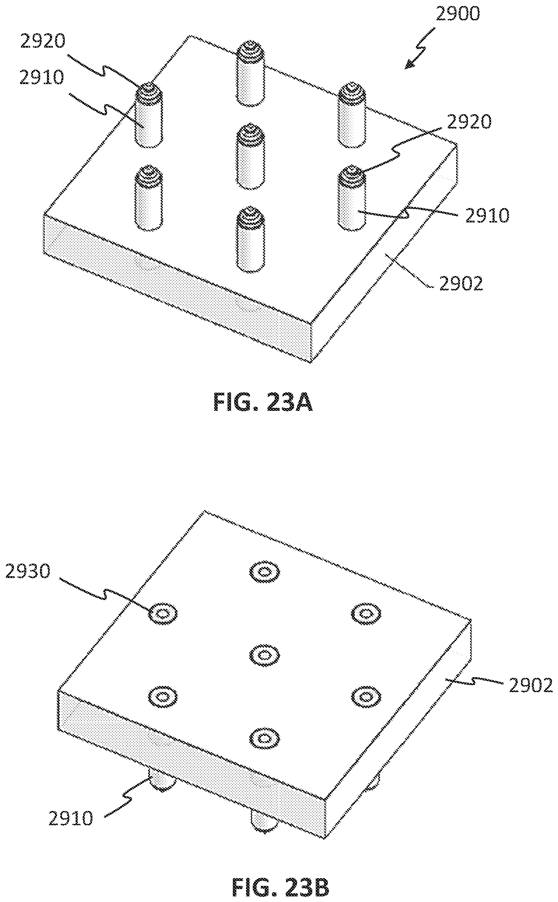

Another consideration for determining configuration of a microneedle array is overall signal level provided by the microneedles. Generally, signal level at each microneedle is invariant of the total number of microneedle elements in an array. However, signal levels can be enhanced by electrically interconnecting multiple microneedles together in an array. For example, an array with a large number of electrically connected microneedles is expected to produce a greater signal intensity (and hence increased accuracy) than one with fewer microneedles. However, a higher number of microneedles on a die will increase die cost (given a constant pitch) and will also require greater force and/or velocity to insert into skin. In contrast, a lower number of microneedles on a die may reduce die cost and enable insertion into the skin with reduced application force and/or velocity. Furthermore, in some variations a lower number of microneedles on a die may reduce the overall footprint area of the die, which may lead to less unwanted localized edema and/or erythema. Accordingly, in some variations, a balance among these factors may be achieved with a microneedle array including 37 microneedles as shown in FIG. 17 or a microneedle array including 7 microneedles are shown in FIGS. 23A and 23B. However, in other variations there may be fewer microneedles in an array (e.g., between about 5 and about 35, between about 5 and about 30, between about 5 and about 25, between about 5 and about 20, between about 5 and about 15, between about 5 and about 100, between about 10 and about 30, between about 15 and about 25, etc.) or more microneedles in an array (e.g., more than 37, more than 40, more than 45, etc.).

Additionally, as described in further detail below, in some variations only a subset of the microneedles in a microneedle array may be active during operation of the cortisol monitoring device. For example, a portion of the microneedles in a microneedle array may be inactive (e.g., no signals read from electrodes of inactive microneedles). In some variations, a portion of the microneedles in a microneedle array may be activated at a certain time during operation and remain active for the remainder of the operating lifetime of the device. Furthermore, in some variations, a portion of the microneedles in a microneedle array may additionally or alternatively be deactivated at a certain time during operation and remain inactive for the remainder of the operating lifetime of the device.

In considering characteristics of a die for a microneedle array, die size is a function of the number of microneedles in the microneedle array and the pitch of the microneedles. Manufacturing costs are also a consideration, as a smaller die size will contribute to lower cost since the number of dies that can be formed from a single wafer of a given area will increase. Furthermore, a smaller die size will also be less susceptible to brittle fracture due to the relative fragility of the substrate.

Furthermore, in some variations, microneedles at the periphery of the microneedle array (e.g., near the edge or boundary of the die, near the edge or boundary of the housing, near the edge or boundary of an adhesive layer on the housing, along the outer border of the microneedle array, etc.) may be found to have better performance (e.g., sensitivity) due to better penetration compared to microneedles in the center of the microneedle array or die. Accordingly, in some variations, working electrodes may be arranged largely or entirely on microneedles located at the periphery of the microneedle array, to obtain more accurate and/or precise cortisol measurements.

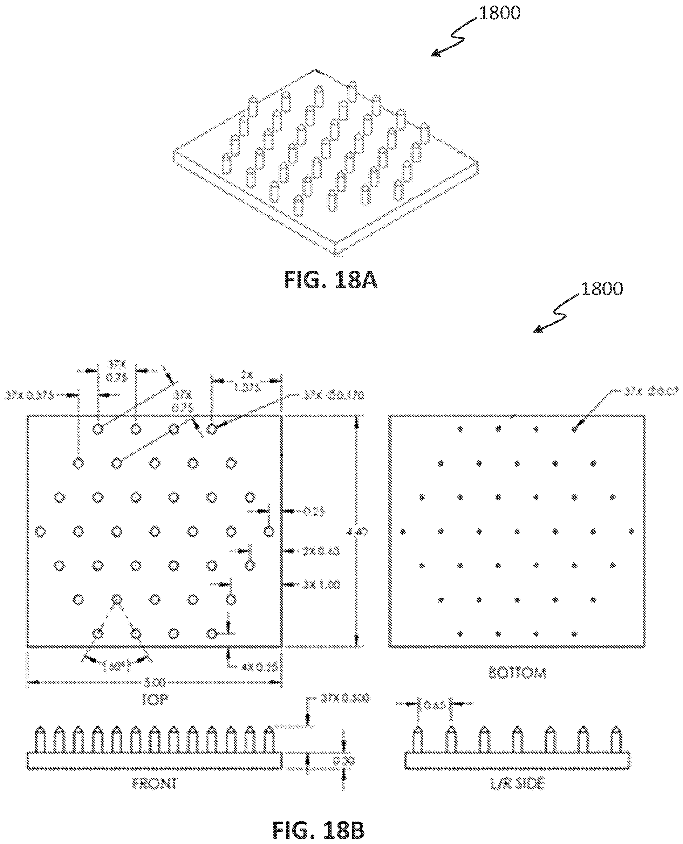

FIG. 17 depicts an illustrative schematic of 37 microneedles arranged in an example variation of a microneedle array. The 37 microneedles may, for example, be arranged in a hexagonal array with an inter-needle center-to-center pitch of about 750 µm (or between about 700 µm and about 800 µm, or between about 725 µm and about 775 µm) between the center of each microneedle and the center of its immediate neighbor in any direction. FIG. 18A depicts an illustrative schematic of an example variation of a die including the microneedle arrangement shown in FIG. 17. Example dimensions of the die (e.g., about 4.4 mm by about 5.0 mm) and the microneedle array are shown in FIG. 18B.

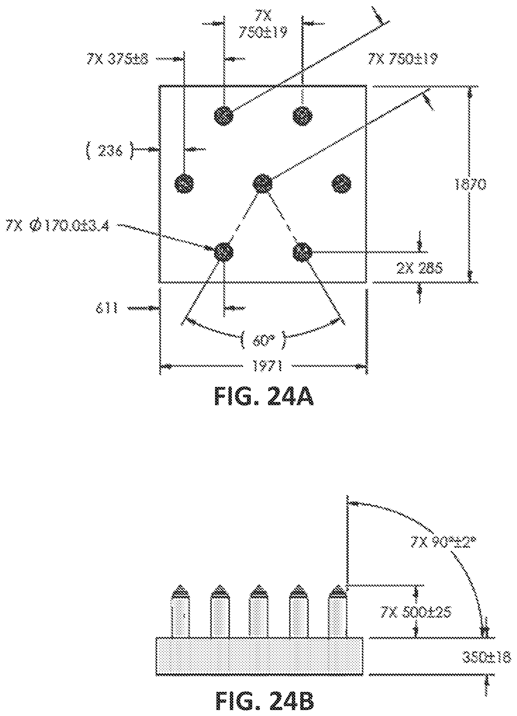

FIGS. 23A and 23B depict perspective views of an illustrative schematic of seven microneedles 2910 arranged in an example variation of a microneedle array 2900. The seven microneedles 2910 are arranged in a hexagonal array on a substrate 2902. As shown in FIG. 23A, the electrodes 2920 are arranged on distal portions of the microneedles 2910 extending from a first surface of the substrate 2902. As shown in FIG. 23B, proximal portions of the microneedles 2910 are conductively connected to respective backside electrical contacts 2930 on a second surface of the substrate 2902 opposite the first surface of the substrate 2902. FIGS. 24A and 30B depict plan and side views of an illustrative schematic of a microneedle array similar to microneedle array 2900. As shown in FIGS. 24A and 24B, the seven microneedles are arranged in a hexagonal array with an inter-needle center-to-center pitch of about 750 µm between the center of each microneedle and the center of its immediate neighbor in any direction. In other variations the inter-needle center-to-center pitch may be, for example, between about 700 µm and about 800 µm, or between about 725 µm and about 775 µm. The microneedles may have an approximate outer shaft diameter of about 170 µm (or between about 150 µm and about 190 µm, or between about 125 µm and about 200 µm) and a height of about 500 µm (or between about 475 µm and about 525 µm, or between about 450 µm and about 550 µm).

Furthermore, the microneedle arrays described herein may have a high degree of configurability concerning where the working electrode(s), counter electrode(s), and reference electrode(s) are located within the microneedle array. This configurability may be facilitated by the electronics system.

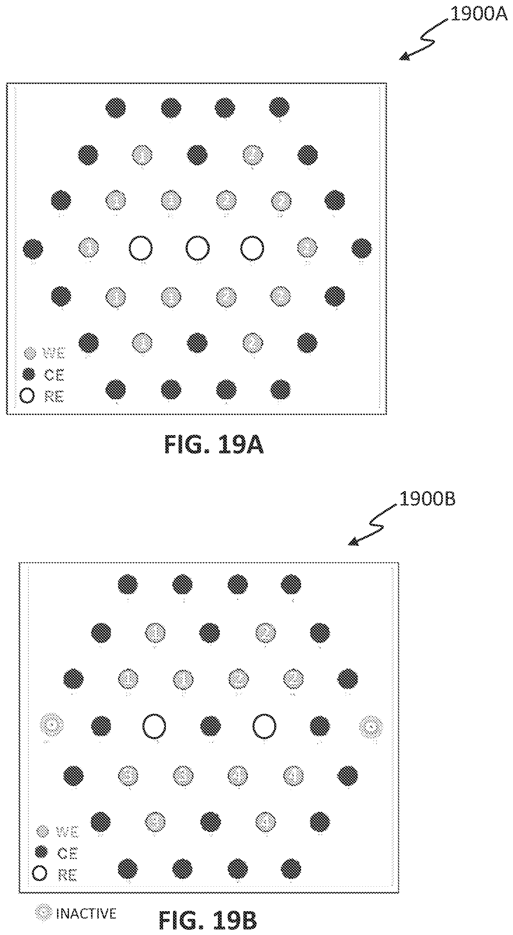

In some variations, a microneedle array may include electrodes distributed in two or more groups in a symmetrical or non-symmetrical manner in the microneedle array, with each group featuring the same or differing number of electrode constituents depending on requirements for signal sensitivity and/or redundancy. For example, electrodes of the same type (e.g., working electrodes) may be distributed in a bilaterally or radially symmetrical manner in the microneedle array. For example, FIG. 19A depicts a variation of a microneedle array 1900A including two symmetrical groups of seven working electrodes (WE), with the two working electrode groups labeled "1" and "2". In this variation, the two working electrode groups are distributed in a bilaterally symmetrical manner within the microneedle array. The working electrodes are generally arranged between a central region of three reference electrodes (RE) and an outer perimeter region of twenty counter electrodes (CE). In some variations, each of the two working electrode groups may include seven working electrodes that are electrically connected amongst themselves (e.g., to enhance sensor signal). Alternatively, only a portion of one or both of the working electrode groups may include multiple electrodes that are electrically connected amongst themselves. As yet another alternative, the working electrode groups may include working electrodes that are standalone and not electrically connected to other working electrodes. Furthermore, in some variations the working electrode groups may be distributed in the microneedle array in a non-symmetrical or random configuration.

As another example, FIG. 19B depicts a variation of a microneedle array 1900B including four symmetrical groups of three working electrodes (WE), with the four working electrode groups labeled "1", "2", "3", and "4." In this variation, the four working electrode groups are distributed in a radially symmetrical manner in the microneedle array. Each working electrode group is adjacent to one of two reference electrode (RE) constituents in the microneedle array and arranged in a symmetrical manner. The microneedle array also includes counter electrodes (CE) arranged around the perimeter of the microneedle array, except for two electrodes on vertices of the hexagon that are inactive or may be used for other features or modes of operation.

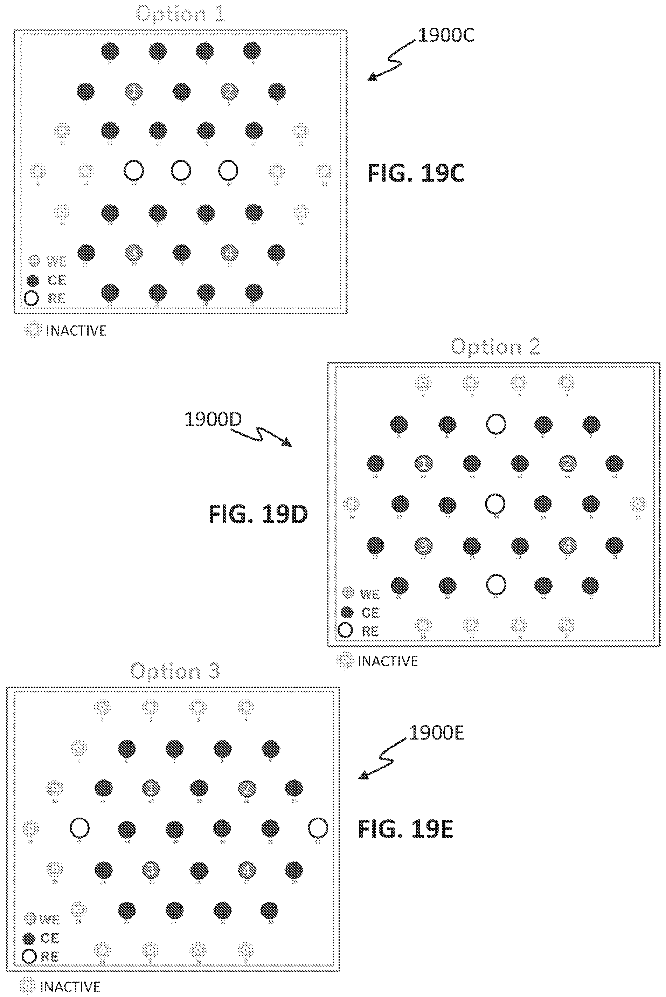

In some variations, only a portion of microneedle array may include active electrodes. For example, FIG. 19C depicts a variation of a microneedle array 1900C with 37 microneedles and a reduced number of active electrodes, including four working electrodes (labeled "1", "2", "3", and "4") in a bilaterally symmetrical arrangement, twenty-two counter electrodes, and three reference electrodes. The remaining eight electrodes in the microneedle array are inactive. In the microneedle array shown in FIG. 19C, each of the working electrodes is surrounded by a group of counter electrodes. Two groups of such clusters of working electrodes and counter electrodes are separated by a row of the three reference electrodes.

As another example, FIG. 19D depicts a variation of a microneedle array 1900D with 37 microneedles and a reduced number of active electrodes, including four working electrodes (labeled "1", "2", "3", and "4") in a bilaterally symmetrical arrangement, twenty counter electrodes, and three reference electrodes, where the remaining ten electrodes in the microneedle array are inactive.

As another example, FIG. 19E depicts a variation of a microneedle array 1900E with 37 microneedles and a reduced number of active electrodes, including four working electrodes (labeled "1", "2", "3", and "4"), eighteen counter electrodes, and two reference electrodes. The remaining thirteen electrodes in the microneedle array are inactive. The inactive electrodes are along a partial perimeter of the overall microneedle array, thereby reducing the effective size and shape of the active microneedle arrangement to a smaller hexagonal array. Within the active microneedle arrangement, the four working electrodes are generally in a radially symmetrical arrangement, and each of the working electrodes is surrounded by a group of counter electrodes.

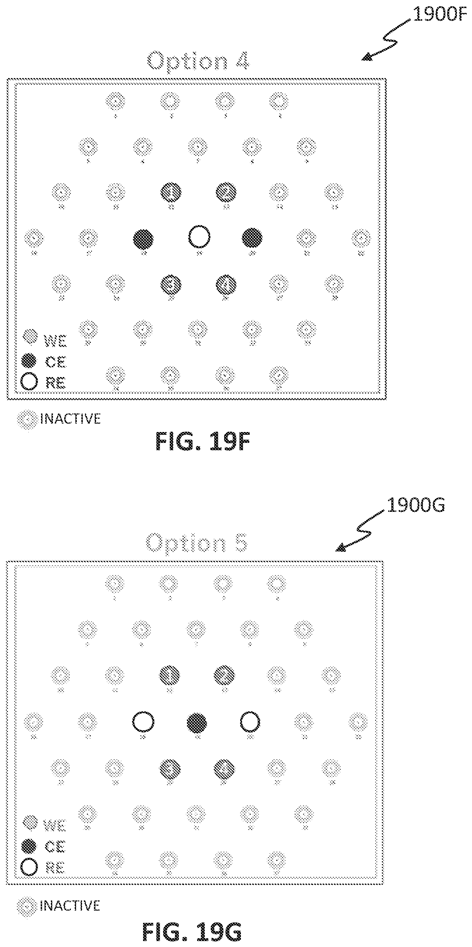

FIG. 19F depicts another example variation of a microneedle array 1900F with 37 microneedles and a reduced number of active electrodes, including four working electrodes (labeled "1", "2", "3", and "4"), two counter electrodes, and one reference electrode. The remaining thirty electrodes in the microneedle array are inactive. The inactive electrodes are arranged in two layers around the perimeter of the overall microneedle array, thereby reducing the effective size and shape of the active microneedle arrangement to a smaller hexagonal array centered around the reference electrode. Within the active microneedle arrangement, the four working electrodes are in a bilaterally symmetrical arrangement and the counter electrodes are equidistant from the central reference electrode.

FIG. 19G depicts another example variation of a microneedle array 1900G with 37 microneedles and a reduced number of active electrodes. The active electrodes in microneedle array 1900G are arranged in a similar manner as that in microneedle array 1900F shown in FIG. 19F, except that the microneedle array 1900G includes one counter electrode and two reference electrodes, and the smaller hexagonal array of active microneedles is centered around the counter electrode. Within the active microneedle arrangement, the four working electrodes are in a bilaterally symmetrical arrangement and the reference electrodes are equidistant from the central counter electrode.

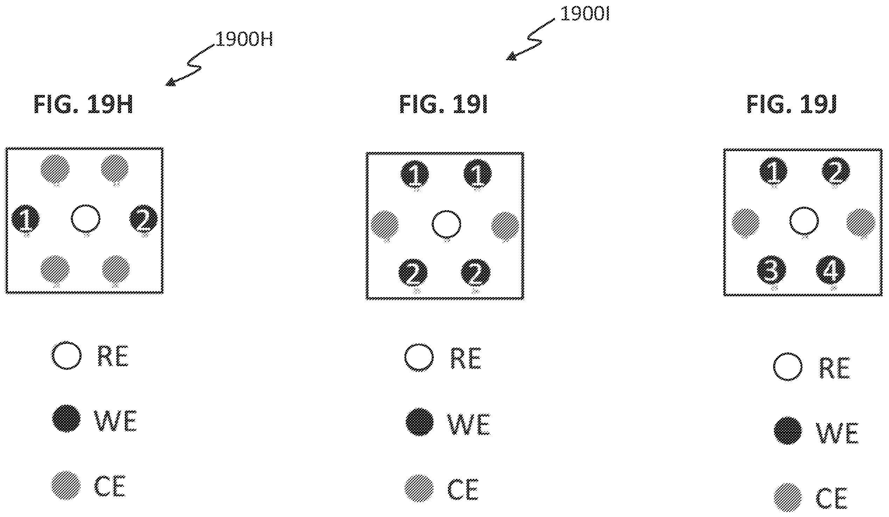

FIG. 19H depicts another example variation of a microneedle array 1900H with 7 microneedles. The microneedle arrangement contains two microneedles assigned as independent working electrodes (1 and 2), a counter electrode contingent comprised of 4 microneedles, and a single reference electrode. There is bilateral symmetry in the arrangement of working and counter electrodes, which are equidistant from the central reference electrode. Additionally, the working electrodes are arranged as far as possible from the center of the microneedle array (e.g., at the periphery of the die or array) to take advantage of a location where the working electrodes are expected to have greater sensitivity and overall performance.

FIG. 19I depicts another example variation of a microneedle array 1900I with 7 microneedles. The microneedle arrangement contains four microneedles assigned as two independent groupings (1 and 2) of two working electrodes each, a counter electrode contingent comprised of 2 microneedles, and a single reference electrode. There is bilateral symmetry in the arrangement of working and counter electrodes, which are equidistant from the central reference electrode. Additionally, the working electrodes are arranged as far as possible from the center of the microneedle array (e.g., at the periphery of the die or array) to take advantage of a location where the working electrodes are expected to have greater sensitivity and overall performance.

FIG. 19J depicts another example variation of a microneedle array 1900J with 7 microneedles. The microneedle arrangement contains four microneedles assigned as independent working electrodes (1, 2, 3, and 4), a counter electrode contingent comprised of 2 microneedles, and a single reference electrode. There is bilateral symmetry in the arrangement of working and counter electrodes, which are equidistant from the central reference electrode. Additionally, the working electrodes are arranged as far as possible from the center of the microneedle array (e.g., at the periphery of the die or array) to take advantage of a location where the working electrodes are expected to have greater sensitivity and overall performance.

While FIGS. 19A-19J illustrate example variations of microneedle array configurations, it should be understood that these figures are not limiting and other microneedle configurations (including different numbers and/or distributions of working electrodes, counter electrodes, and reference electrodes, and different numbers and/or distributions of active electrodes and inactive electrodes, etc.) may be suitable in other variations of microneedle arrays.

*Warm-up:* Many implanted electrochemical sensors require a "warm-up" time, or time for the sensor to attain a stable signal value following implantation. This process has origins in both physiology and sensor dynamics. However, various aspects of cortisol monitoring devices described herein are configured to mitigate factors contributing to warm-up time. For example, the cortisol monitoring devices described herein may have a warm-up time of about 30 minutes or less (e.g., between about 10 minutes and about 30 minutes, between about 15 minutes and about 30 minutes, between about 20 minutes and about 30 minutes, between about 25 minutes and about 30 minutes), about 45 minutes or less, about 60 minutes or less, about 90 minutes or less, or about 120 minutes or less. In some variations, following a warm-up period, the cortisol monitoring device may calibrate during a calibration period.

*Wound response:* For example, the implantation of a sensor creates a wound response due to the localization disruption, displacement, and destruction of tissue. The larger the sensor, or the deeper the implant, the more prolific the wound response. Accordingly, there is a compelling rationale to miniaturize the sensor to elicit an attenuated wound response, which would result in more rapid warm-up.

*Protein adsorption:* Additionally, following implantation of a sensor, the foreign body response is immediately instigated. The foreign body response includes a complex biochemical cascade that aims to encapsulate the foreign material with cellular matter. Hydrophobic surfaces tend to be subject to adsorption of endogenous proteins very rapidly following implant; this is referred to as biofouling. Hydrophilic surfaces, on the other hand, resist biofouling due to high water content. Human serum albumin (HSA) is the predominant protein in the dermal interstitial fluid, constituting about 60% of total protein, and maintains a negative charge at physiological pH. When the sensor is polarized with a positive potential (as in some variation of the cortisol monitoring device), endogenous HSA is subject to electric drift and charge attraction to the positive (working) electrode of the sensor. This can give rise to an increased propensity for the sensor surface to biofoul. This is the rationale behind the implementation of either a hydrophilic diffusion limiting layer or outer biocompatible layer to effectively conceal the sensor from being recognized as a foreign body, as described in further detail above.

As described herein, the cortisol monitoring device reduces the influence of the above physiological factors on warm-up time due to, for example, the shallow nature of the implant, the minimal volume of tissue displaced, the minimal amount of trauma to said tissue during implantation, and the lack of permeation of the vasculature deeper in the reticular dermis, which, when perturbed, can instigate a more prolific wound response that will engender an accelerated effort to encapsulate the implant.

*Attainment of equilibrium:* One example of the effect of sensor dynamics on warm-up time relates to the attainment of equilibrium. An electrochemical sensor requires a finite amount of time to achieve equilibrium when used in a new environment. This is typically associated with the establishment of thermodynamic equilibrium due to an adsorbed surface layer of ions at the electrodes. As the reference electrode in most implantable electrochemical sensors does not employ an internal filling solution with a redox couple that is sealed from the rest of the electrochemical cell, this reference electrode must attain equilibrium with its surroundings in order to establish a stable reference potential.

*Hydration of sensor layers:* The electrode sensor layers must be immersed in an aqueous environment to function properly. The resulting hydration process may activate the electrode's polymer layer(s) and biorecognition element(s) and allows them to rearrange and return to their native active tertiary structure, which is primarily responsible for their activity or unique properties. This process is often known as sensor 'wetting' and allows the medium in which the sensing operation occurs to intercalate the sensor layers to a sufficient extent.

*Decaying of the non-Faradaic response:* The biasing (application of a voltage) of an electrochemical sensor will cause a double layer of ions to form at the electrode surface. This process requires a finite amount of time due to the charging of the adsorbed species on the electrode surface. This gives rise to a double layer capacitance. The non-Faradaic time constant is equal to the product of the said double layer capacitance and the solution resistance. Oftentimes, the non-Faradic response (electrical current) decays to negligible levels more rapidly than other physical phenomena and it is often not the rate-limiting step in the warm-up process. Once the non-Faradaic response decays to negligible levels, the Faradaic response ensues, which is reflective of the electrochemical / redox reaction of interest.

As described herein, the cortisol monitoring device may reduce the influence of sensor dynamics on warm-up time due to, for example, the implementation thin membrane layers (on the order of 10 nm - 5000 nm), which allow the layers to hydrate rapidly. Moreover and owing to the diminutive dimensions of the electrodes described herein (e.g., geometric surface area of the working electrode(s)), the non-Faradaic response transpires for shorter durations (due to reduced double layer capacitance and hence charging of the double layer). In some variations, a high-potential (e.g., > 0.75V) bias for a limited period of time following application of the device to skin may further expedite burn-in or warm-up of the sensor to achieve equilibrium and stable signal levels.

### Electronics system

As shown in the schematic of FIG. 2A of a cortisol monitoring device 110, the electronics system 120 may be integrated within the housing 112, such that the electronics system 120 may be combined with sensing elements (e.g., microneedle array) as part of a single unit. Further details of an example variation of an electronics system 120 are described below.

### PCBs

In some variations, the cortisol monitoring device may include one or more PCBs. For example, the cortisol monitoring device may include at least one PCB in the sensor assembly 320 that includes the microneedle array, and at least one device PCB 350 as shown in FIG. 3E.

For example, as shown in FIGS. 3F-3I, a sensor assembly 320 may include a sensor standoff PCB 322 coupled to a connecting PCB 324. The microneedle array 330 may be attached to the sensor standoff PCB 322 (e.g., FR-4, PTFE, Rogers 4350B), such as through a soldering process combined with an epoxy underfill for mechanical strength. In some variations, an epoxy skirt may be deposited along the edges of the silicon microneedle array 330 to relieve the sharp edges from the silicon dicing processes described above. The epoxy may also provide a transition from the edge of the silicon substrate of the microneedle array silicon to the edge of the PCB 322. Alternatively, this epoxy may be replaced or supplemented by a rubber gasket or the like.

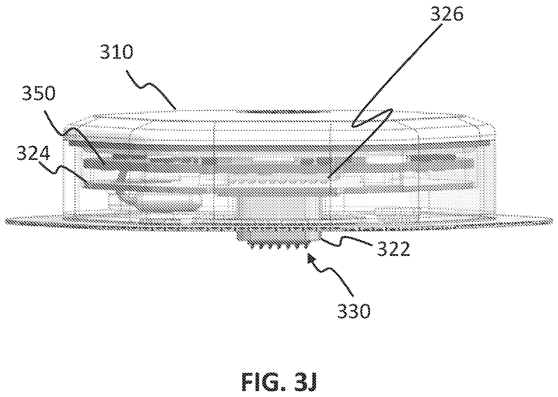

As shown in FIG. 3J, the sensor standoff PCB 322 may function as a standoff that at least in part determines the desired distance to which the microneedle array 330 protrudes from the housing 310. Accordingly, the standoff height of the sensor standoff PCB 322 may be selected to help ensure that the microneedle array 330 is inserted properly into a user's skin. During needle insertion, the bottom surface of the housing 310 will act as a stop for needle insertion. If the sensor standoff PCB 322 has a reduced height and its lower surface is flush or nearly flush with the bottom surface of the housing, then the housing 310 may prevent the microneedle array 330 from being fully inserted into the skin. However, increasing the standoff height may lead to more pressure of the microneedle array on the skin during microneedle insertion, which can lead to dermatological irritation and/or erythema (redness of the skin).

The sensor standoff PCB 322 may be secured to the housing 310 and/or secured within the stack up inside the housing, such as with suitable fasteners or the like. For example, as shown in FIGS. 3H-3J, the sensor standoff PCB 322 (with the microneedle array 330) may be coupled to a first side of the connecting PCB 324, while a second opposite side of the connecting PCB 324 may in turn be coupled to an interposer PCB connector 326. As shown in FIG. 3J, the interposer PCB connector 326 may be communicatively coupled to the device PCB 350, such as for signal processing as described below. Accordingly, signals from the microneedle array 330 may be communicated through the sensor standoff PCB 322 and to the device PCB via the sensor standoff PCB 322, connecting PCB 324, and interposer PCB connector 326. However, in some variations the cortisol monitoring device may include fewer PCBs. For example, in some variations, the sensor assembly 320 may omit the sensor standoff PCB 322, such that the microneedle array 330 may directly communicate electrically to the connecting PCB 324 (or directly to the device PCB 350).

Additionally or alternatively, in some variations at least one of the PCBs in the sensor assembly 320 may include or be coupled to one or more additional sensors in combination with the microneedle array 330. For example, the sensor assembly 320 may include a temperature sensor (e.g., thermistor, resistance temperature detector, thermocouple, bandgap reference, noncontact temperature sensor, etc.). In some variations, temperature measurement may additionally or alternatively be performed by one or more analyte-insensitive electrodes in the microneedle array.

In some variations, the sensor standoff PCB 322 may be between about 0.05 inches and about 0.15 inches, or between about 0.093 inches and about 0.127 inches in thickness. The sensor standoff PCB 322, in some variations, may include one or a plurality of conductive through-substrate vias configured to route electrical signals from an anterior surface of the PCB to a posterior surface of the PCB. In some variations, the sensor standoff PCB 322 may comprise a semiconductor (e.g., silicon) with conductive through-substrate vias configured to route electrical signals from an anterior surface of the semiconductor to a posterior surface of the semiconductor. In yet other variations, the microneedle array 330 may be mounted directly to the PCB 324, without the sensor standoff PCB 322.

### Analog front end

In some variations, the electronics system of the cortisol monitoring device may include an analog front end. The analog front end may include sensor circuitry (e.g., sensor circuitry 124 as shown in FIG. 2A) that converts analog current measurements to digital values that can be processed by the microcontroller. The analog front end may, for example, include a programmable analog front end that is suitable for use with electrochemical sensors. For example, the analog front end may include a MAX30131, MAX30132, or MAX30134 component (which have 1, 2, and 4 channel, respectively), available from Maxim Integrated (San Jose, CA), which are ultra-low power programmable analog front ends for use with electrochemical sensors. The analog front end may also include an AD5940 or AD5941 component, available from Analog Devices (Norwood, MA), which are high precision, impedance and electrochemical front ends. Similarly, the analog front end may also include an LMP91000, available from Texas Instruments (Dallas, TX), which is a configurable analog front end potentiostat for low-power chemical sensing applications. The analog front end may provide biasing and a complete measurement path, including the analog to digital converters (ADCs). Ultra-low power may allow for the continuous biasing of the sensor to maintain accuracy and fast response when measurement is required for an extended duration (e.g. 7 days) using a body-worn, battery-operated device.

In some variations, the analog front end device may be compatible with both two and three terminal electrochemical sensors, such as to enable both DC current measurement, AC current measurement, and electrochemical impedance spectroscopy (EIS) measurement capabilities. Furthermore, the analog front end may include an internal temperature sensor and programmable voltage reference, support external temperature monitoring and an external reference source and integrate voltage monitoring of bias and supply voltages for safety and compliance.

In some variations, the analog front end may include a multi-channel potentiostat to multiplex sensor inputs and handle multiple signal channels.

In some variations, the analog front end and peripheral electronics may be integrated into an application-specific integrated circuit (ASIC), which may help reduce cost, for example. This integrated solution may include the microcontroller described below, in some variations.

### Microcontroller

In some variations, the electronics system of the cortisol monitoring device may include at least one microcontroller (e.g., controller 122 as shown in FIG. 2A). The microcontroller may include, for example, a processor with integrated flash memory. In some variations, the microcontroller in the cortisol monitoring device may be configured to perform analysis to correlate sensor signals to a cortisol measurement. For example, the microcontroller may execute a programmed routine in firmware to interpret the digital signal (e.g., from the analog front end), perform any relevant algorithms and/or other analysis, and route processed data to and/or from the communication module. Keeping the analysis on-board the cortisol monitoring device may, for example, enable the cortisol monitoring device to broadcast cortisol measurement(s) to multiple devices (e.g., mobile computing devices such as a smartphone or smartwatch, therapeutic delivery systems such as insulin pens or pumps, etc.) in parallel, while ensuring that each connected device has the same information.

In some variations, the microcontroller may be configured to activate and/or inactivate the cortisol monitoring device on one or more detected conditions. For example, the device may be configured to power on the cortisol monitoring device upon insertion of the microneedle array into skin. This may, for example, enable a power-saving feature in which the battery is disconnected until the microneedle array is placed in skin, at which time the device may begin broadcasting sensor data. Such a feature may, for example, help improve the shelf life of the cortisol monitoring device and/or simplify the cortisol monitoring device-external device pairing process for the user.

Additionally or alternatively, the microcontroller may be configured to actively confirm the insertion of the microneedle array into skin based on sensor measurements performed with the microneedle array. For example, after two or more microneedles in the microneedle array are presumed to have been inserted into skin, a fixed or time-varying electrical potential or current may be applied to those microneedles. A measurement result (e.g., electrical potential or current value) of a signal generated between the electrodes of the inserted microneedles is measured, and then compared to a known reference value to corroborate successful insertion of the microneedle array into the skin. The reference value may, for example, include a voltage, a current, a resistant, a conductance, a capacitance, an inductance and/or an impedance.

In some variations, the microcontroller may utilize an 8-bit, 16-bit, 32-bit, or 64-bit data structure. Suitable microcontroller architectures include ARM^{®} and RISC^{®} architectures, and flash memory may be embedded or external to the microcontroller for suitable data storage. In some variations the microcontroller may be a single core microcontroller, while in some variations the microcontroller may be a multi-core (e.g., dual core) microcontroller which may enable flexible architectures for optimizing power and/or performance within the system. For example, the cores in the microcontroller may include similar or differing architectures. For example, in an example variation, the microcontroller may be a dual core microcontroller including a first core with a high performance and high power architecture, and a second core with a low performance and low power architecture. The first core may function as a "workhorse" in that it may be used to process higher performance functions (e.g., sensor measurements, algorithmic calculations, etc.), while the second core may be used to perform lower performance functions (e.g., background routines, data transmission, etc.). Accordingly, the different cores of the microcontroller may be run at different duty cycles (e.g., the second core for lower performance functions may be run at a higher duty cycles) optimized for their respective functions, thereby improving overall power efficiency. Additionally or alternatively, in some variations the microcontroller may include embedded analog circuitry, such as for interfacing with additional sensor(s) and/or the microneedle array. In some variations, the microcontroller may be configured to operate using a 0.8V - 5V power source, such as a 1.2V - 3V power source.

### Communication module

In some variations, the electronics system of the cortisol monitoring device may include at least one communication module (e.g., communication module 126 as shown in FIG. 2A), such as a wireless communication module to communicate with one or more devices via wireless signals. For example, the communication module may include a wireless transceiver that is integrated into the microcontroller device and configured to receive and transmit wireless signals. However, the electronics system may additionally or alternatively include a communication module that is separate from the microcontroller device. In some variations, the communication module may communicate via wireless network (e.g., through Bluetooth, NFC, WiFi, RFID, or any type of data transmission that is not connected by cables). For example, devices may directly communicate with each other in pairwise connection (1:1 relationship, unicasting), or in a hub-spoke or broadcasting connection ("one to many" or 1:m relationship, multicasting). As another example, the devices may communicate with each other through mesh networking connections (e.g., "many to many", or m:m relationships, or ad-hoc), such as through Bluetooth mesh networking. Wireless communication may use any of a plurality of communication standards, protocols, and technologies, including but not limited to, Global System for Mobile Communications (GSM), Enhanced Data GSM Environment (EDGE), high-speed downlink packet access (HSDPA), high-speed uplink packet access (HSUPA), Evolution, Data-Only (EV-DO), HSPA, HSPA+, Dual-Cell HSPA (DC-HSPDA), long term evolution (LTE), near field communication (NFC), wideband code division multiple access (W-CDMA), code division multiple access (CDMA), time division multiple access (TDMA), Bluetooth, Wireless Fidelity (WiFi) (e.g., IEEE 802.11a, IEEE 802.11b, IEEE 802.11g, IEEE 802.11n, and the like), or any other suitable communication protocol. Some wireless network deployments may combine networks from multiple cellular networks or use a mix of cellular, Wi-Fi, and satellite communication. In an example variation, the communication module may include a wireless transceiver integrated into the microcontroller and including a Bluetooth Low Energy compatible radio that complies with the Bluetooth Special Interest Group 5.0 specification.

The communication module may further include or be coupled to one or more antennas (e.g., antenna 128 as shown in FIG. 2A). For example, the electronics system may include a chip antenna mounted on the PCB, or an antenna implemented directly onto the PCB, which may provide better range while reducing cost and complexity. In some variations, a user wearing the cortisol monitoring device 110 may function as an antenna (e.g., antenna 128). For example, the antenna input / output 128 of the communication module 126 may be electrically connected to a single microneedle or plurality of microneedles, which are inserted into the wearer's skin (e.g., similar to microneedle array 140 shown in FIG. 2B). This may increase the effective cross-sectional area of the antenna, provide for an adequate impedance match between the antenna input / output of the communication module and free space, and/or help improve operational metrics such as antenna gain, antenna diversity, omni-directionality, and communication module receiver sensitivity / transmitter efficiency.

Devices can come in and out of range from the communication module to connect and reconnect so that the user is able to seamlessly connect and transfer information between devices. In some variations, the microcontroller on each cortisol monitoring device may have a unique serial number, which enables tracking of specific cortisol monitoring devices during production and/or field use.

### Additional sensors

As described above, in some variations, the cortisol monitoring device may include one or more sensors in addition to the microneedle array. For example, the cortisol monitoring device may include one or more temperature sensors configured to measure skin temperature, thereby enabling temperature compensation for the analyte sensor(s). For example, in some variations, a temperature sensor (e.g., thermistor, RTD, semiconductor junction, bimetallic sensor, thermopile sensor) may be coupled to the device PCB within the housing such that the temperature sensor is arranged near a skin-facing portion or bottom portion of the housing 112. The housing may be thinned to reduce thermal resistance and improve heat transfer and hence measurement accuracy. Additionally or alternatively, a thermally conductive material may thermally couple a surface-mount temperature sensor to the user's skin. In variations in which the temperature sensor is coupled to the device PCB near the microneedle array die substrate, the thermally conductive material may, for example, be molded as a skirt to relieve the sharp edges of the die and wrap along the edges of the die and along the surface of the main PCB.

In some variations, the cortisol monitoring device may include at least one microneedle with an electrode configured to function as a cortisol insensitive channel having a known temperature sensitivity, where such a known temperature sensitivity may be used to compensate for temperature. For example, one advantage of using a cortisol insensitive channel includes proximity to the cortisol sensor (e.g., resulting in less error from thermal gradients) and cost (e.g., by reducing external components and specialized processes to thermally couple the sensor to the skin). In some variations, the cortisol monitoring device may include both a cortisol insensitive channel along with a thermistor, with an algorithm that utilizes information from both. Additionally or alternatively, the cortisol monitoring device may include an additional sensor(s) that measures ambient temperature, which may also be useful in the temperature compensation algorithm. In other variations, the cortisol insensitive channel may be used to eliminate or subtract background current or the effect of interferences, which may perturb the cortisol measurement. In yet other variations, the cortisol insensitive channel may be used in tandem with the cortisol-selective channel(s) to obtain a differential measurement that is more reflective of the cortisol concentration and eliminates extraneous source of noise that erode the sensor's signal-to-noise ratio (SNR or S/N).

In some variations, the cortisol insensitive channel may be used to perform differential measurements and/or subtract background noise levels from the cortisol-sensitive channel(s) to improve signal fidelity and/or signal-to-noise ratio. The cortisol insensitive channel may be sensitive to common mode signals that also arise on the cortisol-sensitive channel(s) (e.g., endogenous and pharmacologic interference, pressure attenuations, etc.).

Additionally or alternatively, in some variations, the cortisol monitoring device may include at least one kinetic sensor. The kinetic sensor may, for example, comprise an accelerometer, gyroscope, and/or inertial measurement unit to capture positional, displacement, trajectory, velocity, acceleration, and/or device orientation values. For example, such measurements may be used to infer the wearer's physical activity (e.g., steps, intense exercise) over a finite duration. Additionally or alternatively, in some variations, the kinetic sensor(s) may be employed to enable detection of wearer interactions with the cortisol monitoring device such as touch or tapping. For example, touch or tap detection can be employed to silence or snooze notifications, alerts, and alarms, control a wirelessly connected mobile computing device, or to activate / deactivate a user interface on the cortisol monitoring device (e.g., an embedded display or indicator light). Touching or tapping may be performed in a defined sequence and/or for a predetermined duration (e.g., at least 3 seconds, at least 5 seconds) to elicit certain actions (e.g., display or indicator light deactivation / activation). Additionally or alternatively, in some variations, the cortisol monitoring device may enter into a power saving mode upon detection of limited motion or activity (e.g., absence of significant acceleration) for at least a predetermined period of time (e.g., 15 minutes, 30 minutes, 45 minutes, 1 hour, or other suitable of time), as measured by the kinetic sensor(s).

Additionally, or alternatively, in some variations, the cortisol monitoring device may include at least one real-time clock (RTC). The real-time clock may be employed to track absolute time (e.g., Coordinated Universal Time, UTC, or local time) when the cortisol monitoring device is in storage or during use. In some variations, synchronization to absolute time may be performed following manufacturing of the cortisol monitoring device. The real-time clock may be employed to time-stamp cortisol measurements during operation of the cortisol monitoring device in order to create a time-series data set that is communicated to a connected peripheral device (e.g., mobile computing device), cloud storage, or other suitable data storage device, such as for later review by the user (e.g., wearer of the cortisol monitoring device), their support network, or their healthcare provider, etc.

### Power source(s)

As shown in FIG. 2A, the cortisol monitoring device may include one or more power sources 130 (e.g., battery) in the housing 112 configured to provide power to other components. For example, the cortisol monitoring device may include an AgO battery, which has a high energy density and is more environmentally friendly than lithium batteries. In some variations, a primary (e.g., non-rechargeable) battery may be used. Furthermore, in some variations, a secondary (e.g., rechargeable) battery may be used. However, any suitable power source may be used, including a lithium-based battery.

In some variations, the power source may be coupled to the device PCB using a low profile holder or mount that reduces the overall height of the electronics, thereby minimizing the height or profile of the cortisol monitoring device. For example, whereas traditional battery holders apply force to the topside of the battery using a conductive metal with a spring force, in some variations a lateral mounted battery holder may contact the sides of the battery to complete the electrical circuit.. In some variations, the housing may be sized and/or shaped with suitable tolerances so as to apply vertical or downward force on the battery toward the device PCB, in order to keep the battery in contact with the PCB.

### Applicator

In some variations, the cortisol monitoring device may be applied manually. For example, a user may remove a protective film on the adhesive layer, and manually press the device onto his or her skin on a desired wear site. Additionally or alternatively, as illustrated in FIG. 1, in some variations the cortisol monitoring device may be applied to the skin using a suitable applicator 160. The applicator 160 may, for example, be configured to urge the cortisol monitoring device 110 toward the skin of the user such that the microneedle array 140 of the cortisol monitoring device 110 may be inserted into the skin (e.g., to the desired target depth).

### Kits

In some variations, some or all components of the cortisol monitoring system may be provided in a kit (e.g., to a user, to a clinician, etc.). For example, a kit may include at least one cortisol monitoring device 110 and/or at least one applicator 160. In some variations, a kit may include multiple cortisol monitoring devices 110, which may form a supply of cortisol monitoring devices sufficient that is for a predetermined period of time (e.g., a week, two weeks, three weeks, a month, two months, three months, six months, a year, etc.). The kit may include any suitable ratio of applicators to cortisol monitoring devices (e.g., 1:1, lower than 1:1, greater than 1:1). For example, the kit may include the same number of applicators as cortisol monitoring devices, such as if each applicator is single-use and is configured to be disposed after its use in applying a respective cortisol monitoring device to the user. As another example, the kit may include a number of applicators that is lower than the number of cortisol monitoring devices in the kit (e.g., one applicator per two or three cortisol monitoring devices), such as if an applicator is intended to be reused for applying multiple cortisol monitoring devices or if multiple cortisol monitoring devices are loaded into a single applicator for repeated applications. As another example, the kit may include a number of applicators that is higher than the number of cortisol monitoring devices in the kit (e.g., two applicators per cortisol monitoring device), such as to provide extra or redundant applicators in case of applicator loss or breakage, etc.

In some variations, the kit may further include user instructions for operating the cortisol monitoring device and/or applicator (e.g., instructions for applying the cortisol monitoring device manually or with the applicator, instructions for pairing the cortisol monitoring device with one or more peripheral devices (e.g., computing devices such as a mobile phone), etc.).

### Use of cortisol monitoring system

Described below is an overview of various aspects of a method of use and operation of the cortisol monitoring system, including the cortisol monitoring device and peripheral devices, etc.

### Application of cortisol monitoring device

As described above, the cortisol monitoring device is applied to the skin of a user such that the microneedle array in the device penetrates the skin and the microneedle array's electrodes are positioned in the upper dermis for access to dermal interstitial fluid. For example, in some variations, the microneedle array may be geometrically configured to penetrate the outer layer of the skin, the stratum corneum, bore through the epidermis, and come to rest within the papillary or upper reticular dermis. The sensing region, confined to the electrode at the distal extent of each microneedle constituent of the array (as described above) may be configured to rest and remain seated in the papillary or upper reticular dermis following application in order to ensure adequate exposure to circulating dermal interstitial fluid (ISF) without the risk of bleeding or undue influence with nerve endings.

In some variations, the cortisol monitoring device may include a wearable housing or patch with an adhesive layer configured to adhere to the skin and fix the microneedle array in position. While the cortisol monitoring device may be applied manually (e.g., removing a protective film on the adhesive layer, and manually pressing the patch onto the skin on a desired wear site), in some variations the cortisol monitoring device may be applied to the skin using a suitable applicator.

The cortisol monitoring device may be applied in any suitable location, though in some variations it may be desirable to avoid anatomical areas of thick or calloused skin (e.g., palmar and plantar regions), or areas undergoing significant flexion (e.g., olecranon or patella). Suitable wear sites may include, for example, on the arm (e.g., upper arm, lower arm), shoulder (e.g., over the deltoid), back of hands, neck, face, scalp, torso (e.g., on the back such as in the thoracic region, lumbar region, sacral region, etc. or on the chest or abdomen), buttocks, legs (e.g., upper legs, lower legs, etc.), and/or top of feet, etc.

As described above, in some variations the cortisol monitoring device may be configured to automatically activate upon insertion, and/or confirm correct insertion into skin. Details of these features are described in further detail above.

### Pairing to peripheral device

In some variations, the cortisol monitoring device may be paired to at least one peripheral device such that the peripheral device receives broadcasted or otherwise transmitted data from the cortisol monitoring device, including measurement data. Suitable peripheral devices include, for example a mobile computing device (e.g., smartphone, smartwatch) which may be executing a mobile application. Additionally alternatively, a cortisol monitoring device may be paired (or otherwise combined) with a therapeutic delivery device.

As described above, the pairing may be accomplished through suitable wireless communication modules (e.g., implementing Bluetooth). In some variations, the pairing may occur after the cortisol monitoring device is applied and inserted into the skin of a user (e.g., after the cortisol monitoring device is activated). Additionally or alternatively, the pairing may occur prior to the cortisol monitoring device being applied and inserted into the skin of a user.

Thus, the paired mobile or other device may receive the broadcasted or transmitted data from the cortisol monitoring device. The peripheral device may display, store, and/or transmit the measurement data to the user and/or healthcare provider and/or support network. Furthermore, in some variations, the said paired mobile or wearable device performs algorithmic treatment to the data to improve the signal fidelity, accuracy, and/or calibration, etc. In some variations, measurement data and/or other user info may additionally or alternatively be communicated and/or stored via network (e.g., cloud network).

By way of illustration, in some variations a mobile computing device or other computing device (e.g., smartphones, smartwatches, tablets, etc.) may be configured to execute a mobile application that provides an interface to display estimated cortisol-based values, trend information and historical data, etc.

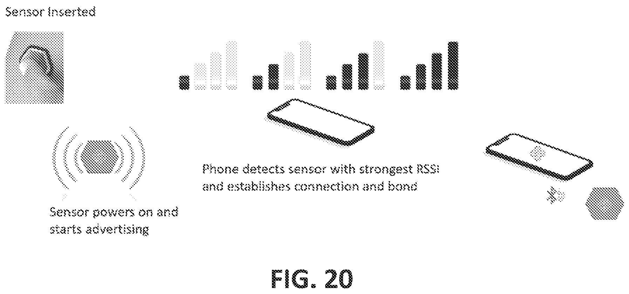

In some variations, the mobile application may use the mobile computing device's Bluetooth framework to scan for the cortisol monitoring device. As shown in FIG. 20, the cortisol monitoring device may power on or initialize as soon as it is applied to the skin, and the cortisol monitoring device may begin the advertising process. The mobile application may then connect to the cortisol monitoring device and begin priming the sensor for measurement. In case the mobile application detects multiple cortisol monitoring devices, the mobile application may detect the cortisol monitoring device that is closest in proximity to itself and/or may request the user (e.g., via the user interface on the mobile device) to confirm disambiguation. In some variations, the mobile application may also be capable of connecting to multiple cortisol monitoring devices simultaneously. This may be useful, for example, to replace sensors that are reaching the end of their lifetime.

In some variations, the Bluetooth^{®} Low Energy^{™} (BLE) protocol may be used for connectivity. For example, the sensor implements a custom BLE peripheral profile for the cortisol monitoring system. Data may be exchanged after establishing a standard secure BLE connection between the cortisol monitoring device and the smartphone, smartwatch, or tablet running the mobile application. The BLE connection may be maintained permanently for the life of the sensor. If the connection is broken due to any reasons (e.g., weak signal) the cortisol monitoring device may start advertising itself again, and the mobile application may re-establish the connection at the earliest opportunity, for example, when in range based on physical proximity.

In some variations, there may be one or more additional layers of security implemented on top of the BLE connection to ensure authorized access consisting of a combination of one or more techniques such as passcode-protection, shared-secrets, encryption and multi-factor authentication.

The mobile application may guide the user through initiating a new cortisol monitoring device. Once this process completes, the mobile application is not required for the cortisol monitoring device to operate and record measurements. A secondary display device like a smartwatch can be authorized from the mobile application to receive cortisol readings from the sensor directly.

Furthermore, in some variations the mobile application may additionally or alternatively help calibrate the cortisol monitoring device. For example, the cortisol monitoring device may indicate a request for calibration to the mobile application, and the mobile application may request calibration input from the user to calibrate the sensor.

### Sensor measurements

Once the cortisol monitoring device is inserted and warm-up and any calibration has completed, the cortisol monitoring device may be ready for providing sensor measurements of cortisol. The cortisol from the biological milieu, through the biocompatible and diffusion-limiting layers on the working electrode, and to the biorecognition layer including the biorecognition element.

A bias potential may be applied between the working and reference electrodes of the cortisol monitoring device, and an electrical current may flow from the counter electrode to maintain the fixed potential relationship between the working and reference electrodes. This causes the oxidation or reduction of the electroactive product, causing a current to flow between the working electrodes and counter electrodes. The current value is proportional to the proximity of the redox reporter molecule functionalized to the cortisol-binding aptamers to electrode material of the working electrode and, specifically, to the concentration of cortisol in the dermal interstitial fluid according to the Cottrell relation, or some derivative thereof, as described in further detail above.

The electrical current may be converted to a voltage signal by a transimpedance amplifier and quantized to a digital bitstream by means of an analog-to-digital converter (ADC). Alternatively, the electrical current may be directly quantized to a digital bitstream by means of a current-mode ADC. The digital representation of the electrical current may be processed in the embedded microcontroller(s) in the cortisol monitoring device and relayed to the wireless communication module for broadcast or transmission (e.g., to one or more peripheral devices). In some variations, the microcontroller may perform additional algorithmic treatment to the data to improve the signal fidelity, accuracy, and/or calibration, etc.

In some variations, the digital representation of the electrical current, or sensor signal, may be correlated to cortisol measurement by the cortisol monitoring device. For example, the microcontroller may execute a programmed routine in firmware to interpret the digital signal and perform any relevant algorithms and/or other analysis. The interpretation of the digital signal may include conversion of the digital signal into user status based on cortisol measurements that is relevant to a user or a care provider. Examples of user status include: cortisol concentration in a bodily fluid, by way of example dermal interstitial fluid or blood; a % change in cortisol concentration; whether or not the cortisol concentration is above, within, or below a threshold; and a psychological state of the user, by way of example a degree of stress and/or whether the stress is acute or chronic or to track diurnal variation in cortisol levels. Keeping the analysis on-board the cortisol monitoring device may, for example, enable the cortisol monitoring device to broadcast cortisol measurement(s) to multiple devices in parallel, while ensuring that each connected device has the same information. Thus, generally, the user's cortisol-based values may be estimated and stored in the cortisol monitoring device and communicated to one or more peripheral devices.

Data exchange can be initiated by either the mobile application or by the cortisol monitoring device. For example, the cortisol monitoring device may notify the mobile application of new cortisol data as it becomes available. The frequency of updates may vary, for example, between about 5 seconds and about 5 minutes, and may depend on the type of data. Additionally or alternatively, the mobile application may request data from the cortisol monitoring device (e.g., if the mobile application identifies gaps in the data it has collected, such as due to disconnections).

If the mobile application is not connected to the cortisol monitoring device, the mobile application may not receive data from the sensor electronics. However, the electronics in the cortisol monitoring device may store each actual and/or estimated cortisol data point. When the mobile application is reconnected to the cortisol monitoring device, it may request data that it has missed during the period of disconnection and the electronics on the cortisol monitoring device may transmit that set of data as well (e.g., backfill).

Generally, the mobile application may be configured to provide display of real-time or near real-time cortisol measurement data, such as on the display of the mobile computing device executing the mobile application. In some variations, the mobile application may communicate through a user interface regarding analysis of the cortisol measurement, such as alerts, alarms, insights on trends, etc. such as to notify the user of cortisol measurements requiring attention or follow-up action (e.g., high cortisol measurements, low cortisol measurements, high rates of change, cortisol measurements outside of a pre-set range, etc.). In some variations, the mobile application may additionally or alternatively facilitate communication of the measurement data to the cloud for storage and/or archive for later retrieval.

### Interpreting cortisol monitoring device user interface

In some variations, information relating to cortisol measurement data and/or the cortisol monitoring device may be communicated via a user interface of the cortisol monitoring device. In some variations, the user interface of the cortisol monitoring device may be used to communicate information to a user in addition to, or as an alternative to, communicating such information via a peripheral device such as through a mobile application on a computing device. Accordingly, a user and/or those around the user may easily and intuitively view the cortisol monitoring device itself for an assessment of cortisol measurement data (e.g., cortisol measurement status such as current and/or trending cortisol measurement levels) and/or device status, without the need to view a separate device (e.g., peripheral device or other device remote from, and in communication with, the cortisol monitoring device). Availability of such information directly on the cortisol monitoring device itself may also enable a user and/or those around the user to more promptly be alerted of any concerns (e.g., cortisol measurements that are above or below target range, and/or cortisol measurements that are increasing or decreasing at an alarming rate), thereby enabling a user to take appropriate corrective action more quickly.

For example, FIGS. 26A-26C depict an example variation of a cortisol measurement device 3200 including a user interface 3220 with multiple indicator lights, including indicator lights 3224a, 3224b, 3224c, which may be selectively illuminated to communicate a user status (e.g., information relating to cortisol measurement in the user). The user interface 3220 may be similar, for example, to user interface 3120 described above with respect to FIG. 25A and/or FIG. 25B. Although the user interface 3220 includes three indicator lights 3224a, 3224b, 3224c, it should be understood that in some variations, the user interface 3220 may include any suitable number of lights, including fewer than three (e.g., one, two) or more than three (e.g., four, five, six, or more).

The indicator lights 3224a, 3224b, 3224c may be arranged in a sequential manner such that their relative positions help a user to intuitively understand information communicated collectively by the user interface. For example, the three indicator lights 3224a, 3224b, 3224c may be illuminated to generally indicate three progressive levels (or ranges) of cortisol measurements: the lowest indicator light 3224a may be illuminated to generally indicate a cortisol measurement that is lowest of the three levels, the middle indicator light 3224b may be illuminated to generally indicate a cortisol measurement that is in the middle of the three levels, and the highest indicator light 3224c may be illuminated to generally indicate a cortisol measurement that is highest of the three levels. In one example variation, the lowest indicator light 3224a may be illuminated to indicate a cortisol measurement that is in a target range (FIG. 26A), the middle indicator light 3224b may be illuminated to indicate a cortisol measurement that is above a target range (FIG. 26B), and the highest indicator light 3224c may be illuminated to indicate a cortisol measurement that is significantly above a target range (FIG. 26C). In another example variation, the lowest indicator light 3224a may be illuminated to indicate a cortisol measurement that is below a target range, the middle indicator light 3224b may be illuminated to indicate a cortisol measurement that is within the target range, and the highest indicator light 3224c may be illuminated to indicate a cortisol measurement that is above a target range.

The threshold values for a target range may be any suitable values. For example, in some variations in which cortisol monitoring is being performed, cortisol measurements may be considered within a target range if they are between about 100 nM and 500 nM and may be considered below a target range if they are below about 100 nM. The different thresholds for "above" a target range and "significantly" above a target range may have any suitable value. For example, in some variations, cortisol measurements may be considered "above" a target range if it is above a first predetermined threshold (e.g., above a threshold value of about 500 nM and cortisol measurement may be considered "significantly above" a target range if it is a predetermined amount (e.g., percentage) above the first predetermined threshold, such as at least 33% above the first predetermined threshold (e.g., 665 nM), or at least about 25% above the first predetermined threshold, at least about 30% above the first predetermined threshold, at least 35% above the first predetermined threshold, or at least 40% above the first predetermined threshold, or other suitable second predetermined threshold.

Furthermore, the thresholds for considering cortisol measurements within target range, or below target range, or "above" target range or "significantly above" target range (or other characterization of the cortisol measurements) may be static or dynamic, and/or may vary based on user information such as historical measurements and/or trends or other historical data (e.g., relative to an average or expected cortisol measurement for the user at particular times or average or expected rate of change). Furthermore, it should be understood that while the user interface 3220 includes three sequential indicator lights, in other variations a user interface on the housing of a cortisol monitoring device may include fewer (e.g., two) or more (e.g., four, five, six, or more) that may be similarly illuminated individually to indicate a cortisol measurement (e.g., each corresponding to a general relative level of cortisol measurement).

In some variations, different illumination colors and/or timing for one or more of the indicator lights 3224a, 3224b, 3224c may additionally or alternatively enable a user to easily distinguish between each cortisol measurement level. For example, when a cortisol measurement is within a target range, the appropriate indicator light(s) may be illuminated in a first color (e.g., blue), while when the cortisol measurement is outside the target range, the appropriate indicator light(s) may be illuminated in another color (e.g., white for below target range, orange for above target range). As another example, when the cortisol measurement is within a target range, the appropriate indicator light(s) may be illuminated in a first temporal pattern (e.g., long, gentle pulse of illumination "on" time), while when the cortisol measurement is outside the target range, the appropriate indicator light(s) may be illuminated in another temporal pattern (e.g., short, flash-like pulse of illumination "on" time). Shorter pulses of illumination "on" time may, for example, be helpful to better attract user attention and/or more intuitively communicate an alert when the cortisol measurement is below a target range, above a target range, or significantly above a target range. Higher frequency illumination may, in some variations, correlate to greater alert level (e.g., significantly below the target range or significantly above the target range).

FIGS. 27A-27D and Table 1 illustrate different illuminating modes used in an example method of operating the user interface 3220 of a cortisol monitoring device. The exact parameter values of these illumination modes are non-limiting and are included for an example variation for illustrative purposes only. For example, in the "below target range" illumination mode, the illumination color may be any suitable color, and/or the illumination "on" time may be between about 0.1 seconds and 1 second, between about 0.2 seconds and 0.5 seconds, or about 0.3 seconds, and/or the illumination "off" time may be between about 0.5 seconds and about 5 seconds, or between about 1 second and about 4 seconds, or between about 2 seconds and about 4 seconds, or about 3 seconds; and/or the ratio between the illumination "on" and illumination "off" times may be about 0.1, about 0.2, about 0.3, about 0.4, about 0.5, and/or other suitable illumination parameters. As another example, in the "in target range" illumination mode and/or the "above target range illumination mode, the illumination color may be any suitable color, and/or the illumination "on" time may be between about .1 seconds and about 3 seconds, between about .5 seconds and about 2 seconds, or about 1 second, and/or the illumination "off" mode may be between about 0.5 seconds and about 5 seconds, or between about 1 second and about 4 seconds, or between about 2 seconds and about 4 seconds, or about 3 seconds, and/or the ratio between the illumination "on" and illumination "off" times may be about 0.1, about 0.2, about 0.3, about 0.4, about 0.5, and/or other suitable illumination parameters. As another example, in the "significantly above target range", the illumination color may be any suitable color, and/or the illumination "on" time may be between about 0.2 seconds and 2 seconds, between about 0.5 seconds and about 1.5 seconds, or about 0.8 seconds, and/or the illumination "off" time may be between about 0.5 seconds and about 5 seconds, or between about 1 second and about 4 seconds, or between about 2 seconds and about 4 seconds, or about 3 seconds and/or other suitable illumination parameters. Furthermore, fewer or more illumination modes for indicating cortisol measurement level may be possible in other variations.

**Table 1. Example illumination modes for indicating cortisol measurement**

| Cortisol measurement level | Figure | Indicator light illuminated | Illumination color | Illumination "on" time t(on) | Illumination "off" time t(off) |
|---|---|---|---|---|---|
| Below target range | FIG. 27A | Lowest | White | 0.3 sec | 3 sec |
| In target range | FIG. 27B | Lowest | Blue | 1 sec | 3 sec |
| Above target range | FIG. 27C | Middle | Orange | 1 sec | 3 sec |
| Significantly above target range | FIG. 27D | Highest | Orange | 0.8 sec | 3 sec |

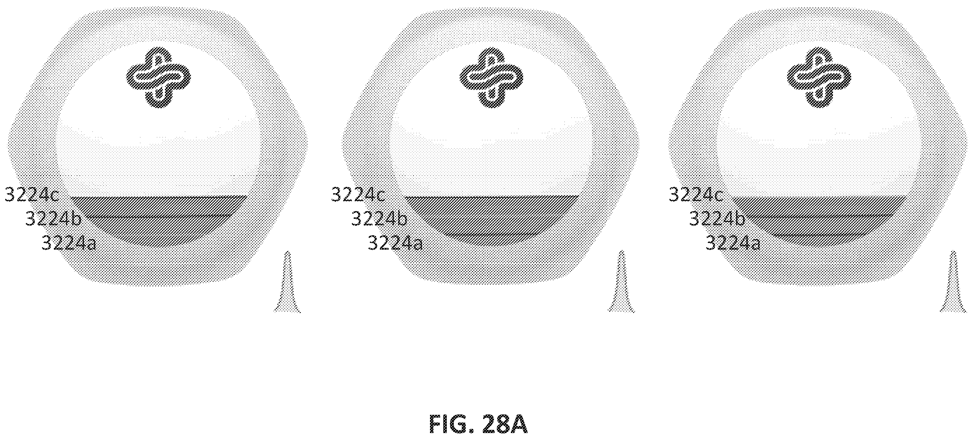

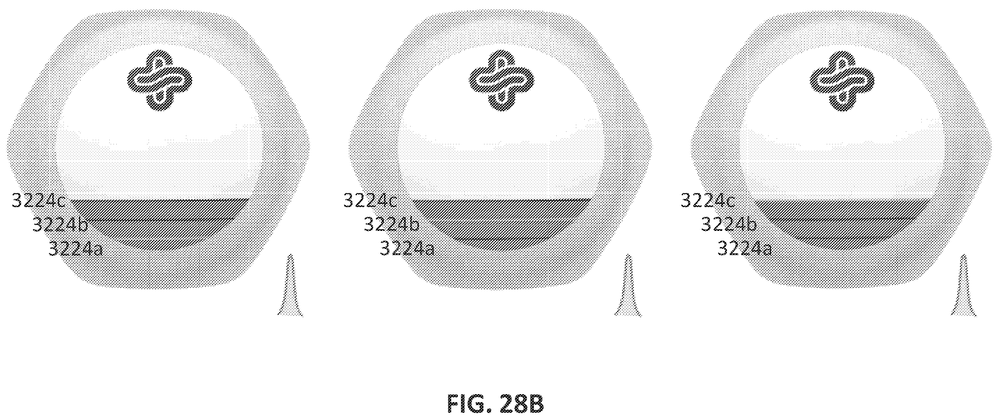

Additionally or alternatively, in some variations, the indicator lights 3224a, 3224b, 3224c may be illuminated in a progressive sequence to indicate trend information of cortisol measurements over time. For example, as shown in FIG. 28A, a progressive sequence of illumination of the indicator lights 3224a, 3224b, 3224c in a first direction from lower indicator light(s) to higher indicator light(s) (e.g., indicator light 3224a followed by indicator light 3244b, followed by indicator light 3224c) may intuitively indicate a trend of increasing cortisol measurement. In some variations, the progressive sequence of illumination could have any suitable illumination color. In some variations, such rising sequential illumination of indicator lights may be in a suitable color to indicate either that the current cortisol measurement is within a target range and rising, or that the current cortisol measurement is above a target range and rising. For example, FIG. 28A illustrates rising progressive illumination in a first color (e.g., blue) to indicate that current cortisol measurement is within the target range and rising, whereas FIG. 28B illustrates rising progressive illumination in a second color(e.g., orange) to indicate that the current cortisol measurement is above (or significantly above) the target range and rising. As yet another example, a rising progressive illumination in a third color (e.g., white) may indicate that the current cortisol measurement is below (or significantly below) the target range and rising.

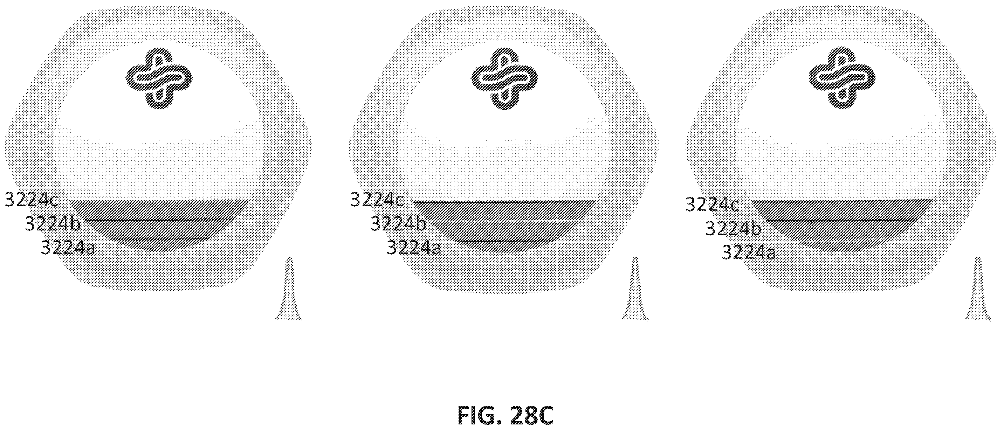

As another example, as shown in FIG. 28C, a progressive sequence of illumination of the indicator lights 3224a, 3224b, 3224c in a second direction (e.g., opposite direction of the first direction) from higher indicator light(s) to lower indicator light(s) (e.g., indicator light 3224c followed by indicator light 3244b, followed by indicator light 3224a) may intuitively indicate a trend of decreasing cortisol measurements. Similar to that described above with respect to FIGS. 28A and 28B, such a falling progressive sequence of illumination of indicator lights may be a suitable color to indicate the status of the current cortisol measurement that is falling (e.g., falling progressive illumination in a first color (e.g., blue) to indicate that current cortisol measurement is within the target range and falling, falling progressive illumination in a second color (e.g., orange) to indicate the current cortisol measurement is above (or significantly above) the target range and falling, or falling progressive illumination in a third color (e.g., white) may indicate that the current cortisol measurement is below (or significantly below) the target range and falling).

It should be understood that other variations of progressive sequences of illumination may be used to similarly indicate cortisol measurement trends. For example, a 1-dimensional array of indicator lights (e.g., arranged in a row, a column, an arc, etc.) may be illuminated in a progressive sequence from a first end of the array to a second end of the array to indicate a rising cortisol measurement trend, and illuminated in a progressive sequence from a second end of the array to a first end of the array to indicate a falling cortisol measurement trend. For example, progressive sequences of illumination may be characterized as left-to-right, right-to-left, top-to-bottom, bottom-to-top, clockwise, counter-clockwise, etc. Furthermore, it should be understood that while the user interface 3220 includes three sequential indicator lights, in other variations a user interface on the housing of a cortisol monitoring device may include fewer (e.g., two) or more (e.g., four, five, six, or more) that may be similarly illuminated in a progressive sequence to indicate rising and/or falling cortisol measurement trends.

In some variations, within each rising or falling sequence of illumination across the indicator lights, the illumination of adjacent indicator lights may be interspersed by an illumination "off" period. Furthermore, in some variations, the pace at which the illumination transitions between indicator lights may indicate rate of change of cortisol measurement. For example, the faster the illumination transitions from lower to higher indicator lights, the faster the rate of change (and potentially the greater urgency or need for user attention to the trend). Additionally or alternatively, each rising or falling sequence of illumination across the indicator lights may be separated by a sequence end illumination "off" time in order to help distinguish between a rising sequence and a falling sequence. The sequence end illumination "off" time may be longer than the illumination "off" period within each sequence. In some variations, the start or end of each rising or falling sequence of illumination may additionally or alternatively be demarcated in any suitable manner (e.g., illuminating all lights simultaneously at the start or end of a rising or falling sequence).

Table 2 illustrates different illumination modes used in an example method of operating the user interface 3220 of a cortisol monitoring device to indicate cortisol measurement trends. The exact parameter values of these illumination modes are non-limiting and are included for an example variation for illustrative purposes only. For example, in a progressive sequence of illumination (e.g., for any one of more suitable illumination modes), the illumination color may be any suitable color, and/or the illumination "on" time may be between about 0.1 seconds and 1 second, between about 0.2 seconds and 0.5 seconds, or about 0.3 seconds, and/or the illumination "off" time between illumination of adjacent indicator lights may be between about 0.05 seconds and about 1 second, between about 0.1 seconds and about 0.5 seconds, or about 0.18 seconds, and/or the ratio between the illumination "on" time and illumination "off" time may be about 1, about 1.5, or about 2, and/or the sequence end may be designated by illumination "off" for between about 2 seconds and about 5 seconds, or about 3 seconds. Furthermore, fewer or more illumination modes for indicating cortisol measurement trends may be possible in other variations.

**Table 2. Example illumination modes for indicating cortisol measurement trends**

| Cortisol measurement trend | Figure | Indicator lights illumination sequence | Illumination color | Illumination "on" time | **Illumination** "off" time | Sequence end |
|---|---|---|---|---|---|---|
| In target range, rising | FIG. 28A | Lower → Higher | Blue | 0.3 sec | 0.18 sec | 3 sec **illumination** "off" |
| Above target range, rising | FIG. 28B | Lower → Higher | Orange | 0.3 sec | 0.18 sec | 3 sec **illumination** "off" |
| Above (and/or significan tly above) target range, dropping | FIG. 28C | Higher → Lower | Orange | 0.3 sec | 0.18 sec | 3 sec illumination "off" |

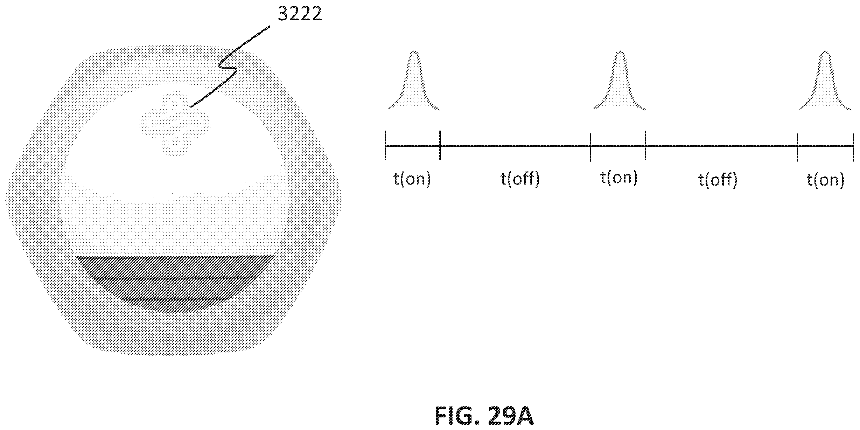

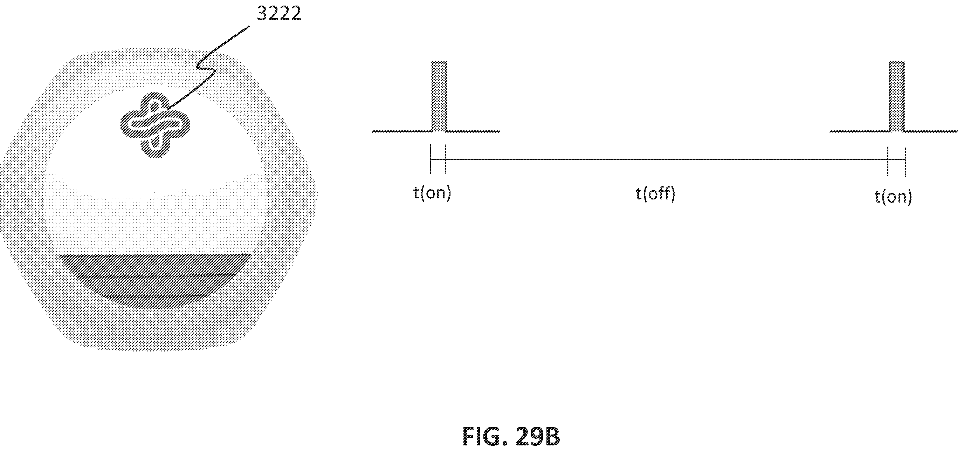

Additionally or alternatively, an indicator light 3222 may be selectively illuminated to communicate a device status. Similar to that described above, color and/or timing of illumination may be varied in a predetermined manner to indicate different device statuses. Status may, for example, include a warm-up period notification, an end-of-life notification, a sensor fault state notification, a sensor failure mode (e.g., improper insertion) notification, a low battery notification, and/or a device error notification. Furthermore, any suitable number of indicators lights may be illuminated individually and/or collectively (e.g., in sequence or simultaneously) to indicate different device statuses. For example, as shown in FIG. 29A, a user interface including an indicator light 3222 may be illuminated in a first illumination mode (e.g., first illumination color such as white and/or first temporal illumination pattern) to indicate a device "wait" mode. The wait mode may, for example, correspond to a device warmup period (as described elsewhere herein), detection of a temporary error (e.g., detection of pressure-induced sensor attenuation). As another example, as shown in FIG. 29B, a user interface including an indicator light 3222 may be illuminated in a second illumination mode (e.g., second illumination color such as red and/or second temporal illumination pattern) to indicate a device "end of life" mode (e.g., determination of an end of a predetermined wear period such as that described below, detection of a permanent error, etc.).

Table 3 illustrates different illumination modes used in an example method of operating the user interface of a cortisol monitoring device to indicate device status. The exact parameter values of these illumination modes are non-limiting and are included for an example variation for illustrative purposes only. For example, in the "wait" illumination mode, the illumination color may be any suitable color, and/or the illumination "on" time may be between about .1 seconds and about 3 seconds, between about .5 seconds and about 2 seconds, or about 1 second, and/or the illumination "off" mode may be between about 0.5 seconds and about 5 seconds, or between about 1 second and about 4 seconds, or between about 2 seconds and about 4 seconds, or about 3 seconds, and/or the ratio between the illumination "on" and illumination "off" times may be about 0.1, about 0.2, about 0.3, about 0.4, about 0.5, and/or other suitable illumination parameters. As another example, in the "end of life" illumination mode, the illumination color may be any suitable color, and/or the illumination "on" time may be between about 0.01 seconds and about 1 second, between about 0.01 seconds and about 0.5 seconds, between about 0.01 seconds and about 0.3 seconds, between about 0.01 seconds and about 0.1 seconds, or about 0.04 seconds, and/or the illumination "off" time may be between about 1 second and about 10 seconds, between about 3 seconds and about 8 seconds, or about 6 seconds, and/or the ratio between the illumination "on" and illumination "off" times may be about 0.3, about 0.2, about 0.1, about 0.05, about 0.01, or less than about 0.01, and/or other suitable illumination parameters. Although only two illumination modes are shown, in some variations a cortisol monitoring device may have fewer or more illumination modes, such as for each of the above statuses (e.g., first illumination mode for a device warmup period, a second illumination mode for detection of a temporary error, a third illumination mode for determination of an end of device lifetime, a fourth illumination mode for detection of a permanent error, etc.).

**Table 3. Example illumination modes for indicating device status**

| Device status | Figure | Illumination color | Illumination "on" time t(on) | Illumination "off" time t(off) |
|---|---|---|---|---|
| Wait | FIG. 29A | White | 1 sec | 3 sec |
| End of life | FIG. 29B | Red | 0.04 sec | 6 sec |

In some variations, a photodiode, phototransistor, photodetector, or other suitable ambient light sensor may be employed to measure the illumination level in the device's immediate environment. The ambient light measurement may, for example, be used to trigger an adjustment (e.g., dimming) of the brightness of the user interface (e.g., display, indicator light(s), etc.) to conserve battery charge in a power saving mode, to improve contrast under various illumination scenarios, and/or to reduce device visibility to other individuals. For example, the cortisol monitoring device may enter the power saving mode in response to measurements from the ambient light sensor indicating general absence of ambient light (e.g., sufficient darkness for at least a predetermined period of time) such as when the device is placed under the clothing of a wearer or when the wearer is asleep in a dark environment. In these scenarios, the power saving mode may be practical because the indicator lights may have limited utility when concealed and out of view of the wearer (e.g., under clothing) or otherwise may be perceived as an annoyance (e.g., during slumber), etc. In response to measurements from the ambient light sensor indicating exposure to ambient light (e.g., sufficient brightness for at least a predetermined period of time), the cortisol monitoring device may then exit the power saving mode and increase the brightness of the user interface accordingly.

### Additional system functions

In some variations, the mobile application may help a user manage the lifetimes and replacement of cortisol monitoring devices. For example, the mobile application may terminate data display when the wear period of the cortisol monitoring device has elapsed. In some variations, the cortisol monitoring device may have enhanced longevity compared to conventional continuous monitoring devices. For example, the cortisol monitoring devices described herein may have a wear period (e.g., intended lifetime) of at least 3 days, at least 5 days, at least 6 days, at least 7 days, at least 10 days, or at least 12 days, between 5 days and between 10 days, between 10 days and 14 days, etc. without material loss in performance.

Additionally or alternatively, mobile application may provide configurable alerts to the user that the wear period is about to elapse, which permits users to apply a new cortisol monitoring device when the current cortisol monitoring device is still active but close to expiry. Additionally, the new cortisol monitoring device can warm up (typically between about 30 minutes and about 2 hours) while the old unit is still delivering cortisol measurements. The old cortisol monitoring device can then be removed upon expiry. The new cortisol monitoring device may then become the primary sensor delivering cortisol measurements to the mobile application. This may provide for an uninterrupted coverage for cortisol measurements. Additionally, the readings from the old cortisol monitoring device may be used to calibrate or algorithmically improve the accuracy of the new cortisol monitoring device.

In some variations, a cortisol monitoring device may have a unique serial number contained within the microcontroller (e.g., located in the electronics system). This serial number may enable sensors to be tracked from manufacturing and throughout the use of the product. For example, sensor device history records including manufacturing and customer use may be transmitted and stored in the cloud database. This enables tracking and inferences to be made on various parameters such as sensor performance metrics and improvement for individual users as well as sensor lots, tracking defective sensor lots back from field data to manufacturing or supplier issues very rapidly, personalized health monitoring features for individual users, etc.

In some variations, the system may be able to track inventory of cortisol monitoring devices from warehousing to purchasing transactions to product use, which may enable the system to assist users in fulfillment of timely orders (e.g., to ensure that users don't run out of cortisol monitoring devices). Additionally or alternatively, fulfillment can be executed automatically as monitoring device utilization is tracked, and timely delivery can be made to the user's residence to help ensure that sensor supply never depletes (e.g. 'just-in-time' delivery). This can interface with virtual or e-pharmacies, logistics centers, and/or web-based sales portals, such as Amazon^{™}.

Through web portals, the cloud infrastructure may also allow users to view their real-time and historical cortisol data / trends and share the said data with caregivers, their healthcare provider(s), support network, and/or other suitable persons.

The foregoing description, for purposes of explanation, used specific nomenclature to provide a thorough understanding of the invention. The following claims define the scope of the invention.

## Claims

1. A microneedle array (500) for use in sensing cortisol, comprising:
a plurality of solid microneedles (510), wherein at least one microneedle of the plurality of solid microneedles comprises:
a tapered distal portion (514) having an insulated distal apex (516); and
a working electrode (520) located on a surface of the tapered distal portion (514) that is proximal to the insulated distal apex (516),
wherein the working electrode (520) is configured to generate a sensor signal that is indicative of a concentration of cortisol in the dermal interstitial fluid when contacting the dermal interstitial fluid.

2. The microneedle array of claim 1, wherein the working electrode comprises an electrode material and a biorecognition layer arranged at least partially over the electrode material, wherein the biorecognition layer comprises an aptamer that selectively and reversibly binds to cortisol.

3. The microneedle array of claim 2, wherein the aptamer is tethered directly or indirectly to the electrode material via a linker.

4. The microneedle array of claim 2, wherein:
the electrode material comprises gold; and
the aptamer is tethered to the electrode material via a thiol link.

5. The microneedle array of claim 2, wherein:
the biorecognition layer comprises a conductive polymer layer arranged at least partially over the electrode material; and
the aptamer is tethered to the conductive polymer layer.

6. The microneedle array of claim 5, wherein the aptamer is tethered to the conductive polymer layer via an amide linker.

7. The microneedle array of claim 2, wherein:
the electrode material comprises a silicon; and
the aptamer is tethered to the electrode material via a silane linker.

8. The microneedle array of claim 2, wherein:
the electrode material comprises carbon; and
the aptamer is tethered to the electrode material via an amide linker.

9. The microneedle array of claims 2-8, wherein the aptamer is covalently bound to a redox-active molecule at the 3' end or the 5' end of the aptamer such that selective binding of the cortisol to the aptamer and a resulting conformational change of the aptamer changes the proximity between the redox-active molecule and a surface of the electrode material to modulate electron transfer between the redox-active molecule and the electrode material, thereby generating the sensor signal.

10. The microneedle array of any one of claims 1-9, wherein the working electrode is an annular electrode comprising a proximal edge and a distal edge, and the distal edge of the annular working electrode is proximate a proximal edge of the insulated distal apex.

11. The microneedle array of claim 10 wherein the annular working electrode is on only a segment of the surface of the tapered distal portion of the microneedle.

12. A cortisol monitoring device comprising:
a wearable housing comprising a user interface; and
the microneedle array of claim 1 extending outwardly from the wearable housing,
wherein the user interface comprises one or more indicator lights, each of the one or more indicator lights configured to be selectively illuminated responsive to the sensor signal.

13. The cortisol monitoring device of claim 12, wherein the cortisol monitoring device is a skin-adhered patch.

14. The microneedle array of any of claims 1-11, wherein a portion of the working electrode overlies and extends beyond an annular recess formed in the surface of the tapered distal portion.

15. The microneedle array of any of claims 1-11 and 14, wherein at least one microneedle of the plurality of solid microneedles comprises:
a body portion comprising a conductive core extending from a substrate and providing an electrical connection to the working electrode; and
an insulating moat arranged around a base of the body portion and extending through the substrate to provide electrical insulation around the microneedle.

## Patentansprüche

1. Mikronadelanordnung (500) zur Verwendung beim Erfassen von Cortisol, umfassend:
mehrere massive Mikronadeln (510), wobei mindestens eine Mikronadel der mehreren massiven Mikronadeln umfasst:
einen verjüngten distalen Abschnitt (514) mit einer isolierten distalen Spitze (516); und
eine Arbeitselektrode (520), die sich auf einer Oberfläche des verjüngten distalen Abschnitts (514) befindet, die proximal zu der isolierten distalen Spitze (516) ist,
wobei die Arbeitselektrode (520) dazu ausgelegt ist, ein Sensorsignal zu erzeugen, das indikativ für eine Konzentration von Cortisol in der dermalen interstitiellen Flüssigkeit angibt, wenn sie mit der dermalen interstitiellen Flüssigkeit in Kontakt kommt.

2. Mikronadelanordnung nach Anspruch 1, wobei die Arbeitselektrode ein Elektrodenmaterial und eine Bioerkennungsschicht umfasst, die zumindest teilweise über dem Elektrodenmaterial angeordnet ist, wobei die Bioerkennungsschicht ein Aptamer umfasst, das selektiv und reversibel an Cortisol bindet.

3. Mikronadelanordnung nach Anspruch 2, wobei das Aptamer über einen Linker direkt oder indirekt an das Elektrodenmaterial gebunden ist.

4. Mikronadelanordnung nach Anspruch 2, wobei:
das Elektrodenmaterial Gold umfasst; und
das Aptamer über eine Thiolverbindung an das Elektrodenmaterial gebunden ist.

5. Mikronadelanordnung nach Anspruch 2, wobei:
die Bioerkennungsschicht eine leitfähige Polymerschicht umfasst, die zumindest teilweise über dem Elektrodenmaterial angeordnet ist; und
das Aptamer an die leitfähige Polymerschicht gebunden ist.

6. Mikronadelanordnung nach Anspruch 5, wobei das Aptamer über einen Amid-Linker an die leitfähige Polymerschicht gebunden ist.

7. Mikronadelanordnung nach Anspruch 2, wobei:
das Elektrodenmaterial Silizium umfasst; und
das Aptamer über einen Silan-Linker an das Elektrodenmaterial gebunden ist.

8. Mikronadelanordnung nach Anspruch 2, wobei:
das Elektrodenmaterial Kohlenstoff umfasst; und
das Aptamer über einen Amid-Linker an das Elektrodenmaterial gebunden ist.

9. Mikronadel-Array nach Anspruch 2-8, wobei das Aptamer kovalent an ein redoxaktives Molekül am 3'-Ende oder am 5'-Ende des Aptamers gebunden ist, so dass die selektive Bindung des Cortisols an das Aptamer und eine resultierende Konformationsänderung des Aptamers die Nähe zwischen dem redoxaktiven Molekül und einer Oberfläche des Elektrodenmaterials ändert, um dadurch den Elektronentransfer zwischen dem redoxaktiven Molekül und dem Elektrodenmaterial zu modulieren, wodurch das Sensorsignal erzeugt wird.

10. Mikronadelanordnung nach einem der Ansprüche 1-9, wobei die Arbeitselektrode eine ringförmige Elektrode ist, die eine proximale Kante und eine distale Kante umfasst, und die distale Kante der ringförmigen Arbeitselektrode nahe einer proximalen Kante der isolierten distalen Spitze ist.

11. Mikronadelanordnung nach Anspruch 10, wobei sich die ringförmige Arbeitselektrode nur auf einem Segment der Oberfläche des sich verjüngenden distalen Abschnitts der Mikronadel befindet.

12. Cortisol-Überwachungsvorrichtung, umfassend:
ein am Körper tragbares Gehäuse, das eine Benutzeroberfläche umfasst; und
die Mikronadelanordnung nach Anspruch 1, die sich von dem am Körper tragbaren Gehäuse nach außen erstreckt,
wobei die Benutzeroberfläche eine oder mehrere Anzeigeleuchten umfasst, wobei jede der einen oder mehreren Anzeigeleuchten dazu ausgelegt ist, als Reaktion auf das Sensorsignal selektiv erleuchtet zu werden.

13. Cortisol-Überwachungsvorrichtung nach Anspruch 12, wobei die Cortisol-Überwachungsvorrichtung ein an der Haut anhaftendes Pflaster ist.

14. Mikronadelanordnung nach einem der Ansprüche 1-11, wobei ein Abschnitt der Arbeitselektrode über einer ringförmigen Vertiefung liegt und sich über diese hinaus erstreckt, die in der Oberfläche des sich verjüngenden distalen Abschnitts gebildet ist.

15. Mikronadelanordnung nach einem der Ansprüche 1-11 und 14, wobei mindestens eine Mikronadel der mehreren massiven Mikronadeln umfasst:
einen Körperabschnitt, der einen leitfähigen Kern umfasst, der sich von einem Substrat erstreckt und eine elektrische Verbindung mit der Arbeitselektrode bereitstellt; und
einen Isolationsgraben, der um eine Basis des Körperabschnitts herum angeordnet ist und sich durch das Substrat erstreckt, um eine elektrische Isolierung um die Mikronadel herum bereitzustellen.

## Revendications

1. Réseau de microaiguilles (500) destiné à être utilisé pour détecter le cortisol, comprenant :
une pluralité de microaiguilles pleines (510), dans laquelle au moins une microaiguille de la pluralité de microaiguilles pleines comprend :
une partie distale effilée (514) ayant un sommet distal isolé (516) ; et
une électrode de travail (520) située sur une surface de la partie distale effilée (514) qui est proximale par rapport au sommet distal isolé (516),
dans lequel l'électrode de travail (520) est configurée pour générer un signal de capteur qui indique une concentration de cortisol dans le liquide interstitiel dermique lors du contact avec le liquide interstitiel dermique.

2. Réseau de microaiguilles selon la revendication 1, dans lequel l'électrode de travail comprend un matériau d'électrode et une couche de bioreconnaissance agencée au moins partiellement au-dessus le matériau d'électrode, dans lequel la couche de bioreconnaissance comprend un aptamère qui se lie de manière sélective et réversible au cortisol.

3. Réseau de microaiguilles selon la revendication 2, dans lequel l'aptamère est relié directement ou indirectement au matériau d'électrode par l'intermédiaire d'un lieur.

4. Réseau de microaiguilles selon la revendication 2, dans lequel :
le matériau d'électrode comprend de l'or ; et
l'aptamère est relié au matériau d'électrode par l'intermédiaire d'une liaison thiol.

5. Réseau de microaiguilles selon la revendication 2, dans lequel :
la couche de bioreconnaissance comprend une couche polymère conductrice agencée au moins partiellement au-dessus du matériau d'électrode ; et
l'aptamère est relié à la couche de polymère conducteur.

6. Réseau de microaiguilles selon la revendication 5, dans lequel l'aptamère est relié à la couche polymère conductrice par l'intermédiaire d'un lieur amide.

7. Réseau de microaiguilles selon la revendication 2, dans lequel :
le matériau d'électrode comprend un silicium ; et
l'aptamère est relié au matériau d'électrode par l'intermédiaire d'un lieur silane.

8. Réseau de microaiguilles selon la revendication 2, dans lequel :
le matériau d'électrode comprend du carbone ; et
l'aptamère est relié au matériau d'électrode par l'intermédiaire d'un lieur amide.

9. Réseau de microaiguilles selon les revendications 2 à 8, dans lequel l'aptamère est lié de manière covalente à une molécule à activité redox au niveau de l'extrémité 3' ou de l'extrémité 5' de l'aptamère de telle sorte que la liaison sélective du cortisol à l'aptamère et un changement de conformation résultant de l'aptamère modifient la proximité entre la molécule à activité redox et une surface du matériau d'électrode pour moduler le transfert d'électrons entre la molécule à activité redox et le matériau d'électrode, générant de ce fait le signal de capteur.

10. Réseau de microaiguilles selon l'une quelconque des revendications 1 à 9, dans lequel l'électrode de travail est une électrode annulaire comprenant un bord proximal et un bord distal, et le bord distal de l'électrode de travail annulaire est proche d'un bord proximal du sommet distal isolé.

11. Réseau de microaiguilles selon la revendication 10 dans lequel l'électrode de travail annulaire se trouve uniquement sur un segment de la surface de la partie distale effilée de la microaiguille.

12. Dispositif de surveillance de cortisol comprenant :
un boîtier portable comprenant une interface utilisateur ; et
le réseau de microaiguilles selon la revendication 1 s'étendant vers l'extérieur à partir du boîtier portable, dans lequel l'interface utilisateur comprend un ou plusieurs voyants lumineux, chacun du ou des voyants lumineux étant configuré pour être sélectivement allumé en réponse au signal de capteur.

13. Dispositif de surveillance de cortisol selon la revendication 12, le dispositif de surveillance de cortisol étant un timbre adhérant à la peau.

14. Réseau de microaiguilles selon l'une quelconque des revendications 1 à 11, dans lequel une partie de l'électrode de travail recouvre et s'étend au-delà d'un évidement annulaire formé dans la surface de la partie distale effilée.

15. Réseau de microaiguilles selon l'une quelconque des revendications 1 à 11 et 14, dans lequel au moins une microaiguille de la pluralité de microaiguilles pleines comprend :
une partie corps comprenant une âme conductrice s'étendant à partir d'un substrat et fournissant une connexion électrique à l'électrode de travail ; et
une cavité isolante agencée autour d'une base de la partie corps et s'étendant à travers le substrat pour assurer l'isolation électrique autour de la microaiguille.
